# EUROPEAN PATENT SPECIFICATION

(11) **EP 2 559 701 B1**
(45) Date of publication and mention of the grant of the patent: **20.04.2016**
(21) Application number: 12003245.3
(22) Date of filing: 23.11.2007
(51) Int. Cl.: C07K 14/705, C12N 15/85, A01K 67/027

(54) **Transgenic rabbit with enhanced immune response and method for the preparation thereof**
Transgenes Kaninchen mit verbesserter Immunantwort und Verfahren zu dessen Erschaffung
Lapin transgénique avec réponse immunitaire améliorée et procédé de préparation associé

(30) Priority: 24.11.2006 HU 0600870; 14.08.2007 HU 0700534
(43) Date of publication of application: 20.02.2013
(62) Divisional of application: 11004318.9
(73) Proprietor: Agricultural Biotechnology Center, 2100 Gödöll (HU); Eötvös Lorand University, 1053 Budapest (HU)
(72) Inventor: Bösze, Zsuzsanna, 2100 Gödöllö (HU); Cervenak, Judit, 2092 Budakeszi (HU); Kacskovics, Imre, 2092 Budakeszi (HU); Hiripi, László, 2100 Gödöllö (HU); Bender, Balázs, 3078 Bátonyterenye (HU)
(74) Representative: Graf von Stosch, Andreas

(56) References cited:
- EP-A- 1 790 716
- WO-A-01/57088
- WO-A1-99/13092
- US-A1- 2002 192 222
- US-A1- 2006 031 954
- BENDER BALAZS ET AL: "Generation of bovine FcRn alpha chain BAC transgenic mice", TISSUE ANTIGENS, vol. 64, no. 4, October 2004 (2004-10), pages 374-375, XP002482022, & 1ST INTERNATIONAL CONFERENCE ON BASIC AND CLINICAL IMMUNOGENOMICS; BUDAPEST, HUNGARY; OCTOBER 03-07, 2004 ISSN: 0001-2815
- LU W ET AL: "OVER-EXPRESSION OF THE BOVINE NEONATAL Fc RECEPTOR GENE IN THE MAMMARY GLAND OF TRANSGENIC MICE", 8TH WORLD CONGRESS ON GENETICS APPLIED TO LIVESTOCK PRODUCTION, BELO HORIZONTE, MG, BRAZIL, 13-18/08/2006 , 13 August 2006 (2006-08-13), XP002482023, Retrieved from the Internet: URL:http://www.wcgalp8.org.br/wcgalp8/arti cles/paper/19_627-2040.pdf [retrieved on 2008-05-26]
- BALÁZS BENDER ET AL: "Position independent and copy-number-related expression of the bovine neonatal Fc receptor [alpha]-chain in transgenic mice carrying a 102 kb BAC genomic fragment", TRANSGENIC RESEARCH, KLUWER ACADEMIC PUBLISHERS-PLENUM PUBLISHERS, NE, vol. 16, no. 5, 27 June 2007 (2007-06-27), pages 613-627, XP019534556, ISSN: 1573-9368
- YOSHIDA MASARU ET AL: "Neonatal Fc receptor for IgG regulates mucosal immune responses to luminal bacteria", JOURNAL OF CLINICAL INVESTIGATION, vol. 116, no. 8, August 2006 (2006-08), pages 2142-2151, XP002482024, ISSN: 0021-9738
- AKILESH SHREERAM ET AL: "The MHC class I-like Fc receptor promotes humorally mediated autoimmune disease", JOURNAL OF CLINICAL INVESTIGATION, NEW YORK, NY, US, vol. 113, no. 9, 1 May 2004 (2004-05-01), pages 1328-1333, XP002417288, ISSN: 0021-9738
- ROGEL-GAILLARD CLAIRE ET AL: "Construction of a rabbit bacterial artificial chromosome (BAC) library: Application to the mapping of the major histocompatibility complex to position 12q1.1", MAMMALIAN GENOME, vol. 12, no. 3, March 2001 (2001-03), pages 253-255, ISSN: 0938-8990

## Description

The present invention relates to the field of immunology. More specifically, the invention provides a transgenic (Tg) rabbit capable of developing an enhanced humoral immune response, in particular capable of developing a more robust immune response, against an antigen as compared to a non-transgenic control animal of the same species, comprising an introduced genetic construct providing for the expression of a nucleic acid sequence encoding the α-chain of the rabbit FcRn protein, wherein said genetic construct provides for enhanced MHC class I-related neonatal Fc receptor (FcRn) activity. Also provided is a method for producing such a transgenic (Tg) rabbit comprising introducing into said rabbit the genetic construct providing for the expression of a nucleic acid sequence encoding the α-chain of the rabbit FcRn protein, wherein said genetic construct provides for enhanced FcRn activity, as well as the use of such rabbit in a non-therapeutical method comprising a step of developing an enhanced humoral immune response in said Tg rabbit against an antigen of interest. The present invention further provides methods for producing immunoglobulins using such a transgenic rabbit.

In response to antigen, plasma cells develop from B lymphocytes, in a specific humoral immune reaction that peaks at about 1 to 2 weeks after antigenic insult. Secondary encounter with antigen results in the secretion of antibodies of increased affinity to the antigen, an increased peak of specific serum antibody titer, and persistent levels of antibodies in the serum. Maintenance of specific serum antibody levels requires continuous secretion of Ig by plasma cells and protecting them from fast elimination. While IgM, IgA, IgE are eliminated relatively rapidly from the body, the serum half-life of IgG is prolonged. In 1958, Brambell described a saturable receptor system that mediates the maternal IgG transport (Brambell, 1958); then, he inferred the presence of a similar or identical receptor that protected IgG from catabolism to make it the longest surviving of all plasma proteins (Brambell et al., 1964) The Brambell receptor (FcRB) was eventually shown both to mediate the transmission of IgG in antenatal and/or neonatal period, in this expression termed FcRn (neonatal Fc receptor), and to mediate the protection of IgG from catabolism (Junghans, 1997).

FcRn was first identified in rodents as the receptor that transfers maternal immunoglobulins from mother to newborn via the neonatal intestine (Rodewald, 1976; Simister and Rees, 1985). Since its original description in rat neonatal intestine by Simister and Mostov (Simister and Mostov, 1989), various studies have shown that FcRn plays a central role in regulating the transport of IgG within and across cells of diverse origin (Antohe et al., 2001; Claypool et al., 2004; Dickinson et al., 1999; Kobayashi et al., 2002; McCarthy et al., 2000; Ober et al., 2004; Spiekermann et al., 2002). It also serves to rescue IgG and albumin, the two most abundant soluble proteins in serum, from degradation, and thereby prolong their half-lives (Ghetie et al., 1996; Israel et al., 1996; Junghans and Anderson, 1996). The mechanism was originally thought to be mediated mainly by endothelial cells that line blood vessels (Borvak et al., 1998), however recent findings suggest that this process occur in other cells, too (Akilesh et al., 2007; Lu et al., 2007). Inside these cells, FcRn predominantly resides in early/recycling endosomes, where it encounters IgG and albumin internalized by fluid phase endocytosis. The acidic environment of the endosomes facilitates the interaction. Bound IgG and albumin are recycled back to the surface and released from the cell, while unbound ligands are shuttled downstream to lysosomal degradation (Anderson et al., 2006; Roopenian and Akilesh, 2007). More recent data support a novel concept in which FcRn fulfills a major role in IgG-mediated phagocytosis (Vidarsson et al., 2006).

The functional FcRn molecule is a heterodimer composed of an MHC class-1 like α-chain (or heavy chain) and the beta 2-microglobulin (β2m; alternative name: light chain) (Simister and Mostov, 1989) that binds IgG and albumin in a pH dependent manner (Chaudhury et al., 2003; Simister and Mostov, 1989) although at different binding sites (Andersen et al., 2006; Chaudhury et al., 2006).

FcRn has been cloned from a wide variety of mammalian species, among them the rat (Simister and Mostov, 1989), mouse (Ahouse et al., 1993), human (Story et al., 1994), bovine (Kacskovics et al., 2000) possum (Adamski et al., 2000), sheep (Mayer et al., 2002) pig (Schnulle and Hurley, 2003; Zhao et al., 2003), camel and dog (Kacskovics et al., 2006b). For example, WO 01/57088 discloses FcRn α-chain DNA molecules and respective proteins of cow, dromedary and sheep, and vectors containing such FcRn α-chain DNA molecules. More recently the present investors cloned and characterized the rabbit FcRn α-chain. Although most of the functions that are fulfilled by the FcRn have been described in mice, studies in other mammalians suggest that the role of FcRn in IgG catabolism is similarly crucial in all investigated mammalians, like rodents, human and primates (Ghetie and Ward, 2002), pig (Harmsen et al., 2005) and bovine (Kacskovics et al., 2006a).

It was recently shown in two different mouse models that bovine FcRn α-chain (bFcRn) overexpression significantly elongated the half-life of the mouse IgG in these animals (Bender et al., 2007; Lu et al., 2007) indicating that bFcRn forms a functional complex with the mouse β2m (mβ2m), and that it binds to mouse and human IgGs. The present inventors also found that overexpression of the bFcRn in transgenic (Tg) mice (Bender et al., 2007) enabled these animals to produce significantly elevated level of antigen specific IgG and IgM upon immunization.

WO2007061292 and its priority document EP 1 790 716, which were filed earlier but published after the priority date of the present application, disclose the preparation of monoclonal antibodies, wherein the antibody-producing cells are transfected with a nucleic acid encoding the gene for FcRn. The modified cells express FcRn and the antibody production is elevated. The authors do not provide enabling disclosure for neither the preparation of transgenic animals, nor the animals itself, still they mention the advantages of non-human animals with at least one (additional) copy of nucleic acid encoding FcRn for upregulating the antibody levels in the blood. Nevertheless, the teaching is clearly theoretical and based on their description focuses on monoclonal antibody production *in vivo* e.g. in mouse ascites fluid. Neither did they indicate the advantage of the FcRn overexpression in enhancing the immune response. NOR the production of a rabbit transgenic for a nucleic acid sequence encoding the alpha-chain of the rabbit FcRn protein.

It is clear that there is still a need in the art for the improved provision of diagnostic, research and therapeutic immunoglobulins in high quantity and quality. To fulfill this need the present invention provides transgenic rabbits that respond to antigenic challenges with a dramatically elevated level of specific humoral immune reaction.

It was surprisingly found that the BAC Tg mice with copy number dependent over-expression of the bovine FcRn α-chain (encoded by the bovine FCGRT gene) resulted not only that the bovine FcRn α-chain formed a functional complex with the mouse β2m and significantly increased the half-life of exogenously administered mouse and human IgG, but these transgenic animals also showed dramatically augmented humoral immune response upon immunization compared to their wild type controls. Most of the antigen specific antibodies was IgG, however the IgM titer was also increased during the secondary immune response. When analyzing the possible reasons, the present inventors detected a significantly increased number of antigen specific B-cells, dendritic cells and a massive neutrophil influx in the secondary lymphoid organs upon immunization in the bFcRn transgenic mice compared to their wild type controls. Noteworthy that similar, albeit less dramatic changes were observed in the wild type controls. As a consequence, it could be shown that the increased antigen-specific IgG and IgM levels in the bFcRn Tg animals was the result of not only the improved IgG protection, but also the greater antigen specific clonal B-cell expansion and consequently a more robust immunoglobulin synthesis compared to their wild type controls. These results point to a novel role for FcRn in the immune response. The present invention is the first to disclose that due to the remarkable overexpressed FcRn status not occurring in normal animals, it has a profound effect on the immune response upon immunization, which makes the system especially useful for producing different types of antibodies against a wide range of antigens.

### Summary of the invention

The invention in its broadest sense is as defined in the independent claims.

Accordingly, the present invention provides a method for producing a Transgenic (Tg) rabbit capable of developing an enhanced humoral immune response against an antigen as compared to a non-transgenic control animal of the same species, comprising introducing into said rabbit a genetic construct providing for the expression of a nucleic acid sequence encoding the α-chain of the rabbit FcRn protein, wherein said genetic construct provides for enhanced MHC class I-related neonatal Fc receptor (FcRn) activity.

In another aspect, the present invention provides a Tg rabbit capable of developing an enhanced humoral immune response, in particular capable of developing a more robust immune response, against an antigen as compared to a non-transgenic control animal of the same species, comprising an introduced genetic construct providing for the expression of a nucleic acid sequence encoding the α-chain of the rabbit FcRn protein, wherein said genetic construct provides for enhanced FcRn activity.

In a further aspect, the present invention provides the use of a Tg rabbit according to the invention comprising a genetic construct providing for the expression of a nucleic acid sequence encoding the α-chain of the rabbit FcRn protein, wherein said genetic construct provides for enhanced FcRn activity, in a non-therapeutical method comprising a step of developing an enhanced humoral immune response in said Tg rabbit against an antigen of interest.

In still another aspect, the present invention provides a method for producing immunoglobulins, comprising a step of using a Tg rabbit according to the invention, comprising a genetic construct providing for the expression of a nucleic acid sequence encoding the α-chain of the rabbit FcRn protein, wherein said genetic construct provides for enhanced FcRn activity, in accordance with any established protocol enabling the production of immunoglobulins.

In a preferred embodiment, the present invention provides a method, use or a Tg rabbit according to the invention, wherein said enhancement of the humoral immune response comprises the production of elevated levels of immunoglobulins upon immunization with an antigen, wherein said FcRn having specific affinity for the immunoglobulins being produced by said animal upon said immunization.

In a preferred embodiment, the present invention provides a method, use or a Tg rabbit according to the invention, wherein said enhancement of the humoral immune response comprises the enhancement of the antigen specific clonal B cell expansion.

In a preferred embodiment, the present invention provides a method, use or a Tg rabbit according to the invention, wherein said enhancement of the humoral immune response comprises the stimulation of the influx of neutrophils into the secondary lymphoid organs.

In a preferred embodiment, the present invention provides a method, use or a Tg rabbit according to the invention, wherein said enhancement of the humoral immune response comprises the stimulation of the influx of dendritic cells and/or macrophages into the secondary lymphoid organs.

In a preferred embodiment, the present invention provides a method, use or a Tg rabbit according to the invention, wherein said secondary lymphoid organs comprise the spleen.

In a preferred embodiment, the present invention provides a method, use or a Tg rabbit according to the invention, wherein said antigen is a weak immunogen.

In another aspect, the present invention provides a method for producing immunoglobulins, comprising using a Tg rabbit according to the invention comprising a genetic construct providing for the expression of a nucleic acid sequence encoding the α-chain of the rabbit FcRn protein, wherein said genetic construct provides for enhanced FcRn activity, said method comprising at least one step in which the applied protocol is adjusted to the fact that said Tg rabbit develops the same level of humoral immune response upon fewer number of antigen challenges as compared to a non-transgenic control rabbit.

In yet another aspect, the present invention provides a method for producing immunoglobulins, comprising using a Tg rabbit according to the invention comprising a genetic construct providing for the expression of a nucleic acid sequence encoding the α-chain of the rabbit FcRn protein, wherein said genetic construct provides for enhanced FcRn activity, said method comprising at least one step in which the applied protocol is adjusted to the fact that said Tg rabbit develops the same level of humoral immune response faster as compared to a non-transgenic control rabbit.

In a preferred embodiment, the present invention provides a method, use or a Tg rabbit according to the invention, wherein said transgenic rabbit is obtained from a strain useful for monoclonal antibody production or being modified genetically to become more suitable for monoclonal antibody production.

In another aspect, the present invention provides a hybridoma cell line, generated from cells obtained from a transgenic rabbit according to the invention or from a transgenic rabbit produced or used according to the method or use according to the invention, wherein said cells comprise a genetic construct providing for enhanced FcRn activity, wherein the genetic construct provides for the expression of a nucleic acid sequence encoding the alpha-chain of the rabbit FcRn protein.

In a preferred embodiment, the present invention provides a method, use or a Tg rabbit according to the invention, wherein said transgenic rabbit is obtained from a strain useful for polyclonal antibody production or being modified genetically to become more suitable for polyclonal antibody production.

In a preferred embodiment, the present invention provides a method, use or a Tg rabbit according to the invention, wherein said immunoglobulin is IgG.

In a preferred embodiment, the present invention provides a method, use or a Tg rabbit according to the invention, wherein said immunoglobulin is IgM.

In a preferred embodiment, the present invention provides a method, use or a Tg rabbit according to the invention, wherein said animal is transgenic for producing human or humanized immunoglobulins.

In a preferred embodiment, the present invention provides a method, use or a Tg rabbit according to the invention, wherein the said nucleic acid sequence encoding the α-chain of the FcRn protein is mutated.

In a preferred embodiment, the present invention provides a method, use or a Tg rabbit according to the invention, wherein said mutation renders the albumin binding site of said FcRn protein non-functional.

In a preferred embodiment, the present invention provides a method, use or a Tg rabbit according to the invention, wherein said nucleic acid sequence encodes a chimeric FcRn protein.

In another aspect, the present invention provides an animal propagation material obtained from a Tg rabbit according to the invention or from comprising a genetic construct providing for enhanced FcRn activity, wherein the genetic construct provides for the expression of a nucleic acid sequence encoding the α-chain of the rabbit FcRn protein.

In a preferred embodiment, the present invention provides such animal propagation material, that is selected from the group consisting of sperm, spermatogonium, ovum, ovarian tissue, embryo or tissue sample for somatic cloning.

In a preferred embodiment, the present invention provides a method, use or a Tg rabbit according to the invention, wherein said enhanced FcRn activity is provided by integrating more then one functional copy of a nucleic acid sequence encoding said FcRn into the genome of said animal.

In a preferred embodiment, the present invention provides a method, use or a Tg rabbit according to the invention, wherein said genetic construct comprises the rabbit insert of the BAC clone #262E02.

In a preferred embodiment, the present invention provides a method according to the invention, wherein said genetic construct provides for the expression of a nucleic acid sequence encoding the α-chain of the rabbit FcRn protein, and wherein the immunoglobulin binding activity of said FcRn protein is eliminated.

### Detailed description

The invention in its broadest sense is as defined in the independent claims.

In a first aspect, the present invention provides a method for producing a Tg rabbit capable of developing an enhanced humoral immune response against an antigen as compared to a non-transgenic control animal of the same species, comprising introducing into said rabbit a genetic construct providing for the expression of a nucleic acid sequence encoding the α-chain of the rabbit FcRn protein, wherein said genetic construct provides for enhanced MHC class I-related neonatal Fc receptor (FcRn) activity.

In a second aspect, the present invention provides a Tg rabbit capable of developing an enhanced humoral immune response, in particular capable of developing a more robust immune response, against an antigen as compared to a non-transgenic control animal of the same species, comprising an introduced genetic construct providing for the expression of a nucleic acid sequence encoding the α-chain of the rabbit FcRn protein, wherein said genetic construct provides for enhanced FcRn activity. In this respect, it must be noted that several state of the art disclosures discuss Tg animals that harbor FcRn transgenes.

Bender et al. (Bender et al., 2004) disclosed a Tg animal that was made by introducing the gene encoding the α-chain of the bovine FcRn protein into an FVB/N mouse using the bovine BAC clone #128E04, in order to study the transcriptional regulation and function of the bovine FcRn *in vivo.* The publication does not disclose those specific characteristics of the transgenic animal as claimed herein.

US2006/0031954 and Petkova et al. (Petkova et al., 2006) disclose Tg knock-out mice comprising a homozygous FcRn disruption and a human FcRn (hFcRn) transgene. This addition significantly increases the half-life of exogenously administered human IgG, and prefers to express the hFcRn similar to the endogenous expression levels. They also mention, without adding any specific details, that expressing FcRn at levels substantially higher than endogenous might be useful. It is speculated that the expression levels can be increased up to 10 to 100-fold over the endogenous expression level by using strong expression vectors. These disclosures teach several distinct constructs to obtain the Tg animals. In a first instance, a 33 kb human cosmid clone clone was used that includes the complete hFcRn gene plus 10 kb 5' and 10 kb 3' flanking sequences. The authors termed this Tg animal as "genomic hFcRn transgenic line 32". In a second instance, the "cDNA transgenic line 276" line harbors a genetic construct comprising the hFcRn α-chain cloned into vector E carrying a CMV enhance and chicken β-actin promoter. In a third instance, their transgenic animals harbor a genetic construct which comprises a 34 kb XhoI fragment that contains the complete hFcRn gene from a human-derived bacterial artificial chromosome (BAC) library from Genome Systems, Inc. The authors apparently created several Tg mice lines either with direct genetic manipulation (such as with C57BL/6J and BXSB/MpJ mice), or conventional crossing (e.g. with MRL/MpJ and NZM2410 strains), therefore a correct enumeration of all animals would be almost impossible.

Lu et al. disclosed Tg mice harboring bFcRn in their mammary glands by using a construct with mammary gland specific regulatory elements (Lu et al., 2006). Several mouse lines were established with different copy numbers ofbFcRn in the range of 1 to 15, and with bovine β2m copy numbers in the range of 1 to 10. Expression is examined and variations in the level of serum IgG are documented. This system, however, provides only limited bFcRn expression upon lactation in the mammary epithelial cells of the acini and was used to study the IgG transport in the lactating mammary gland. These animals have not been used for analyzing the immune response. The constructs used for generating these Tg mice were two expression vectors in which the goat beta-casein promoter regulates the transcription of the interested and inserted genes, one that comprises the sequences encoding the heavy (a) chain (pBCl-bFcRn) and the other encoding the light chain (pBC1-bβ2m) of the bovine FcRn. The genetic constructs being inserted into the Tg animals are fragments generated by digestion with NotI and SalI enzymes to yield a 16.9 kilobase (kb) heavy chain and a 16.1-kb light chain, which were microinjected into fertilized Kunming White mouse eggs at an equal concentration.

Yoshida et al. (Yoshida et al., 2006) studied the role of mouse FcRn (mFcRn) in intestinal epithelium in mediating antimicrobial immunity. They established FcRn Tg mouse lines in which mFcRn and mβ2m were specifically expressed by intestinal epithelial cells using the tissue-specific intestinal fatty acid-binding protein gerie promoter (IFABP) to create IFABP-mFcRnTg/mβ2mTg mice. They overexpressed the β2m, too, to ensure that it was not substrate limiting in expression of the FcRn transgene. Here again the Tg animals were further backcrossed to FcRn-/- mice then onto BALB/c or C57BL/6 (CD45.2+).

As used herein, the term "non-human animal" encompasses animals of the order of Vertebrae, preferably a mammal. Animals for which the present invention is applicable can be selected based on the functional criteria being able to produce immunoglobulins or functionally equivalents thereof. The present invention refers to rabbits. It is within the scope of the invention to use any rabbit commonly used in the art for producing immunoglobulins, either monoclonal antibodies or polyclonal antisera.

As used herein, the expression "control animal of the same species" or "non-transgenic control animal of the same species" is used to describe an animal, in particular a rabbit, that was not subjected to the procedures involved in producing the Tg rabbit of the invention. However, the control animal of the same species is treated and kept similarly to the Tg rabbit of the invention during the procedures necessary for the production of either polyclonal or monoclonal antibodies, for example for the primary immunization, for the booster immunization(s) as necessary, and in the course of the whole antibody production process.

As used herein, the term "immunoglobulins" refers to glycoproteins in the immunoglobulin superfamily that function as antibodies. The terms "antibody" and "immunoglobulin" are used interchangeably herein. Antibodies are host proteins that comprise one of the principal effectors of the adaptive immune system. Their utility has been harnessed as they have been and continue to be used extensively as a diagnostic and research reagent. They are also becoming an important therapeutic tool in the clinician's armamentarium to treat diseases. Antibodies are utilized for analysis, purification and enrichment, and to mediate or modulate physiological responses. The ability of antibodies to bind an antigen with a high degree of affinity and specificity has led to their ubiquitous use in a variety of scientific and medical disciplines. Antigen - antibody interaction is central to the antibody's natural biological function as well as its use as a research or therapeutic reagent.

In structure, the antibodies are globulins (in the γ-region of protein electrophoresis). In mammals there are five types of antibody: IgA, IgD, IgE, IgG, and IgM (Ig stands for immunoglobulin, which is also used throughout the present description for antibodies). These are classified according to differences in their heavy chain constant domains. Each immunoglobulin class differs in its biological properties and has evolved to deal with different antigens. IgA can be found in areas containing mucus (e.g. in the gut, in the respiratory tract or in the urogenital tract) and prevents the colonization of mucosal areas by pathogens. IgD functions mainly as an antigen receptor on B cells. IgE binds to allergens and triggers histamine release from mast cells (the underlying mechanism of allergy) and also provides protection against helminths (worms). IgM is expressed on the surface of B cells and also in a secreted form for eliminating pathogens in the early stages of B cell mediated immunity. IgG provides the majority of antibody-based immunity against invading pathogens.

In a preferred embodiment of the present invention, the immunoglobulin being produced is IgG, although the scope of the invention is not limited to the IgG isotype (class), or the fact that the IgG might comprise modifications, as discussed below.

IgGs can be classified into different forms or subclasses, which are the result of gene duplication in various mammals. There are multiple subclasses of IgG in rodents, humans, domesticated ruminants, horses, swine, camelids and guinea pigs, where the different subclasses possess different biological characteristics. For example, human IgG1 and IgG3 decorate phagocytic cells because of their high affinity for FcγR1. IgG1 and IgG3 may therefore be especially important in: (a) the removal of small IgG-Ag complexes and (b) positively or negatively stimulating B cell development and antibody production. IgG3 is a powerful activator for complement and IgG1 is the major serum IgG that is preferentially transported across the placenta (Janeway Jr. et al., 2001). It is well known that subclass diversity varies a great deal among mammals. Rabbits have only one gene for IgG whereas mice and humans express four IgGs and horses have seven. Cattle have three IgGs, and there are five putative IgG subclass genes in swine. It is likely that similar differences in biological functions and relative expression will be found among immunoglobulin subclasses in less-well studied mammals (Butler, 2006).

The present invention can be utilized to enhance the production of any one or more of the IgG subtypes without specific limitations. However, it can be possible that an IgG isotype being produced by the recipient animal binds tighter to the FcRn protein comprising a specific exogenous FcRn α-chain rather than the FcRn protein comprising another exogenous FcRn α-chain. It is within the scope of the present invention to utilize a specific rabbit FcRn α-chain transgene to enrich the amount or ratio of a specific IgG subtype in the antiserum produced by selecting the FcRn transgene with the optimum binding capability and activity towards the said IgG subtype.

In another preferred embodiment of the present invention, the immunoglobulin being produced is IgM. IgM makes up approximately 10% of whole plasma immunoglobulin and forms a major component of early-stage antibodies produced against cell membrane antigens that are of complex antigenicity, infectious microorganisms, and soluble antigens. With respect to its structure, IgM has a pentamer structure in vivo. The five subunits, which constitute the pentamer structure of IgM, have a four-chain structure similar to IgG. One of the major differences compared to the structure of IgG that the IgM is characterized by having µ heavy chain. Additionally, they are different in that the µ chain has one more domain in its constant region than the γ chain and IgM has a polypeptide chain called J chain, which is not found in IgG and is considered to facilitate polymerization of µ chains before IgM is secreted from antibody-producing cells. There are attempts in the art to produce by recombinant methodologies IgM that is functionally equivalent to the natural product (see, for example, US2007154469). In contrast to the approaches tried to augment the IgM production by introducing heterologous or otherwise modified immunoglobulin genes into an animal, the present invention provides an alternative solution by providing a Tg rabbit comprising a genetic construct providing for the expression of a nucleic acid sequence encoding the α-chain of the rabbit FcRn protein, wherein said genetic construct provides for enhanced FcRn activity, which rabbit in turn produces elevated levels of the endogenous IgM in response to an antigen.

As used herein, the term "humoral immune response" refers to a process in living organisms wherein antibodies are produced in response to molecules and organisms, which they ultimately neutralize and/or eliminate. The specificity of the antibody response is mediated by T and/or B cells through membrane-associated receptors that bind antigen of a single specificity. Following binding of an appropriate antigen and receipt of various other activating signals, B lymphocytes divide, which produces memory B cells as well as terminally differentiating into antibody secreting plasma cell clones, each producing antibodies that recognize the identical antigenic epitope as was recognized by its antigen receptor. Memory B lymphocytes remain dormant until they are subsequently activated by their specific antigen. These lymphocytes provide the cellular basis of memory and the resulting escalation in antibody response when re-exposed to a specific antigen.

With reference to the humoral immune response, the term "enhanced" refers to an immune response wherein the level and polyclonality of the antibodies produced in response to a given antigen is significantly higher, or the development of said immune response is significantly faster, or the immune response is more robust in response to a given antigen, compared to the same characteristics of the antibodies produced in response to a given antigen or to the immune response when a control animal of the same species is contacted with the same antigen.

As used herein, the term "immunization" refers to a process by which an individual is exposed to an agent, often combined with adjuvants, that is designed to fortify his or her immune system against that agent. The agent is referred to as "immunogen" or "antigen". Immunization is the same as inoculation and vaccination in that inoculation and vaccination use a viable infecting agent like immunization does. In addition to the initial immunization process, it has been found that the effectiveness of immunizations can be improved by periodic repeat injections of the agent, which is referred to as "booster" immunization.

With reference to the immunization, the expression "elevated levels" of immunoglobulin refers to a concentration of specific serum immunoglobulin that is significantly higher than the level of the same specific immunoglobulin in the serum of a control animal of the same species, as measured by the strength of the immune response, e.g., as gauged by the peak antibody titer that results. This elevated level is usually at least 50 % higher, preferably 75 % higher, more preferably 100% higher, even more preferably even 150% higher.

The outcome of an immunization highly depends on the nature of antigen. It is widely accepted that any substance that can elicit an immune response is said to be immunogenic and is called immunogen. There is a clear operational distinction between an immunogen and an antigen. An antigen is defined as any substance that can bind to a specific antibody. All antigens therefore have the potential to elicit specific antibodies, but some need to be attached to an immunogen in order to do so. This means that although all immunogens are antigens, not all antigens are immunogenic. The antigens used most frequently in experimental immunology are proteins, and antibodies to proteins are of enormous utility in experimental biology and medicine. Purified proteins are, however, not always highly immunogenic and to provoke an immune response have to be administered with an adjuvant. Carbohydrates, nucleic acids and other types of molecule are all potential antigens, but will often only induce an immune response if attached to a protein carrier. Thus, the immunogenicity of protein antigens determines the outcome of virtually every immune response. The route by which antigen is administered affects both the magnitude and the type of immune response obtained. The most common routes by which antigen are introduced into the body are injection into tissue by subcutaneous, intradermal, intramuscular, intravenous or intraperitoneal injection or transfusion. Oral administration provides antigen into gastrointestinal tract, while intranasal administration or inhalation brings antigen into the respiratory tract.

Antibodies can recognize as antigens almost every kind of biologic molecule, including simple intermediary metabolites, sugars, lipids, autacoids, and hormones, as well as macromolecules such as complex carbohydrates, phospholipids, nucleic acids, and proteins. However, only macromolecules are capable of stimulating B lymphocytes to initiate humoral immune responses. Small chemicals, such as dinitrophenol, may bind to antibodies but can not activate B cells on their own (i.e. they are not immunogenic). To generate antibodies specific for such small chemicals, immunologists commonly attach them to macromolecules before immunization. In these cases, the small chemical is called a "hapten", and the macromolecule is called a carrier. The hapten-carrier complex, unlike free hapten, can act as an immunogen (Abbas and Lichtman, 2003). Similar and further immunization protocols are widely known in the art, and the selection of the appropriate strategy will be readily determined by the skilled person reducing the invention to practice.

The primary immune response usually results in only a slight, non-significant increase in the level of both the specific antibodies produced against the immunogen/antigen introduced, and the total immunoglobulin levels. The magnitude of the immune response depends on the dose of immunogen administered. Below a certain threshold dose, most proteins do not elicit any immune response. Above the threshold dose, there is a gradual increase in the response as the dose of antigen is increased, until a broad plateau level is reached, followed by a decline at very high antigen doses. In general, secondary and subsequent immune responses occur at lower antigen doses and achieve higher plateau values, which is a sign of immunological memory. This is the reason why usually a booster immunization is necessary to achieve significantly elevated titers of the specific antibody. The number of booster immunization can vary according to several factors, such as the immunogenicity of the antigen, the type of immunological adjuvant, immunization route and schedule (Stills, 2005).

Because most antigens are highly complex, they present numerous epitopes that are recognized by a large number of lymphocytes. Each lymphocyte is activated to proliferate and differentiate into plasma cells, and the resulting antibody response is polyclonal antibodies (PAbs). An important feature of the adaptive immune response is the immunological memory. Immunological memory is produced as a result of the initial or primary immunization, which evokes the primary immune response.

Most proteins are poorly immunogenic or nonimmunogenic when administered by themselves. Strong adaptive immune responses to protein antigens always require that the antigen be injected in a mixture known as an adjuvant. An adjuvant is any substance that enhances the immunogenicity of substances mixed with it. Adjuvants differ from protein carriers in that they do not form stable linkages with the immunogen. Priming with an adjuvant, immunogens - even an immunogen which produces a weak response or even no response - will produce a substantial immune response when a booster dose of adjuvant obligatory immunogen in the absence of adjuvant is given (Janeway Jr. et al., 2001; Leenaars and Hendriksen, 2005; Lipman et al., 2005; McCullough and Summerfield, 2005; Schunk and Macallum, 2005; Stills, 2005).

The response to each immunization is increasingly intense, so that secondary, tertiary and subsequent responses are of increasing magnitude. Repetitive challenge with antigen to achieve a heightened state of immunity is known as hyperimmunization. Many of the antibodies used in clinical and immunochemical techniques are polyclonal antibodies that are raised by hyperimmunization of a suitable animal, e.g., rodents, rabbit, goat, donkey, or sheep, with a suspension of the appropriate antigen. Serum is harvested at the peak of antibody production and specific immunoglobulin G (IgG) concentrations of approximately 1 to 10 mg/mL serum can be obtained by this method. Although monoclonal antibodies (mAbs) have proven to be a source of well-characterized, low immunogenicity and highly effective tools; there still is a place for polyclonal antibodies. Just as the natural immune system uses polyclonal rather than mAbs in responding to pathogens, polyclonal antibodies may also be preferable for passive immunotherapy in many cases. Advantages of polyclonal antibodies include their potentially increased potency in immune complex formation, their utility in combating infectious diseases caused by diverse strains of pathogens or that require neutralization of multiple epitopes for successful treatment. Polyclonal antibodies are also advantageous in immunochemical techniques as they are generally relatively easy to make, and much less expensive to produce. Also, polyclonal antibodies may be generated in a variety of species, including rabbit, goat, sheep, donkey, chicken and others, giving the users many options in experimental design. However, the amount of specific antibodies in a polyclonal preparation sometimes represents only a minute fraction of the total antibody protein. Therefore, the primary goal of the antibody production for these purposes is to obtain high titer, high affinity antisera.

In hyperimmunization protocols widely used in the art, regular booster immunizations are necessary to maintain a high IgG level, since immunoglobulin catabolism is increased in normal animals, as it had been already shown very early in studies of IgG metabolism (Andersen and Bjorneboe, 1964). Conversely, the present invention provides a more efficient protocol to achieve stably elevated levels of immunoglobulin production, where high IgG level are preserved for a relatively long time without further immunization or less frequent immunization.

In the production of polyclonal antibodies, a number of critical steps can be identified that may influence the outcome of the animal experiment, like the immunological results and the pain and suffering of the animals. When the antigen to which antibodies are to be evoked is poorly immunogenic, the immune system requires a stimulus to induce an effective immune response. Adjuvants can be used for this purpose, and can direct an immune response against a more cellular or humoral response. Although more than 100 adjuvants have been described, only a few adjuvants are routinely utilized for polyclonal antibody production (e.g. Freund's complete adjuvant (FCA); Freund's incomplete adjuvant, aluminum salts, Quil A, Iscoms, Montanide, TiterMax™, and RIBI™, etc). FCA is frequently used for the production of polyclonal antibodies because high antibody titers are induced to almost all types of antigens. However, many investigators have reported severe side effects after injection of, for example, FCA, TiterMax and RIBI adjuvants. The severity of pathological changes depends not only on the adjuvant but also on the type of antigen used. Moreover, the alternative adjuvants have not often induced effective antibody responses. The injected volume has been found to have an additional effect on the extent of the lesions produced (Leenaars and Hendriksen, 2005). Based on these factors, the present invention provides a more efficient protocol allowing less frequent immunization that is a great advantage in term of animal welfare, beyond of economic value, since this protocol reduces animals' pain and distress while obtaining optimal immune responses.

Accordingly, the present invention provides a method for producing polyclonal immunoglobulins, comprising using a Tg rabbit according to the invention comprising a genetic construct providing for the expression of a nucleic acid sequence encoding the α-chain of the rabbit FcRn protein, wherein said genetic construct provides for enhanced FcRn activity, in accordance with any established immunization protocol enabling the production of polyclonal immunoglobulins. The transgenic rabbit is preferably obtained from a strain useful for polyclonal antibody production or being modified genetically to become more suitable for polyclonal antibody production.

In a further preferred aspect, the disclosure provides a polyclonal antibody, generated by the method, use or Tg rabbit according to the present invention. The antibodies generated according to the present invention may be clearly and unambiguously differentiated from preparations not generated according to the invention by analyzing a crude or partially purified serum preparation containing the antibodies by standard molecular biological techniques. These may involve a comparison to a control serum sample obtained from a known non-transgenic control animal of the same species by determining the copy number or species specificity of the FcRn heavy-chain in small genomic DNA fragments in the serum being analyzed (Emanuel and Pestka, 1993; Lin and Floros, 2000; Sandford and Pare, 1997).

In contrast to PAbs, monoclonal antibodies (MAbs) are antibodies produced by a single B lymphocyte clone. In the mid-1970s, Köhler and Milstein devised the technique for generating monoclonal antibodies of a desired specificity, for which they were awarded the Nobel prize (Kohler and Milstein, 1975). After decades of practice and experiences cumulated in the field of MAbs, a person with average skills in the art should be adequately proficient in all aspects of the production of monoclonal antibodies. To produce a monoclonal antibody specific for a defined antigen, a mouse, rat or rabbit is immunized with that antigen, and B cells may be used as splenocytes, lymph node lymphocytes, or other peripheral blood lymphocytes or lymphocytes of other tissue of the animal. The mammalian host may be subject to extra immunizations to further enhance the desired antigen specific B cell population as well as antigen specificity. These isolated B cells are then fused with an appropriate immortalized cell line. Myeloma lines are the best fusion partners for B cells because like cells tend to fuse and give rise to stable hybrids more efficiently than unlike cells. The immortal cell is a lymphoblastoid cell or a plasmacytoma cell such as a myeloma cell, which is an antibody producing cell and is malignant. Supernatants of the hybridomas are screened to select the optimal hybridomas that have desirable antigen binding properties. The selected hybridomas are cloned and cryopreserved.

In this context, with respect to monoclonal antibodies, animal refers to any rabbit. The invention further includes several types of transgenic rabbits used to produce the lymphoid cell line.

Besides of the conventional or classical techniques, the use of recombinant technologies has initiated a new era in polyclonal and monoclonal antibody production, which now allow for engineering antibodies (e.g., reducing the antibody to its functional size, or making humanized antibodies) (Lonberg, 2005; Peterson, 2005).

Accordingly, the present invention provides a method for producing monoclonal immunoglobulins, comprising using a Tg rabbit according to the invention comprising a genetic construct providing for the expression of a nucleic acid sequence encoding the α-chain of the rabbit FcRn protein, wherein said genetic construct provides for enhanced FcRn activity, in accordance with any established immunization protocol enabling the production of monoclonal immunoglobulins. The transgenic rabbit is preferably obtained from a strain useful for monoclonal antibody production or being modified genetically to become more suitable for monoclonal antibody production.

In further preferred embodiments, the invention provides a hybridoma cell line, generated by using cells obtained from a transgenic rabbit according to the invention or from a transgenic rabbit used according to the method or use according to the invention in a state-of-the art protocol. Monoclonal antibodies are also provided that are generated according to the method, use or Tg rabbit of the invention.

As used herein, the term "transgenic" within the context of the expression "transgenic animal" refers to an animal that contains a gene or other nucleic acid sequence that it would not have obtained through normal breeding or mating practices. The term "gene" refers to a nucleic acid fragment that expresses mRNA, functional RNA, or specific protein, including regulatory sequences. The term "native gene" refers to a gene as found in nature. A "transgene" refers to a gene that has been introduced into the genome by transformation and is stably maintained. In this context, the term "transformation" is herein used as a broad term for introducing foreign DNA into a cell. The term is also meant to cover other functional equivalent methods for introducing foreign DNA into a cell, such as e.g., microinjection, transfection, infection, transduction or fusion of a donor cell and an acceptor cell. Transgenes may include, for example, genes that are either heterologous or homologous to the genes of a particular animal to be transformed. Additionally, transgenes may comprise native genes inserted into a non-native organism, or chimeric genes. The term "endogenous gene" refers to a native gene in its natural location in the genome of an organism.

Introduction of a transgene into a recipient animal is generally done by using a genetic construct carrying the transgene of interest. As used herein, the term "genetic construct" is meant to include artificially created recombinant DNA comprising nucleic acid sequences that, upon introduction into the recipient cell, provides the expression of the said introduced nucleic acid sequences. A genetic construct may comprise coding sequences and regulatory sequences. The term "coding sequence" refers to a DNA or RNA sequence that codes for a specific amino acid sequence and excludes the non-coding sequences. The terms "regulatory sequences" refers to nucleotide sequences located upstream (5' non-coding sequences), within, or downstream (3' non-coding sequences) of a coding sequence, and which influence the transcription, RNA processing or stability, or translation of the associated coding sequence. Regulatory sequences include enhancers, promoters, translation leader sequences, introns, and polyadenylation signal sequences. They include natural and synthetic sequences, as well as sequences which may be a combination of synthetic and natural sequences. Some regulatory sequences useful in the present invention will include, but are not limited to constitutive promoters, tissue-specific promoters, developmental stage-specific promoters, inducible promoters and viral promoters.

The term "promoter" refers to a nucleotide sequence, usually upstream (5') to its coding sequence, which controls the expression of the coding sequence by providing the recognition for RNA polymerase and other factors required for proper transcription. The term "promoter" includes a minimal promoter that is a short DNA sequence comprised usually of a TATA-box and other sequences that serve to specify the site of transcription initiation, to which regulatory elements are added for control of expression. "Promoter" also refers to a nucleotide sequence that includes a minimal promoter plus regulatory elements that are capable of controlling the expression of a coding sequence or functional RNA. This type of promoter sequence consists of proximal and more distal upstream elements, the latter elements often referred to as enhancers. Accordingly, an "enhancer" is a DNA sequence which can stimulate promoter activity and may be an innate element of the promoter or a heterologous element inserted to enhance the level or tissue specificity of a promoter. It is capable of operating in both orientations (normal or flipped), and is capable of functioning even when moved either upstream or downstream from the promoter. Both enhancers and other upstream promoter elements bind sequence-specific DNA-binding proteins that mediate their effects. Promoters may be derived in their entirety from a native gene, or be composed of different elements derived from different promoters found in nature, or even be comprised of synthetic DNA segments. A promoter may also contain DNA sequences that are involved in the binding of protein factors which control the effectiveness of transcription initiation in response to physiological or developmental conditions. The regulation of transcription also may be dependent on the presence of different chemical agents, hormones, inducers and the like. The person skilled in the art will be readily able to select and assemble the regulatory sequences that are the best suited for the specific application.

Transgenic technology has been widely used for scientific research and several different protocols have been established. The present invention is not limited in any way by the selection of the specific modifications that lead to the production of the Tg rabbit of the invention. Although there are several reports in the art on the limitations of conventional transgenic techniques, levels of transgene expression vary between lines (Palmiter et al., 1984) and sometimes even between animals in the same line (Dobie et al., 1996; Sutherland et al., 2000), particularly when cDNAs rather than genomic fragments are used. The variegated transgene expression depends on the integration sites of the transgenes into the host genomes and compromises transgene expression (Opsahl et al., 2003). The limitations of plasmid based transgene microinjection can be overcome by using cloning systems which accommodate submegabase DNA such as YACs (yeast artificial chromosome), BACs (bacterial artificial chromosome) or PAC (P1 phage artificial chromosome). These techniques are well established in the generation of Tg mice (reviewed by (Giraldo and Montoliu, 2001). The skilled person will be able to carry out the necessary steps for the production and characterization of the Tg rabbit of the present invention based on the state of the art and the teaching provided herein.

In an aspect, the genetic construct according to the disclosure comprises the insert of the bovine BAC clone #128E04. The BAC clone #128E04 is a clone from a bovine BAC library made from DNA from the genital ridge of a male foetus from a high ranking Holstein bull (obtained from the INRA bovine BAC library, (Eggen et al., 2001). The insert of clone #128E04 is defined as a segment of chromosome 18 of the bovine genome between nucleotide positions 53543852 and 53652024 as presented by the NCBI Map Viewer, *Bos taurus* (cow) Build 3.1 (based on Btau 3.1) current as on August 11, 2007.

In a preferred embodiment, the genetic construct according to the disclosure comprises the insert of the rabbit BAC clone #262E02. The BAC clone #262E02 was isolated from a rabbit BAC library ((Rogel-Gaillard et al., 2001). The BAC library was constructed in the pBeIoBAC11 vector, the high molecular weight DNA was prepared from white blood cells of a New Zealand rabbit. The rabbit BAC library is handled by the INRA resource centre for domestic animals and is publicly available.

The specific modification present in the Tg rabbit of the invention provides for the overexpression of a gene encoding the α-chain of a protein with FcRn activity. As used herein, the term "overexpression" refers to expression levels that are higher than expected from the two genomic copies of the gene of interest in the given species. Overexpression can be assessed at several levels of the biochemical processes, for example at the level of transcription, translation, post-translational-modifications, etc., as long as the elevated expression level results in detectable changes in FcRn function compared to basic level expression in wild type animal. For example, the protein encoded for by a gene of interest can be expressed in increased quantity, as in several examples of the present invention, the α-chain of a protein with FcRn activity is synthesized at elevated levels.

Overexpression can be achieved by several different means that are well known for the person skilled in the art of molecular biology. Exemplary ways of overexpressing a gene of interest are increasing the number of copies of the gene, or increasing the binding strength of the promoter region, or upregulating of enhancer elements, or conversely, inhibiting or blocking repressor elements, and the like.

In preferred embodiments of the present invention, overexpression is independent of the site of integration and dependent on the number of integrated copies. However, the skilled person in the art will be able to determine when it is advantageous or necessary to use any of the available genetic elements to achieve limited expression of the transgene. Examples of inducible regulatory elements, as well as tissue, organ, development stage etc. dependent regulatory elements allowing fine tuned expression of the gene of interest are abundant in the prior art and at the disposal of the person skilled in the art to enable him to reduce the invention to practice.

In further preferred embodiments of the present invention, overexpression can be achieved by integrating more than one functional copy of the gene of interest into the genome of the Tg rabbit of the invention. Preferably, DNA fragments containing an FcRn α-chain gene are isolated from animals. Such large DNA fragments can be isolated by screening a library of cosmids, YACs or BACs, and the like, prepared from the genomic DNA of the rabbit. YAC clones can carry DNA fragments of up to 2 megabases, BAC clones are capable of carrying DNA fragments of smaller sizes (about 150 - 250 kb). The source animal can be of any species, for example one that have significant role in commercial polyclonal or monoclonal antibody production, such as mouse, rat, rabbit, sheep, goat, cattle, swine, donkey and horse. It is clear that the selection of the source of the transgene is not limiting, and the suitability of the gene in the methods according to the present disclosure can be ascertained by the person skilled in the art based on the teaching provided herein.

Procedures for introducing the transgene into the recipient animal and for the selection of Tg rabbits are well known for the skilled person. Briefly, transgenic vectors carrying an FcRn α-chain gene are introduced into the recipient cell or cells and then integrated into the genome of the recipient cell or cells by random integration or by targeted integration. For random integration, a transgenic vector containing an FcRn locus can be introduced into an rabbit recipient cell by standard transgenic technology. For example, a transgenic vector can be directly injected into the pronucleus of a fertilized oocyte. A transgenic vector can also be introduced by co-incubation of sperm with the transgenic vector before fertilization of the oocyte. Transgenic rabbits can be developed from fertilized oocytes.

Another way to introduce a transgenic vector is lentiviral transgenesis. This recently developed method - though it is restricted in term of the transgene size - was proved to be highly efficient in creating Tg animals in a number of species e.g. mice, rat, pig and it is especially promising tool in developing gene therapy strategies (Pfeifer, 2006). Another way to introduce a transgenic vector is by transfecting embryonic stem cells and subsequently injecting the genetically modified embryonic stem cells into developing embryos. Ultimately, chimeric Tg rabbits are produced from the embryos that contain the FcRn transgene integrated in the genome of at least some somatic cells of the Tg rabbit. For targeted integration, a transgenic vector can be introduced into appropriate rabbit recipient cells such as embryonic stem cells or already differentiated somatic cells.

In specific embodiments, the integration of the transgene can result in the loss of the corresponding endogenous FcRn α-chain locus by using homologous recombination procedures. Alternatively, the native FcRn locus may be knocked out independently from the introduction of the transgene. For example, the desired animal can be obtained by classic breeding and mating practices using parents with knocked out genotypes. However, the replacement of the endogenous FcRn locus is not necessary to achieve the object of the invention. The skilled person in the art will be able to determine if the presence of the endogenous gene is detrimental to the elevated immunoglobulins production. However, when multiple copies of the gene encoding the α-chain of a protein with FcRn activity are inserted for achieving overexpression of the gene, under normal circumstances no need is envisaged for such replacement strategy.

If there is a need to enrich a specific IgG isotype that binds tighter to the FcRn from an exogenous FcRn α-chain rather than its endogenous counterparts then it is preferable to delete (knock out) the endogenous FcRn and/or replace it by exogenous FcRn α-chain. An important example is the overproduction of the human IgG in Tg rabbits that carry human chromosomal DNA encoding the heavy and light chain of the human immunoglobulin genes. It is predictable that in animals overexpressing e.g. the bovine FcRn α-chain and deleting the host FcRn α-chain is beneficiary for enriching the human IgG at the expense of the IgG of the host that binds weaker to the exogenous FcRn α-chain and hence clears faster from the animal. A similar example is when one of the host IgG isotypes is preferred to enrich at the expense of other IgG isotypes.

The selected cells may then be fused with enucleated nuclear transfer unit cells, e.g. oocytes. Fusion is performed in accordance with conventional techniques, which are well established in the art (see, for example, (Cibelli et al., 1998). Enucleation of oocytes and nuclear transfer can also be performed by microsurgery using injection pipettes (see, for example, (Wakayama et al., 1998)). The resulting egg cells are then cultivated in an appropriate medium, and transferred into synchronized recipients for generating Tg rabbits. Alternatively, the selected genetically modified embryonic stem cells can be injected into developing embryos that are subsequently developed into chimeric animals.

A preferred embodiment of the present invention is where the Tg rabbit harbors multiple copies of the transgene. Theoretically, there is no limit envisaged with respect to the copy number of the transgene introduced into the Tg rabbit of the invention. The person skilled in the art will be readily able to determine if the overexpression of the gene encoding the α-chain of a protein with FcRn activity provides relatively high FcRn expression to afford the advantageous effects of the invention but have not riskily intense expression of the gene that would impair cellular homeostasis and functionality. In the examples presented herein, no phenotypic modifications were observed among the different Tg lines of animals harboring up to 10 copies of the complete bFcRn gene (Bender et al., 2007). In addition, Lu et al. (Lu et al., 2007) presented data for Tg lines harboring up to 15 copies of the bFcRn cDNA under a mammary gland specific promoter, without significant phenotypic modifications.

As used herein, the term "FcRn activity" is used to denote a series of events taking place *in vivo.* As it was already discussed during the description of the state of the art, FcRn has been first identified in rodents as the receptor that transfers maternal immunoglobulins from mother to newborn via the neonatal intestine, and then studies have shown that FcRn plays a central role in regulating the transport of IgG within and across cells of diverse origin. Within the context of the present description, the term "FcRn activity" mainly refers to the rescue of IgG from degradation. Accordingly, activity of FcRn, as used herein, is defined as the ability to bind the IgG-Fc and protect IgG from degradation.

In addition, as first disclosed herein, the term "FcRn activity" as used herein also refers to the ability of FcRn to enhance the humoral immune response, more specifically to enhance the antigen specific clonal B-cell expansion and, consequently, IgM and IgG synthesis.

FcRn activity, for example, can be determined at one or more steps of the IgG rescue process, the mechanism of which is thought to be mediated mainly by endothelial cells that line blood vessels. Inside these cells, FcRn predominantly resides in early/recycling endosomes, where it encounters IgG internalized by fluid phase endocytosis. The acidic environment of the endosomes facilitates the interaction. Bound IgG and albumin are recycled back to the surface and released from the cell, while unbound ligands are shuttled downstream to lysosomal degradation.

Some of the steps, however, can be modeled and defined independently from the overall meaning of the term.

The binding ability of an IgG of interest or molecules comprising an IgG constant domain or Fc fragment thereof to FcRn can be characterized by various *in vitro* assays. WO 97/34631 discloses various methods in detail and is incorporated herein in its entirety by reference.

One method for the determination of binding between IgG and FcRn is an *in vitro* assay that utilizes an isolated complex of FcRn and β2m retaining the *in vivo* function of binding IgG-Fc at about pH 6, with release of the bound IgG-Fc occurring upon shift in the pH to about pH 7.2. The term "about", as used herein with reference to pH, refers to ±0.2 pH value. The FcRn/β2m complex is bound to a solid support such as a microliter well, filter, membrane, column, or beads. Commonly used materials for solid support are nylon, polystyrene, polypropylene, and agarose. Contacting of molecular components is generally achieved by adding the components together in aqueous solution, usually appropriately buffered and allowing the components to react with each other for a predetermined amount of time. Depending on the assay setup, washing steps may be necessary between the additions of different components. These components serve as the determination of the binding taking place between the components, and can be set up according to several well-known assay format, such as in competitive binding assays, direct binding assays, sandwich assays, and the like. After all the necessary binding steps took place, the detection is made by techniques usually employed in the art, for example by the detection of a signal that can be radioactive, enzymatic, fluorescence, or other well-known signal. The measured signal is either proportional to the measured signal (such as in direct binding assays) or competitive.

The generalized method for determining binding between IgG and FcRn can be suitably adapted to identify the appropriate FcRn α-chain and/or β2m pair that forms the ideal FcRn heterodimer binding to the IgG of interest. In this case, the FcRn α-chain derived from different species or the result of mutation and a β2m that binds with the highest affinity to the IgG of interest are used in the test. First an IgG known to be bound by FcRn (e.g., human IgG to bFcRn) is used under conditions appropriate for binding. This initial setup is followed by experiments in which the original FcRn α-chain will be replaced by the FcRn α-chain of a different species or generated by in vitro mutagenesis and binding to IgG in question will then be assayed. The best FcRn α-chain can be identified by comparing binding affinity of the complex to the original binding complex. Similar method could be used to find the best possible β2m to be paired with a predetermined FcRn α-chain. Alternatively, or in addition, high throughput techniques may be employed to test simultaneously several pairs of FcRn α-chain and β2m combinations. The isolated complex of FcRn and β2m, which retains the in vivo function of IgG-Fc binding, is preferably produced by *in vitro* synthesis from engineered nucleic acids encoding the respective proteins. The proteins may be synthesized separately, and then added together to produce the complex. DNA segments encoding such proteins may be incorporated into a recombinant vector in a position so as to render the vector capable of expressing a protein. Techniques for the manipulation of DNA segments in this manner, for example, by genetic engineering using restriction endonucleases, will be known to those of skill in the art in light of both the present disclosure and references such as (Ausubel et al., 1998). Those of skill in the art recognize that additional methods exist which may be used to produce a complex suitable for the *in vitro* method described directly above. Alternatively, endogenous complex components can be isolated from an appropriate cellular source.

Affinity of an IgG of interest to FcRn can be measured by surface plasmon resonance (SPR) measurement using, for example, a BIAcore 2000 (BIAcore Inc.) as described previously (Karlsson et al., 1991; Popov et al., 1996). In this method, FcRn molecules are coupled to a BIAcore sensor chip (e.g., CM5 chip by Pharmacia) and the binding of IgG in request to the immobilized FcRn is measured at a certain flow rate to obtain sensorgrams using BIA evaluation 2.1 software, based on which on- and off-rates of the IgG in request, constant domains, or fragments thereof, to FcRn can be calculated.

Relative affinities of IgG of interest or fragments thereof for FcRn can be also measured by for example with a competitive binding assay. The IgG of interest is added in different amounts to the wells of a 96-well plate in which FcRn is immobilized. A constant amount of radio-labeled IgG of interest is then added to each well. Percent radioactivity of the bound fraction is plotted against the amount of wild type IgG and the relative affinity of it can be calculated from the slope of the curve. Furthermore, affinities of the IgG of interest or fragments thereof for FcRn can be also measured by a saturation study and Scatchard analysis or by other means e.g. non-linear regression (curve fit) calculations.

Transfer of IgG in request or fragments thereof across the cell by FcRn can be measured by *in vitro* transfer assay using radiolabeled IgG or fragments thereof and FcRn-expressing cells and comparing the radioactivity of the one side of the cell monolayer with that of the other side.

Another assay to identify FcRn from protecting IgG *in vivo* is a cell culture assay. Mammalian cells functionally expressing FcRn in culture are either generated or identified from preexisting mammalian cells. Cells suitable for use in this assay are capable of catabolizing IgG, with expression of FcRn in these cells causing a decrease in the catabolism. Functional expression of FcRn as referred to in the context of an *in vivo* assay indicates that the FcRn α-chain complexes with β2m binds to an IgG of interest and protects the bound IgG from degradation. To identify the appropriate FcRn α-chain, a series of cells expressing different FcRn α-chains and IgG of interest is contacted with the cells. The cells are incubated under conditions appropriate and conducive to normal cell function, and are then assayed for IgG catabolism. A substantial decrease in IgG catabolism in the cells expressing FcRn compared to IgG catabolism in control cells that do not express FcRn is an indication that the candidate FcRn α-chain protects IgG from degradation. This assay detects FcRn function via binding to IgG, internalization and protection. Cell lines known in the art, and also primary cells of the species that is selected to produce antibody upon immunization, may be used for the cell culture assay. This assay is crucial to test those hybrid FcRn α-chain molecules that are composed of an extracellular part superior in IgG binding and a transmembrane and cytoplasmic segment that is specific for the species that will be used for immunization.

As mentioned with respect to the description of the state of the art, FcRn is a heterodimer molecule composed of an MHC class-1 like α-chain and the β2-microglobulin (β2m). The FcRn α-chain (approved symbol: FCGRT, approved name: Fc receptor, IgG, alpha chain transporter, Fc fragment immunoglobulin G receptor; neonatal Fc receptor, FcRn alpha chain) gives the molecule its specific characteristics, while the β2m is a ubiquitous component present in several different protein complexes. It is obvious for the skilled person in the art that for the methods of the present invention, both subunits of the heterodimer must be present in adequate quantities. The defining feature for producing elevated levels of immunoglobulins according to the invention is the overexpression of a gene encoding the α-chain of the FcRn molecule, as described above. However, it is clear that the equimolar amounts of the other chain, the β2m is necessary to produce functional FcRn. Therefore, for the practice of the invention the skilled person should take into account the cellular availability of β2m. The correlation between the expected levels of FcRn heterodimer based on the genetic modifications made to the Tg rabbit and the measured FcRn activity should be indicative of any problems arising from insufficient supply of β2m. Methods for assessing both factors are available for the skilled person in the prior art and in the present description. Similarly, when it is necessary, the level of β2m can be increased by the skilled person, for example by using state of the art transgenic methodologies. In specific embodiments, the present invention may be further enhanced by providing Tg animals overexpressing simultaneously both the FcRn α-chain and the β2-microglobulin chain from and/or by the same or different genetic constructs.

As it is clear from the section above discussing the activity of FcRn, the composition of the heterodimer can be a significant factor in determining its activity. Accordingly, the present invention can provide any desired combination of the α-chain and the β2m to achieve the increased level of immunoglobulins in the serum of the Tg rabbit according to the invention. The implications of the binding characteristics and specific affinity of FcRn are detailed above; however, these properties of the FcRn heterodimer are also determined by its subunit composition. As it was discussed in the description of the prior art, the strength of the binding between the bovine FcRn and the human IgG is higher than between human FcRn and the human IgG. It is therefore a very important aspect of the present invention to provide FcRn molecules with superior binding characteristics to that of the native FcRn molecule of the Tg rabbit. In particular, this goal can be achieved by testing and selecting the best possible combination of the α-chain and the β2m of the FcRn for the production of immunoglobulins in the Tg rabbit of the invention. This can be achieved by introducing either a suitable α-chain or a β2m from a different or the same animal species. The skilled person in the art will be able to carry out the necessary modifications in the Tg rabbit of the invention after making the determination of the binding characteristics as detailed herein. Accordingly, when the present specification refers to the Tg rabbit of the invention, it is intended to mean a rabbit that not only incorporates a gene encoding the α-chain of a protein with FcRn activity, but if necessary, the gene encoding the α-chain of a protein with FcRn activity and the gene encoding β2-microglobulin.

It is also within the scope of the present invention to achieve the enhancement of the FcRn activity by introducing into a transgenic rabbit according to the invention a genetic construct encoding β2m. The person skilled in the art will be able to determine the conditions necessary to achieve this enhancement. All considerations discussed with respect to the α-chain of FcRn will apply *mutatis mutandi* to the modifications involving the β2-microglobulin (β2m).

It is also within the scope of the present invention to use mutant versions of either the α-chain or the β2m, as long as the modifications results in a functional FcRn molecule that fulfils the activity requirements as described above. Mutation technologies are well known to person skilled in the art of molecular biology. However, to define the suitable FcRn protein, instead of the activity requirement, the structural homology can be established between the mutant and native protein. It is emphasized here that the structural homology is an available, but not limiting feature to characterize the transgene according to the invention. The α-chain of the FcRn protein may have a sequence identity of approximately 60% or more with the sequence of the bovine FcRn protein as used in the present teaching of the invention. The terms "sequence identity", "homology" and "variant" are used interchangeably in the present specification. When one amino acid is said to be homologous to another, it means that one of the amino acid sequences in question is having at least 60%, preferably at least 70%, more preferably at least 80%, even more preferably at least 90%, and still more preferably at least 95% sequence identity with the other sequence. To determine whether the sequences are, say, at least 60% identical, the FastDB program of EMBL or SWISSPROT data bases can be used. Other algorithms and computerized embodiments thereof well known in the art may also be used for the determination of this homology.

In a specific embodiment, the mutant version of the α-chain of the FcRn protein is devoid of a functional albumin binding site. As discussed above, the FcRn has two independent binding sites for immunoglobulins and albumin. Albumin is a 67 kD molecule that constitutes two-thirds of the protein mass of serum. It transports a great assortment of molecules such as fatty acids, bile acids, eicosanoids, vitamins, hormones, ions, toxins, and drugs. As well albumin endows blood with most of its colloid osmotic pressure and is the major pH buffering protein of serum. In normal condition, the total IgG level is in the range of 10-15 mg/ml (Manz et al., 2005) and the albumin level is 40-48 mg/ml (Beers and Berkow, 1999). About 75 percent of the total colloid osmotic pressure of the plasma results from the albumin fraction, 25 percent from the globulins (mostly IgG) (Guyton, 1991). Since, the molecular weight of the IgG is almost three times as big as albumin (150-160 versus 67 kDa), based on a simple calculation of their molecular ratio (which counts in the colloid osmotic pressure), the increase of three units of IgG is identical to the increase of one unit albumin, regarding the plasma oncotic pressure (which is the pressure attributed to the protein fraction of the serum). Therefore, overexpression of the FcRn for the purpose of generating more antigen specific IgG may be advantegous, however may lead to a rather harmful condition, especially if it coincides with higher than normal level of albumin. As the FcRn binds IgG and albumin at two different sites (Andersen et al., 2006; Chaudhury et al., 2006) and a recent study demonstrated that mutational deletion of the albumin binding site in the FcRn α-chain did not lead to the loss of IgG binding in vitro (Andersen et al., 2006), it can be concluded that there is no competition between the two ligands.

Even in lack of said competition effect, depending of the copy number of the FcRn present in the Tg rabbit according to the invention, the albumin level of the blood serum might increase to a level so high that can cause problems for the rabbit. Accordingly, the rabbit harboring the mutant FcRn with non-functional albumin binding site will be able to function better according to the invention to produce useful antibodies. The type of the specific mutation causing the malfunctioning albumin binding site will be readily determined by the person skilled in the art, as well as he/she will be able to carry out the necessary molecular biological and other steps to create the Tg rabbit according to the invention without undue experimental burden.

As already mentioned herein, the overall health of the rabbit according to the present invention is very important in order to practice the invention in an economically viable way. The Tg animals as presented herein were shown to live for more than a year without pathological symptoms.

By a non-claimed method a Tg animal is utilized that harbors a protein with FcRn activity that comprises a chimeric α-chain comprising the endogenous intracellular domain of the FcRn protein of the Tg animal in combination with an extracellular domain of foreign origin. The extracellular domain component is selected as described above. In this embodiment the chimeric α-chain could help overcome those situations when the protein encoded by the transgene would not function correctly because its intracellular signaling properties are not compatible with that of the recipient animal. This would render the FcRn heterodimer non-functional; however, the superior immunoglobulin binding capability of the foreign receptor makes it worthwhile to utilize such a chimera. The person skilled in the art will be readily able to apply the state of the art genetic engineering methodologies to generate the chimeric receptor according to this embodiment.

In another preferred embodiment of the present invention, the Tg rabbit of the invention is made from a rabbit that is already transgenic for producing immunoglobulins that are human or humanized. The art recognized the importance of producing human or humanized immunoglobulins in Tg animals, and such animals of different species have been generated recently. One technology that has been explored to generate low immunogenicity mAbs for *in vivo* human therapy involves the use of Tg mice expressing repertoires of human antibody gene sequences. In the future, it may be possible to extend this technology beyond rodents and use Tg farm animals (e.g. cattle, rabbit) to directly generate and produce human sequence polyclonal sera (Lonberg, 2005). US2006117395 describes the production of Tg cattle which comprises a genetic modification that results in inactivation and loss of expression of its endogenous antibodies, and the expression of xenogenous antibodies, preferably human antibodies. This is effected by inactivation of the bovine IgM heavy chain expression and, optionally, by inactivation of the bovine Ig light chain expression, and by the further introduction of an artificial chromosome which results in the expression of non-bovine antibodies, preferably human antibodies. US20070033661 discloses another approach to increase immunoglobulin expression in non-human transgenic animals by overexpressing the apoptosis inhibitor, whose expression is driven by a B-cell specific promoter specifically in the B-cell of the animal, thereby enhancing the survival of B-cells. WO0212437 discloses humanized antibodies produced from Tg non-human animals that are genetically engineered to contain one or more humanized immunoglobulin loci which are capable of undergoing gene rearrangement and gene conversion in the Tg non-human animals to produce diversified humanized immunoglobulins. Tg rabbits expressing humanized antibody repertoire has been reported recently (Thorey et al., 2006).

The present invention provides significant advantages in these Tg rabbits for improved immunoglobulin production. For example, the selection and introduction of the FcRn transgene according to procedures outlined in the present invention could ensure that the immunoglobulin production in these Tg rabbits is optimized by utilizing the most effective FcRn binding partner for the immunoglobulins produced by the recipient animal, either they are endogenous immunoglobulins or transgenically produced human or humanized antibodies. Therefore, it is contemplated by the present invention to use already Tg rabbits producing human or humanized immunoglobulins as a starting point to introduce one ore more copies of FcRn α-chain and provide the advantages of the present invention. Alternatively, a double transgenic rabbit with the advantageous phenotype provided by the present invention could be created by mating a Tg rabbit producing humanized immunoglobulins with a rabbit generated according to the present invention.

In another aspect of the present invention, a method is provided for the production of elevated levels of immunoglobulins in the serum of an animal comprising providing a Tg rabbit as described above and using said rabbit according to any established immunization protocol for immunoglobulin production.

In a further aspect of the present invention, the enhancement of the immune response includes enhancing the antigen specific clonal B cell expansion. The role of the FcRn in modulating the immune response via the B cell clonal expansion and immunoglobulin synthesis is reported herein the first time. This is a striking and unexpected finding. Most of the studies that described the humoral immune status in animals that lacked functional FcRn, either in earlier studies in β2m knock-out mice or more recently in FcRn α-chain knock-out animals, did not show impairment of the IgG synthesis and the low serum IgG levels were defined by the impaired IgG protection in these animals (Junghans and Anderson, 1996; Roopenian et al., 2003). Other studies, however, reported that IgG synthesis was reduced in animals that lacked β2m, however they did not reach to a definitive explanation (Ghetie et al., 1996; Israel et al., 1995).

It is presented herein that 25 days after OVA immunization there were twice as many cells secreting OVA-specific IgM, and three times more cells secreting OVA-specific IgG in Tg mice compared with wt animals. The enhanced antigen specific B-cell clonal expansion can be explained partly by the finding that the spleens of the Tg mice were significantly bigger compared with their wt controls, as it was also observed. A more augmented immune reaction is further supported by the cellular immunological profiles, in which similar changes were found in the proportion of cellular components after immunization in Tg and wt mice, but the Tg mice showed much radical change. In both groups, there was a proportional decrease of cells expressing B220, CD3, parallel with a significant, although moderate increase of CD11b+ / CD11c+/MHC II, and a significantly massive influx of CD11^{high} / Gr- 1^{high} bearing cells, suggesting that the dominant cell population that influxed the spleen during the secondary immune response was neutrophils and, to a lesser extent, macrophages and/or dendritic cells. Accordingly, in another aspect of the present invention, the enhancement of the immune response includes stimulating the influx of neutrophils, macrophages and/or dendritic cells into the secondary lymphoid organs.

Peripheral or secondary lymphoid organs are the sites where adaptive immune responses are initiated and where lymphocytes are maintained. These are the lymph nodes, the spleen, and the mucosal lymphoid organs. Accordingly, in preferred embodiments, the secondary lymphoid organ is the spleen and lymph nodes.

Although FcRn expression was observed in monocytes, macrophages and dendritic cells (Sachs et al., 2006; Stirling et al., 2005; Zhu et al., 2001), its function in these cells are still unclear, and thus the role of FcRn in the antigen presentation, which would have direct consequence to immunoglobulin synthesis, is still unclear. On the other hand, a more recent study revealed that FcRn fulfills a major role in IgG-mediated phagocytosis in polymorphonuclear leukocytes and monocytes (Vidarsson et al., 2006). Since bFcRn expression was detected in neutrophils and macrophages derived from the peritoneum of the Tg mice, it can be hypothesized that FcRn overexpression in these cells mediates enhanced antigen (in immunocomplex form) phagocytosis and even antigen presentation, which then results more antigen specific IgM and IgG producing plasma cells in the secondary lymphoid organs. Should this theory be correct this enhancement was obvious once IgG is produced and not earlier. Indeed, increased IgM titers were only found after the OVA specific IgG appeared, mainly in the secondary immune response. Nevertheless, the analysis of the cellular immunological profile of the spleen showed that the dominant cell type that influxed the spleen after immunization was neutrophils. Based on another recent report (Maletto et al., 2006), neutrophils comprised the main population of cells bearing the antigen in lymphoid organs, once there was a specific immune response occurring and IgG-antigen immunocomplex was formed. It was also showed that the neutrophils in secondary lymphoid organs mainly expressed TNF-alpha and contributed to the quality of the established secondary immune response. This finding gives a plausible explanation why there is a remarkable influx of CD11b^{high} and Gr-1^{high} bearing cells in the inventors' wt and Tg mice. Considering that Tg mice produced much more OVA specific IgG and overexpress FcRn in these cells, it can also be explained why Tg mice show greater influx of these cells into the spleen, larger antigen specific B cell clonal expansion and consequently a more robust antibody response compared to their wild type controls.

An important aspect of the present invention that the Tg rabbit according to the invention comprising a genetic construct providing for the expression of a nucleic acid sequence encoding the α-chain of the rabbit FcRn protein, wherein said genetic construct provides for enhanced FcRn activity, can be used in a non-therapeutical method in which the same level of humoral immune response is developed upon fewer number of antigen challenges as compared to a non-transgenic control animal of the same species. This feature of the present invention is very advantageous when large quantities of antisera are needed at the same time and a large number of animals are immunized in parallel, with sometimes expensive antigens. The cost saving may be substantial even when only one booster injection can be eliminated.

Another important aspect of the present invention that the Tg rabbit according to the invention comprising a genetic construct providing for the expression of a nucleic acid sequence encoding the α-chain of the rabbit FcRn protein, wherein said genetic construct provides for enhanced FcRn activity, can be used in a non-therapeutical method in which the same level of humoral immune response is developed faster as compared to a non-transgenic control animal of the same species. The person skilled in the art will be readily able to adapt any suitable immunization protocol when using the animal according to the invention, and determine the preferred time to harvest the antibodies produced. When the animal used for antibody production is used repeatedly (such as goat, sheep, other large animals), this feature of the invention will lead to improved cycle times for harvesting the antisera, the commercial implications of which are evident. When the animal is sacrificed at the end of the protocol (such as rabbits) the commercial advantage is even bigger: an antibody-producer specialized in providing custom antisera for antigens supplied by the customer will be able to provide the requested antiserum faster, thus enabling the customer to be in a position that is desired in today's world where scientific developments are accelerated and competition abounds.

In another non-claimed aspect, a method is provided for producing albumin, comprising using a Tg animal comprising a genetic construct providing for enhanced FcRn activity in accordance with any established protocol enabling the production of albumin. As discussed above, the FcRn has two independent binding sites for immunoglobulins and albumin. Although in this case no competition is present between the two ligands, the person skilled in the art will readily recognize that the Tg animal according to the invention is useful when increased production of albumin is desired. When the albumin level is too high, however, it might cause harmful effects to the health of the animal. Accordingly, care should be taken when the animal according to the invention is used for albumin production, especially in respect of the copy number of functional FcRn genes present in the animal.

In a related embodiment, the genetic construct carried by the Tg rabbit according to the invention provides for the overexpression of a gene encoding a mutant version of the α-chain of the FcRn protein, in which the immunoglobulin binding is eliminated. Accordingly, the Tg rabbit harboring the mutant FcRn with non-functional immunoglobulins binding site will be able to function better according to the invention to produce more albumin. The type of the specific mutation causing the malfunctioning immunoglobulin binding site will be readily determined by the person skilled in the art, as well as he/she will be able to carry out the necessary molecular biological and other step to create the Tg rabbit according to the invention without undue experimental burden.

In a further aspect of the invention, an animal propagation material is provided, obtained from a Tg rabbit according to the invention or from a Tg rabbit obtained by the method according to the invention, comprising a genetic construct providing for the expression of a nucleic acid sequence encoding the α-chain of the rabbit FcRn protein, wherein said genetic construct provides for enhanced FcRn activity. As used herein, the term "animal propagation material" refers to any biological material derived from an animal that allows the skilled person to generate another animal which has substantially the same genetic composition. The animal propagation material is preferably selected from the group consisting of sperm, spermatogonium, ovum, ovarian tissue, embryo or tissue sample for somatic cloning.

In another non-claimed aspect, a method is provided for enhancing the humoral immune response of a model animal useful for performing tests concerning conditions associated with an altered immune response comprising the step of introducing to said animal a genetic construct providing for enhanced FcRn activity. In preferedd embodiments, the Tg animal provided is suitable as a model animal for autoimmune diseases. Based on the discussion herein, it is clear that the animal with enhanced FcRn activity will have an increased overall serum immunoglobulin level, which in turn will maintain the diseased state leading to the autoimmune condition at a higher level and/or for an elongated period of time. Such an animal will be useful for testing compounds for the treatment of such autoimmune conditions, or for any other purposes related thereto.

### Brief description of the figures

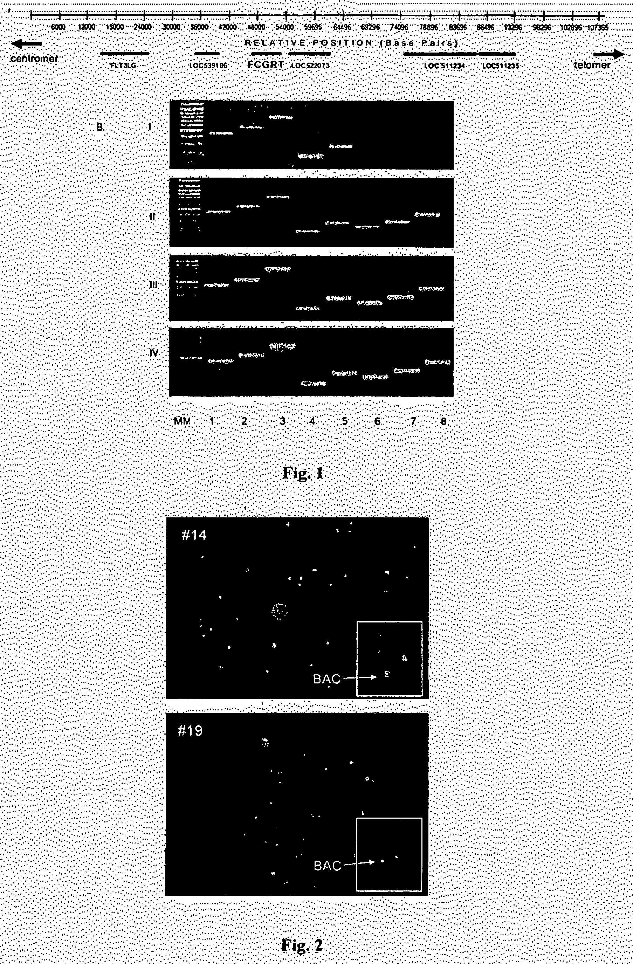

**Figure 1****.** Structure and characterization of the 128E04 bovine BAC transgene A: Schematic representation of the 128E04 BAC genomic fragment with the relative positions of bFCGRT and the five putative protein encoding genes: FLT3LG, LOC539196 and the LOC522073, LOC511234, LOC511235. B: Intactness of the integrated transgene was detected by PCR. I: genomic DNA from transgenic line #9; II: genomic DNA from transgenic line #14; III: genomic DNA from transgenic line #19 and IV: control BAC 128E04 genomic DNA templates. Slots: MM: 1kb ladder, 1: BAC 128E04 5'-end, 2: FLT3LG, 3: LOC539196, 4: FCGRT, 5: LOC522073, 6: LOC511234, 7: LOC511235, 8: BAC 128E04 3'-end specific PCRs. Note that LOC511234, LOC511235 and the 3' end specific fragments had not been amplified from transgenic line #9 genomic DNA.
**Figure 2****.** Metaphase spreads from fibroblasts derived from transgenic lines #14 and #19. FISH analysis revealed, that the fluorescently labeled BAC 128E04 clone, carrying the bFCGRT, hybridized to entirely different chromosomal segments in the #14 and #19 mouse fibroblasts respectively, which excludes the possibility that the phenotype of the transgenic mouse strains #14 and #19 is integration site dependent.

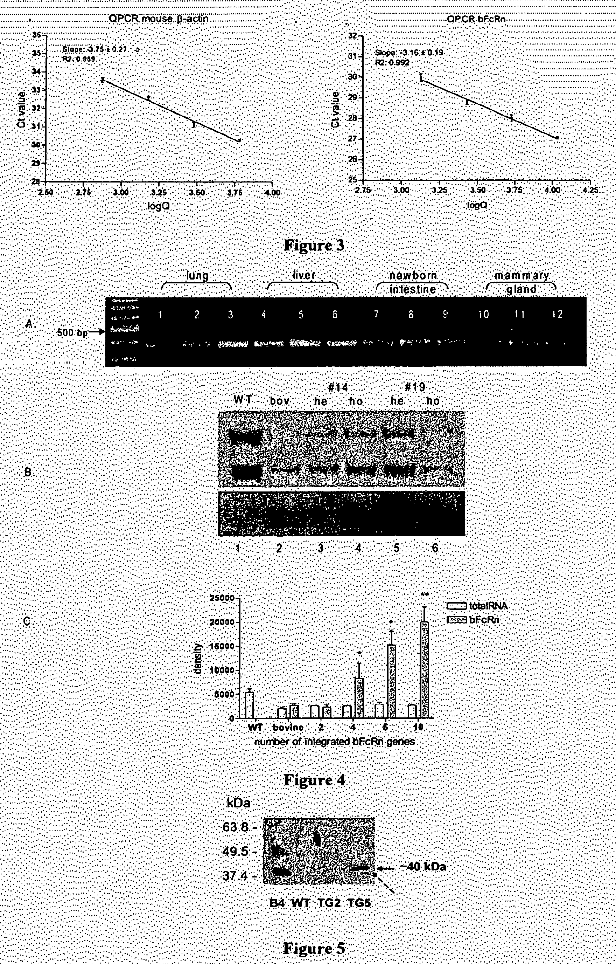

**Figure 3****.** Standard curves of Ct versus log copy number (Q) in case of the mouse β-action and bFcRn β-chain (bFCGRT), respectively, in order to determine the absolute quantity of the bFcRn copy number in Tg mice (line #14 and #19) using quantitative real-time PCR. Ct is the cycle at which fluorescence crosses a threshold value using quantitative real-time PCR. Detection was performed using fluorogenic 5k nuclease technology (TaqMan, (Lee et al., 1993) on an ABI Prism7000 Sequence Detection System (Applied Biosystems Foster City CA, USA).
**Figure 4****.** Transgene copy number dependent bFcRn specific mRNA expression. **A:** RT-PCR from transgenic lines #9 (1,4,7,10), #14 (2,5,8,11), #19 (3,6,9.12) hemizygote tissue samples respectively. Slots: MW: 1kb ladder, 1-3 lung; 4-6 liver; 7-9 newborn intestine; 10-12 mammary gland. **B:** Northern analysis to detect bFcRn α-chain mRNA expression in the liver total RNA samples. Slot 1: wild type mouse; 2: bovine, 3-4: hemi- and homozygote mice of line #14 correspond to two and four copies of the bFcRn transgene; 5-6: hemi- and homozygote mice of line #19 correspond to five and ten copies of the bFcRn transgene. **C:** Quantitative evaluation of the transgene copy number dependent expression of bFcRn as detected by Northern analysis. Columns represent optical density (mean), while error bars represent the standard error of the mean. Statistical significance is indicated as follows: *, p < 0.05; **, p < 0.01.
Figure 5. bFcRn copy number dependent protein level in the lung of wt and Tg mice. Western blot of total cellular protein (30 µg/lane) by using affinity purified rabbit antiserum (B4 - the bFcRn α-chain stable transfected MAC-T cell line extract (Kacskovics et al., 2006a); WT - wild type mouse; TG2 - hemizygous, mouse, line #14; TG5 - hemizygous mouse, line#19). The molecular weight markers in kilodaltons are indicated on the left. The bFcRn α-chain specific affinity purified serum (Mayer et al., 2002) detected an approximately 40 kDa band in transgenic lung tissue samples similar to that in the bFcRn transfected (B4) positive control cell extract. The second band (dashed arrow) possibly represents a less abundant non-glycosylated form of the receptor. Comparing the amount of the recombinant FcRn α-chain in TG2 and TG5, which corresponds to two and five transgene copies, respectively, reveals that copy number dependent expression is obvious not only at mRNA level but also at protein level.

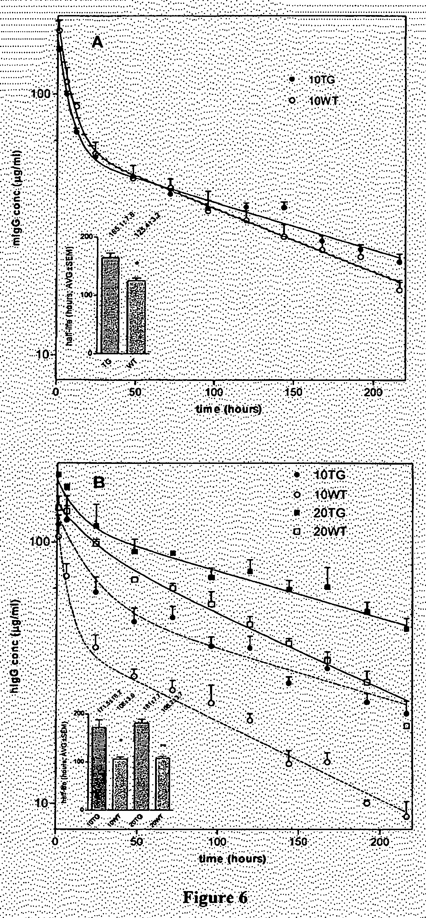

**Figure 6****.** Pharmacokinetics of mouse (**A**) and human (**B**) IgGs in bFcRn Tg (line #14, homozygote, carries 4 copies of bFcRn) and wt mice. The modeled data (simulated based on the geometric mean of the primary pharmacokinetic parameters) as well as the observed mean serum antibody concentration (µg/ml) for the three to five animals (young adult littermates) were plotted as a function of time (hours after injection) of mouse (10 mg/BW_{kg}) and human (10 and 20 mg/BW_{kg}) IgGs. Inserts show the half-life values of the injected IgGs that were calculated with WinNonLin professional software applying the two compartmental model. IgG half-lives in Tg animals were significantly longer compared to the wt mice. Samples were assayed in duplicate and error bars represent standard error of the mean (SEM). Statistical significance is indicated as follows: *, p < 0.05; **, p < 0.01.

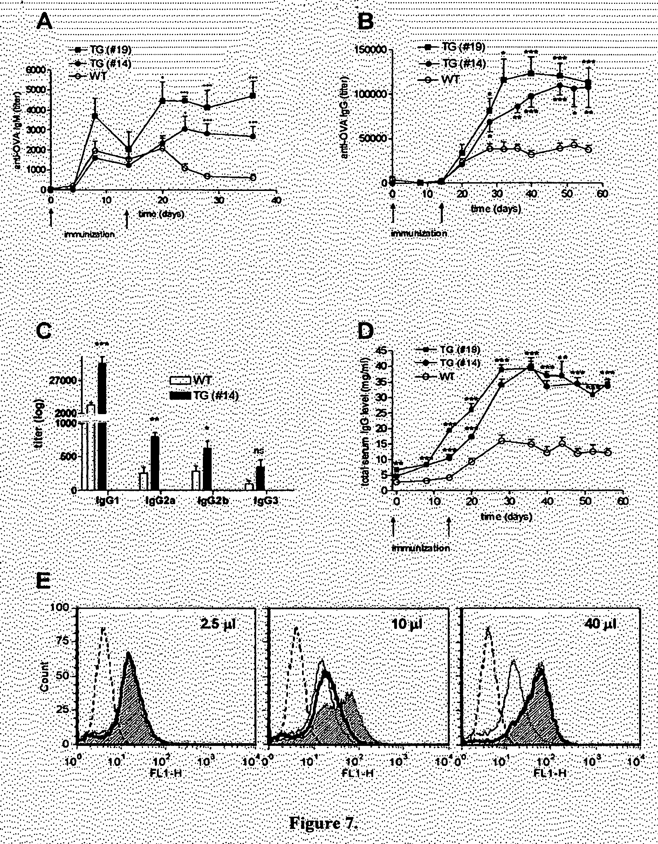

**Figure 7****.** bFcRn overexpression results a robust augmentation of the immune response in Tg mice, without disturbing its nature. Tg and wt mice were immunized i.p. by OVA in CFA and challenged 14 days later with OVA in IFA. Serially sampled sera were analyzed for OVA specific IgM, and IgG. In case of Tg mice the IgM titers were higher in the secondary immune response compared to the IgM levels in the primary immune response, and significantly higher compared to that of the wt mice. The significance levels indicate the difference between the Tg and wt mice (**A**). OVA-specific IgG titers were nearly tripled in Tg mice compared to that of wt animals during the secondary immune response. Significance levels indicate the difference between the Tg and wt mice (**B**). At 32 days of immunization titers of the OVA specific IgG isotypes were analyzed. Tg mice produced significantly higher titers of the IgG isotypes (except IgG3), nevertheless the proportion of them were not different compared to that of the wt mice, indicating that bFcRn overexpression increased of the OVA specific IgG production without disturbing it (**C**). Total IgG production reflected OVA-specific IgG titers, Tg mice produced significantly higher amount of IgG compared to their wt siblings. Noteworthy, is that they showed higher levels of IgG even before of immunization (**D**). Values shown are the mean ± SEM. *, p < 0.05; **, p < 0.01; ***, p < 0.001; ns, p > 0.05.
   The efficiency of FITC-OVA immunocomplex uptake was analyzed by FACS (**E**). Black dotted line indicates the autofluorescency of the P388 cells, gray line indicates uptake of non-immunocomplexed OVA-FITC; black line indicates uptake of OVA-FITC immunocomplex prepared from wt sera, while grey filled area represents uptake of OVA-FITC immunocomplex prepared from Tg sera. We found no enhanced phagocytosis by using 2.5 µl sera compared to OVA-FITC uptake (**2.5 µl**). However, a significant enhancement was observed by using 10 µl sera from Tg mice, while there was no phagocytosis augmentation at that amount from wt mice (**10 µl**). Finally, 40 µl sera from Tg and wt mice resulted equally increased phagocytosis compared to the non-immunocomplex form of OVA-FITC (**40 µl**).

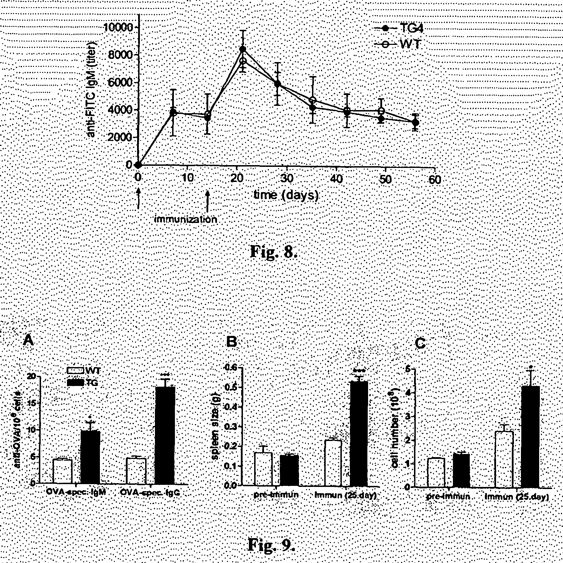

**Figure 8****.** Tg and wt mice responded similarly to FITC-dextran immunization. Tg (line #14) and wt mice were intraperitoneally immunized with FITC-dextran and challenged them similarly two weeks later. We observed primarily FITC specific IgM, with no difference between the Tg and wt animals. Analyzing the immune response during the immunization course we noted an increased IgM production after the second immunization both in the Tg and wt mice.
**Figure 9****.** Spleen cells from mice (three male animals in each group) immunized with OVA+CFA, challenged on days 14 with OVA+IFA, and harvested on day 25. OVA-specific IgM producer cells were doubled, while OVA specific IgG cells were more than tripled in the spleen of the Tg mice compared to that of the wt animals (**A**). 25 days after OVA immunization there was an increase in spleen weight (**B**) and cell numbers (**C**) both in the wt and Tg mice, however, spleen size and cell numbers were significantly greater in Tg compared with wt mice. Values shown are the mean ± SD (*, p<0.05; ***, p < 0.01; p < 0.001).

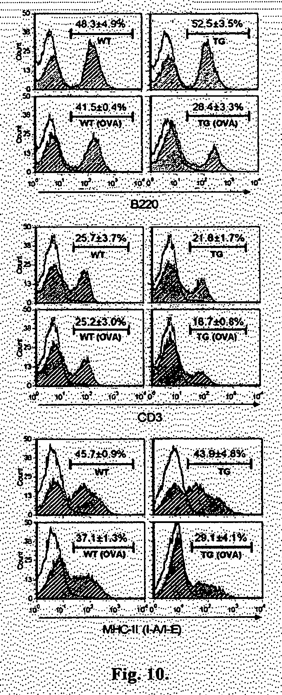

**Figure 10****.** Analysis of the cellular distribution of the spleen in normal and OVA immunized (25 days after immunization) wt and Tg mice. We found that after immunization the ratios of cells bearing CD45R/B220 (B lymphocytes) and I-A/I-E (MHC class II) antigens significantly reduced (p<0.05). There was not a difference or a less radical decrease regarding the cells bearing CD3 marker (T-lymphocyte) in wt and Tg mice, respectively. The histograms show the percentage of cells (mean ± SD) bearing CD45R/B220 (B lymphocytes), CD3 (T-lymphocytes) and I-A/I-E (MHC class II) antigens (dashed area) or isotype specific controls (black line). One typical experiment of 2 performed is shown (n = 3 mice per group).

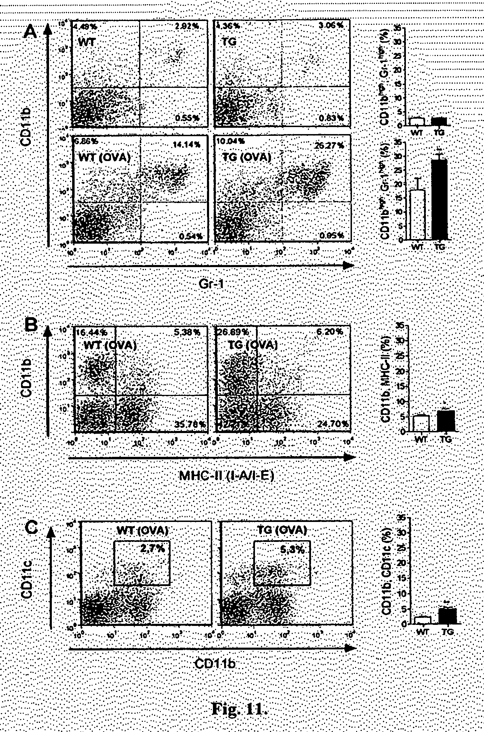

**Figure 11****.** Immunization resulted massive influx of neutrophils into the spleen. To further characterize the cells migrating into the spleen 25 days after immunization, we analyzed them by flow cytometry using double labeling technique. We found that cells bearing CD11b^{high} and Gr-1^{high} antigens were raised approximately five-fold in wt and nine-fold in Tg mice after immunization (**A**). We also found that the ratio of the cells bearing CD11b and MHC class II antigens (macrophages, dendritic cells) (**B**) and CD11b and CD11c antigens (dendritic cells) (C - gated cells) were significantly elevated in Tg mice compared to their wt controls. Density plot graphs show one typical experiment of 2 performed (n = 3 mice per group). Bar graph values shown are the mean ± SD; *, p<0.05; **, p < 0.01.

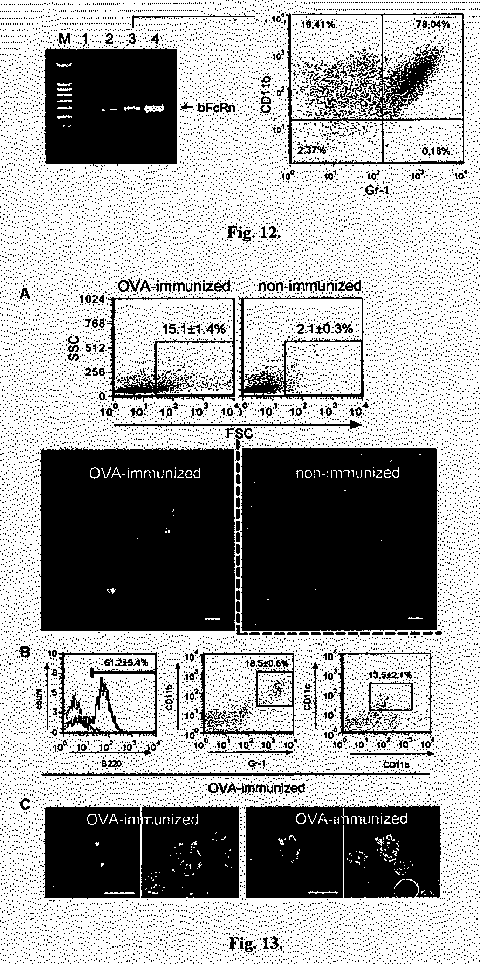

**Figure 12****.** bFcRn specific RT-PCR expression in neutrophils and macrophages derived from peritoneal cells. bFcRn specific amplicons were successfully amplified from samples of Tg mice. Labels: M1 - BenchTop 100bp DNA ladder (Promega); 1 - non-purified peritoneal cells wt mice; 2 - non-purified peritoneal cells from Tg mice; 3 - purified neutrophils (CD11b^{high}, Gr-1^{high}), macrophages and dendritic cells (CD11b^{high}, Gr-1^{low}) from Tg mice also analyzed by flow cytometry, 4 - amplification from a plasmid containing the bFcRn α-chain cDNA. Arrowhead indicates a 548 bp DNA band, specific for the bFcRn. Density plot graph show one typical experiment of 2 performed.
**Figure 13****.** Presence of OVA-FITC-bearing cells in the spleen. OVA-immunized (56 days after immunization) and non-immunized transgenic mice (homozygote #14), were intraperitoneally treated with OVA-FITC. Five hours after the treatment the spleen cells were analyzed by flow cytometry and we found that 15.1±1.4% OVA-FITC positive cells were present in the spleen of OVA-immunized mice, while we did not find considerable number of OVA-FITC positive cells in non-immunized animals (2.1±0.3%). The confocal images show cells from spleen of OVA-immunized and non-immunized mice. The nuclei of the cells were stained with a DRAQ5 red fluorescent cell-permeable DNA probe while FITC-OVA can be seen as bright spots **(A).** Among the FITC-positive cells, 61.2±5.4% were B220 positive (B-lymphocytes), 18.5±0.6% were CD11b^{high} and Gr-1^{high} (neutrophils) and 13.5±2.1% were CD11b and CD11c positive (dendritic) cells in the OVA immunized animals (**B**). OVA-FITC is internalized in a typical neutrophil that has a polylobed nucleus, while OVA-FITC is detected on the surface of a cell that has large, round shaped nucleus and a thin rim of cytoplasm, presumably B-lymphocyte (**C**). The density plot graphs and histograms show one typical experiment (n = 3 mice per group); data indicate the percentage of cells (mean ± SD). Scale bars indicate 10 µm.

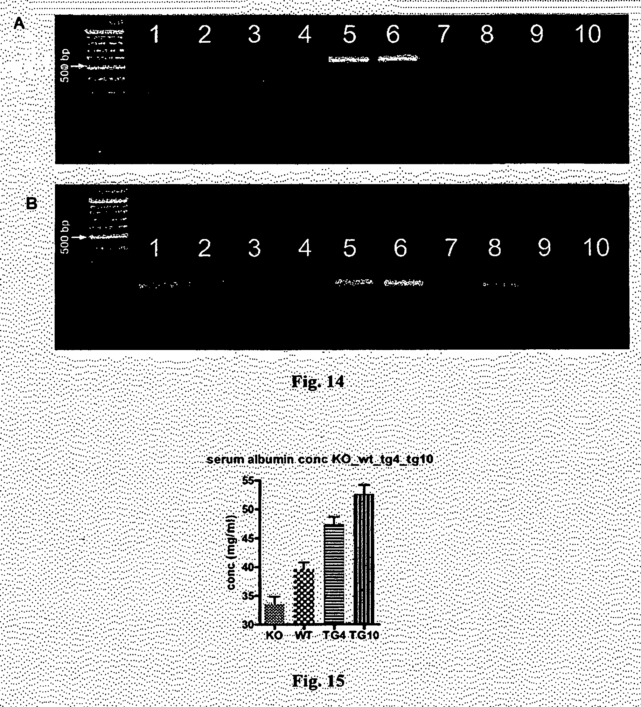

**Figure 14****.** RT-PCR analysis of a HIV-P2-FcRn transgenic founder mice. A RT-PCR with primers planned for P2 promoter, expected fragment size: 579 bp, **B** RT-PCR with primer planned for bovine FcRn exon 4, expected fragment size: 161 bp. Samples: 1. HIV-P2-FcRn mice lung; 2 HIV-P2-FcRn mice liver; 3 HIV-P2-FcRn mice intestine; 4. HIV-P2-FcRn mice spleen; 5. HIV-P2-FcRn mice genomic DNA; 6. Bovine genomic DNA; 7. Control mice genomic DNA; 8. Bovine liver cDNA; 9. Control mice cDNA, 10. Negative control.
**Figure 15****.** bFcRn overexpression seriously influence albumin metabolism. Serum albumin levels in FcRn deficient (FcRn KO), wt, homozygous #14 and #19 (express 4 and 10 copies of bFcRn, respectively). Significant differences was observed when we compared the albumin levels of KO and WT mice (p<0.01); WT and TG4 or TG10 mice (p<0,001).

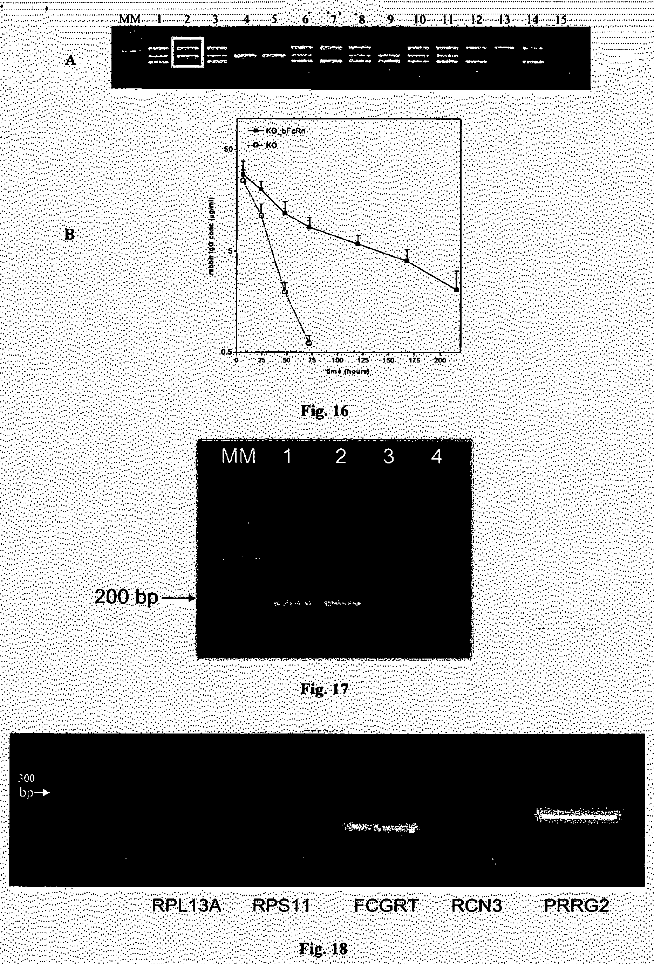

**Figure 16****.** Rabbit IgG clearance in FcRn^{-/-} (KO) and in FcRn^{-/-} bovine FcRn transgenic (KO_bFcRn) mice. **A**. Multiplex PCR to identify F2 mice with the bFcRn /mFcRn^{-/-} genotype (indicated with a rectangle). F2 mice were created through crossbreeding of F1 mice born from crossing of the bFcRn/mFcRn X mFcRn^{-/-}neo^{+µ} parental strains. Primers are described in example 12. Expected fragment sizes: bFcRn: 610 bp; neo: 345 bp; mFcRn 278 bp. Slots: 1. bFcRn⁺, neo⁺, mFcRn⁺; 2. bFcRn⁺, neo⁺, mFcRn⁻; 3. bFcRn⁺, neo⁺, mFcRn⁺; 4. bFcRn⁻, neo⁺, mFcRn⁻; 5. bFcRn⁻, neo⁺, mFcRn⁻; 6. bFcRn⁺, neo⁺, mFcRn⁺; 7. bFcRn⁺, neo⁻, mFcRn⁺; 8. bFcRn⁺ neo⁺, mFcRn⁺; 9. bFcRn⁻, neo⁺, mFcRn⁺;10. bFcRn⁺, neo⁺, mFcRn⁺; 11. bFcRn⁺, neo⁺, mFcRn⁺; 12. bFcRn⁺, neo⁻, mFcRn⁺; 13. bovine genomic DNA; 14. bFcRn⁺, neo⁻, mFcRn⁺; 15. negative control **B.** Pharmacokinetics of rabbit IgG in FcRn^{-/-} (KO) and in FcRn^{-/-} bovine FcRn transgenic (KO_bFcRn) mice. Rabbit IgG was cleared rapidly in FcRn^{-/-} mice (half-life: 15 hours), but it was protected in bFcRn/FcRn^{-/-} (half-life: 67 hours). The animals were injected i.v. with 150 µg rabbit IgG. Three mice/group were treated and the data are representative of two independent experiments. Data show average values and error bars represent the standard error of the mean (SEM).
**Figure 17****.** PCR analysis of a bFcRn transgenic rabbit. PCR with primers designed to detect the bovine FCGRT 4th exon. Expected size is 160 bp. Samples: 1. 38/JT rabbit genomic DNA; 2. Bovine genomic DNA; 3. FVB/n mouse genomic DNA; 4. Blank control; MM 1kb ladder.
**Figure 18****.** Characterization of the 262E02 rabbit BAC. Primers and PCR conditions used for evaluating the presence of rabbit genes are described in Table 7 in Example 15.

### EXAMPLES

### Example 1 - Isolation and characterization of the BAC clones that harbor the bFcRn α-chain gene (bFCGRT)

### Isolation of BAC clones that harbor the bFCGRT

The 90α BAC was isolated from a bovine BAC library made from DNA from lymphocytes of an adult male (2 years) Bos taurus Jersey (obtained from the Resource Center/Primary Database of the German Human Genome Project (RZDP), Max Planck Institute for Molecular Genetics, Berlin, Germany; http://www.rzdp.de/). A bFCGRT positive BAC clone was identified by PCR screening with primers specific to the bFcRn α-chain mRNA [206 -425 bp; GenBank AF139106) was PCR amplified BFc1S: 5'-CAGTACCACTTCACCGCCGTGT-3' (SEQ ID.NO.: 1); BFclas: 5'-CTTGGAGCGCTTCGAGGAAGAG-3'(SEQ.ID.NO.: 2)]. As a following step the bFCGRT DNA was sequenced with primers that anneal to the exons of the gene (Kacskovics et al., 2000). In order to analyze the bFCGRT upstream flanking region the BAC DNA was digested with BamHI, and the digested DNA was separated on an agarose gel. Southern blot detected a 9 kb long positive band using a DNA fragment from α1 domain, as a probe. The 9 kb long BamHI fragment was then subcloned into the pGEM-11zf(+) vector. An additional subcloning process resulted a 2 kb of the promoter segment with exon1 until exon3 in the same vector. The insert was then completely sequenced by ABI Prism BigDye Terminator Cycle sequencing Ready Reaction Kit (AB1, 373A-Stretch, Perkin Elmer) in the Cybergene Company (Huddinge Sweden).

The 189HO2 and 128EO4 BACs were isolated by bFCGRT specific primers: FcRnF: 5'-CGGCCACCTCTATCACATTT-3' (SEQ.ID.NO.: 3) and FcRnR: 5'-TGCATTGACCACACTTGGTT-3' (SEQ.ID.NO.: 4) (GenBank NW_929385) from a bovine BAC library (Eggen et al., 2001). The size of the insert of the isolated BAC clones was analyzed by digesting the clones with NotI restriction endonuclease. Expand Long Template PCR System (Roche) was used to determine the size of the 5' and 3' bordering regions of the bFCGRT in the inserts. Two sets of primers were designed: pBAC-upper (5'-ACCTCTTTCTCCGCACCCGACATAG, SEQ.ID.NO.: 5, U80929 11380-11404) and bFcRn-antisense (GTTCAAGTCCAAAGGCAGGCTATCT, SEQ.ID.NO.: 6) primers amplified the 5' overhang region. On the other hand, a bFcRn-sense (CCTTTACCCACACCCACTCCCCACA, SEQ.ID.NO.: 7) and a pBAC-lower (AGAAGTTCGTGCCGCCGCCGTAGTA, SEQ.ID.NO.: 8, U80929, 3801-3777) as antisense primers were used to amplify the 3' overhang region of the bFCGRT.

### Characterization of the bFCGRT

Approximately 1800 bp of the region upstream of the transcription start as well as the entire gene of the bFCGRT was sequenced and compared to the deposited bovine genomic sequences at NCBI by using the BLAST program. The sequenced fragment was screened for interspersed repeats and low complexity DNA sequences using the RepeatMasker program (Smit et al., 1996-2004) (http: repeatmasker.org). The interspersed repeat databases screened by RepeatMasker are based on repeat databases (Repbase Update; (Jurka et al., 2005). The data agrees well with the deposited cow sequence in NCBI and the first 3477 bp (until the non-sequenced 2.5 kb region) shows 99% identity to clone NW_929385 (*Bos taurus* chromosome 18 genomic contig), although it has a 285 bp long gap between 2002 and 2287 bp that belongs to the first intron. The second part of the analyzed sequence contained 1780 bp (after the non-sequenced 2.5 kb segment) that shows 99% identity to the same genomic contig. This fragment also has a 167 bp long gap (between 1203 - 1370 bp) that belongs to intron 6. The two unidentified segments contain repetitive sequences (simple-repeat), which explains the lack of homology. Based on these alignments the size of the missing part was calculated to be 2556 bp. Thus, it was found that the sequence shows high homology to the cattle genomic contig NW_929385, between 648611 and 656426 bp.

The transcription initiation site by aligning the available bFcRn α-chain cDNA sequences have been also analyzed (reference sequences as well as the sequences from the EST database; NCBI) to the bFCGRT genomic sequence. The genomic organizations of the bovine, human, rat and mouse FCGRT genes were compared using the NCBI Map Viewer (data not shown).

### Characterization of the bFCGRT positive BAC clones

Three different BAC clones - 90α, 189HO2 and 128E04 - containing the bovine neonatal Fc receptor α-chain gene (bFCGRT) and its own genomic environment were isolated. Following NotI digestion, the genomic inserts from the BAC vectors were analyzed by pulsed field gel electrophoresis which revealed that clone 189HO2, clone 128E04 and clone 90α contains approximately 130 kb, 100 kb and 90 kb size bovine genomic inserts, respectively.

Then, the size of the bordering regions of the bFCGRT gene was determined with long-range PCR. Data showed that the 90α and 189HO2 BACs were carrying 8.5 kb and 14 kb 5' and 3' flanking regions respectively, which based on their sizes, may not possess all the regulatory elements that ensure the integration site independent, tissue specific expression of the bFCGRT gene. PCR amplification of clone 128HO2 with the two primer sets didn't reveal any products. Since Expand Long Template PCR System is a robust amplification method and generate up to 25 kb amplicons from phage DNA, we concluded that both 5' and 3' bordering regions of the bFCGRT in this clone are longer than 25 kb, therefore we selected 128EO4 BAC for microinjection.

Recent data at the Bovine Genome Resources website (http://www.ncbi.nlm.nih.gov/projects/genome/guide/cow/) and the sequences of the 5'and 3' end of the 128E04 BAC enabled us to determine the genomic context and the exact size of bordering regions of bFCGRT: the 5' regulatory region extends up to 44 kb, while the 3' is 50 kb long. Data revealed that 128E04 BAC contained five putative protein coding genes (FLT3LG, LOC539196, LOC522073, LOC511234, LOC511235) and the bFCGRT (**Fig. 1A****).**

### Example 2 - Generation and genotyping of transgenic mice carrying bovine BAC 12BE04 (not claimed)

### Preparation of the 102 kb. genomic insert from BAC clone 128E04 for microinjection and generation of transgenic mice carrying bovine BAC 128E04

Preparation of BAC (clone 128E04) DNA for microinjection has been performed by slight modification of the protocol published by Schedl et al (Schedl et al., 1996). The purified BAC (Qiagen plasmid purification for very low-copy plasmid) was digested with Not I (Fermentas) to release the insert, which was isolated in a preparative pulsed field 1% agarose gel. The gel slice containing the insert was run into an LMP (low melting point) gel which was digested with Gelase (Epicentre). Microcon YM50 (Millipore) column was used to clean the insert from the agarose. The insert was eluted in a buffer suitable for microinjection (10 mM Tris-HCl, pH 7.5, 0.1 mM EDTA, pH 8.0, 100 mM NaCl supplied with or without 0,03 mM spermine/0,07mM spermidine (SIGMA). The DNA concentration was adjusted to 0.4 ng/µl using microinjection buffer (10mM Tris-HCl, pH 7.5, 0.1 mM EDTA, 100 mM NaCl) and injected into fertilized FVB/N mouse oocytes. Recipients were 10 weeks old CD1 females. Experimental animals were obtained from the Charles Rivers Laboratories Hungary Ltd. (Budapest).

### Characterization of the transgenic mice

In order to detect the presence of the bFCGRT in the mice, genomic DNA was isolated from tail biopsies of the litters born from embryo transfer and the G1 and G2 progeny of founders and screened by two PCR amplifications. The two primer pairs were designed based on the bFCGRT sequence of the 90α BAC clone. The first primer pair was composed of the bFcSuf; 5'-CTCCTTTGTCTTGGGCACTT-3' (SEQ.ID.NO.: 9) as sense and BFcL 5'-GCCGCGGATCCCTTCCCTCTG -3' (SEQ.ID.NO.: 10) as antisense; which gave rise a 600 bp product (1275 - 1894 bp), while the second primer pair was Fcmfpr/in 5'-AAAGTTTCTCGAGAGAGGCAGAGAC-3' (SEQ.ID.NO.: 11) as sense and Fcmrpr/in 5'-TAGTTACAGAGCCTGGATAGGCTGA-3' (SEQ.ID.NO.: 12) as antisense which gave a 410 bp product (1698 - 2108 bp). The results of the transgenesis experiments are shown in **Table 1.**

**Table 1. Generation of transgenic mice carrying bovine BAC 128E04**

| | Injected construct | 128E04 |
|---|---|---|
| Number of embryos | Microinjected | 550 |
| | Transferred | 360 |
| Number of animals | Born | 41 |
| | Transgenic | 6 |
| Efficiency of microinjection | No. of born animal/No. of Tg | 14.6 |

A shown in **Table 1,** a total of 41 pubs were born and genotyped from tail DNA for the presence of the bFCGRT. From the six founders three independent Tg lines have been established. Two of these lines #14, #19 showed Mendelian pattern of the transgene inheritance in the first generation (17 and 12 from a total 30 and 34 litters carried the transgene), however the third line #9 indicated a degree of mosaicism in the founder animal. Tg mice were indistinguishable from their littermates based on their weight and overall health.

### Analysis of long-range transgene integrity

The integrity of the transgene in the three Tg lines was evaluated by specific primer pairs designed for the 5' and 3' ends of the BAC 128E04 and for the five putative protein encoding genes that are localized on the injected BAC based on the bovine genomic map (GenBank MapViewer Build 3.1 (based on Btau 3.1) bovine chromosome 18; region between 53543852-53652024 bp). The primer sequences and conditions are described in **Table 2.**

**Table 2. Primers and PCR conditions for evaluating the intactness of the integrated BAC 128E04**

| Putative gene | Primer | Tm (°C) | Product (bp) |
|---|---|---|---|
| BAC128EOH 5'-end | 5'-TTT AGC TGC ATC GGG ATC TT-3' (SEQ.ID.NO.: 13) | 61 | 441 |
| | 5'-GGA GTG ATG GCA TTT GGT TT-3' (SEQ.ID.NO.: 14) | | |
| FLT3LG | 5'-TCG GAG ATG GAG AAA CTG CT-3' (SEQ.ID.NO.: 15) | 61 | 547 |
| | 5'-CTG GAC GAA GCG AAG ACA G-3' (SEQ.ID.NO.: 16) | | |
| LOC539196 | 5'-AGA ACG TGC GTA CCA AAA GC-3' (SEQ.ID.NO.: 17) | 61 | 787 |
| | 5'-AGC GGT TGT ACT TTC GGA TG-3' (SEQ.ID.NO.: 18) | | |
| bFCGRT | 5'-CCA AGT TTG CCC TGA ACG-3' (SEQ.ID.NO.: 19) | 61 | 161 |
| | 5'-GTG TGG GCA GGA GTA GAG GA-3' (SEQ.ID.NO.: 20) | | |
| LOC522073 | 5'-AGT GGT CCT GGG ATT GAC AG-3' (SEQ.ID.NO.: 21) | 61 | 266 |
| | 5'-TCA CTG AGT CCC GTA TGT GC-3' (SEQ.ID.NO.: 22) | | |
| LOC511234 | 5'-CTA CGT GTG CGC CGT GAC-3' (SEQ.ID.NO.: 23) | 61 | 220 |
| | 5'-AAT CAG CTT CTC CAC GCA CT-3' (SEQ.ID.NO.: 24) | | |
| LOC511235 | 5'-GTT GTT CAC ACC AGG GAA CC-3' (SEQ.ID.NO.: 25) | 61 | 295 |
| | 5'-CCT TTG CCA TTG TAG ATG TAG C-3' (SEQ.ID.NO.: 26) | | |
| BAC128EOH 3'-end | 5'-AGT CGT GTC CGA CTC TTT GC-3' (SEQ.ID.NO.: 27) | 61 | 416 |
| | 5'-CAG CCT GTC TGG TGT TCT GA-3' (SEQ.ID.NO.: 28) | | |

BAC128EOH 5'-end and BAC128EOH 3'-end sequences were obtained from A. Eggen (Inra, Jouy-en-Josas, France). All primer pairs gave the same PCR products as the 128E04 BAC and the bovine genomic DNA indicating integration of the intact BAC except line #9 DNA in which the LOC 511234, LOC 522235 and the BAC128E04 3'-end specific PCR did not result PCR products (**Fig. 1B**). Therefore, it can be concluded that in this Tg line an estimated 30 kb long fragment from the 3' end of the integrated BAC transgene was missing. The loss of genomic fragments both from 5' and 3' ends of large transgenes is a common phenomenon (Raguz et al., 1998). Nevertheless to avoid the possibility of altered bFCGRT expression due to the absence of not characterized regulatory elements which might lie in the missing part of the BAC DNA, line #9 was not included in the further studies.

### Chromosomal localization of the transgene

In order to exclude the possibility that the bovine BAC 128E04 clone was accidentally integrated at an identical segment of a mouse chromosome in both transgenic lines and thereby the phenotype of transgenic mouse lines #14 and #19 resulted from insertional mutagenesis of unidentified gene/s/ at the transgene's integration sites, fluorescence in situ hybridization (FISH) was performed to visualize the genomic integration of the 128E04 transgene. The 128E04 BAC DNA was labeled by nick-translation with biotin-14-dATP (BioNick labeling kit, Invitrogen, USA). Mitotic chromosomes were obtained from vinblastine treated fibroblasts, which were isolated from 13.5 day old homozygote #14 and #19 embryos respectively, following standard protocols involving hypotonic treatment and methanol:acetic acid (3:1) fixation.

FISH was performed essentially as described previously (Hayes et al., 1992). The biotinylated probe was denatured and allowed to hybridize to denatured chromosome spreads overnight at 37 °C. Hybridization sites on chromosomes were amplified with an anti-biotin antibody raised in goat (Vector Laboratories Inc, Burlingame, CA), and visualized by further incubation with fluorescein conjugated rabbit anti-goat IgG (Nordic Immunological Laboratories, Tilburg, The Netherlands). Chromosome preparations were counterstained with diamidino-2-phenylindole (DAPI; Vector Laboratories, Burlingame, CA) and observed with an Nikon Eclipse E600 epifluorescence microscope (Nikon Instruments; Kawasaki, Japan). Fluorescence images were captured using a Cohu 4910 CCD camera (Cohu, Inc.; San Diego, CA, USA) and digitized with MacProbe 4.3 FISH software (Applied Imaging; Newcastle upon Tyne, UK) running on an Apple Macintosh G4 computer.

The result of FISH analysis revealed that the fluorescently labeled BAC 128E04 hybridized to entirely different chromosome segments in the #14 and #19 mice strains respectively. This excludes the possibility that their phenotypes are integration site dependent. The single spots in the chromosomes indicate that multiple copies (2 in line #14, and 5 in line #19) of the transgene integration most probably occurred in the form of tandem repeats **(****Fig. 2****).**

### Example 3 - Effect of the copy number of the bovine FCGRT (bFCGRT) gene on FcRn expression (not claimed)

### Transgene copy number determination with real-time quantitative polymerase chain reaction

Real-time PCR is a quantitative and precise method to determine the copy number and zygosity of transgenes in Tg animals (Tesson et al., 2002). The 128E04 BAC transgene copy numbers were determined by absolute quantification of the bFCGRT and the internal standard mouse β-actin genes as follows.

The 128E04 BAC transgene copy numbers were determined with TaqMan method, using the ABI Prism 7000 Sequence Detection System (Applied Biosystems, Foster City, CA). Primer and probe oligonucleotide sequences were designed with the Primer Express v2.0 program (Applied Biosystems) using default parameters (primers and probes are indicated in **Table 3**). Conventional phenol/chlorophorm method was used for DNA extraction from tail samples of hemyzigous animals with an additional chlorophorm extraction step.

**Table 3. Primers and probes for bFCGRT transgene copy number determinations**

| | | |
|---|---|---|
| mouse β-actin | Probe | VIC-TGGCTTTCTGAACTTGACAACATTAT-TAMRA (SEQ.ID.NO.: 29) |
| | Forward | TTCACCTGCCCTGAGTGTTTC (SEQ.ID.NO.: 30) |
| | reverse | TGAAGGTCTCAAACATGATCTGTAGA (SEQ.ID.NO.: 31) |
| bovine FCGRT | probe | FAM-CACAGTCAAGAGTGGCGACGAGCAC-TAMRA (SEQ.ID.NO.: 32) |
| | forward | GCACCACGCAGCGGTAGT (SEQ.ID.NO.: 33) |
| | reverse | CCTTCTACGCCTGGTCATCAC (SEQ.ID.NO.: 34) |

The mouse β-actin and bFCGRT genes were quantified in each sample by absolute quantification using calibration curves. Standard curves, using five points, diluted over a 32-fold range led to a high linearity with the primer sets. Linearity and efficiency of PCR quantification were validated before quantification. Samples were run in duplicate (**Fig. 3**).

The endogenous β-actin gene which is represented by two copies in each cell was used as internal standard to determine the DNA concentration. Mouse genomic DNA was used to set up the calibration curve for the β-actin gene. Absolute quantification of the bFCGRT gene was performed based on a standard curve generated from serial dilutions, of the 128E04 BAC supplemented with mouse genomic DNA. The standard curves enabled us to determine the copy numbers of bFCGRT gene based on the following calculations: the exact amount of DNA determined the number of diploid genomes in the samples, while the bFCGRT gene calibration curve determined its copy number in DNA samples of hemizygous animals from lines # 14 and # 19. After carrying out these calculations, the copy number of the bFCGRT gene was determined to be 2 and 5 in hemizygous animals of line # 14 and #19 Tg mice, respectively (**Table 4**).

**Table 4. Absolute quantity of cell number and absolute quantity of FcRn copy number of samples were derived from the mouse β-actin and bFcRn calibration curves, respectively (Fig. 3). Estimated copy number of bFcRn transgene in hemizygous animals was 2 in line #14 and 5 in line #19.**

| #line | gene | Ct | Absolute quantity of gene in sample (AGN) | Absolute quantity of cell number in sample (ACN) | bFcRn copy number (ACNb-FcRn/ACN) | Estimated copy number of bFcRn transgene in a diploid hemizygous animal |
|---|---|---|---|---|---|---|
| 14 | Mouse β-actin | 31.01 | 3760.49 | 1880.248 | | |
| | bFcRn | 28.86 | 3852.48 | | 2.04 | 2 |
| 19 | Mouse β-actin | 30.89 | 4075.44 | 2037.722 | | |
| | bFcRn | 27.05 | 10468.18 | | 5.13 | 5 |

### Copy number determination at transcription level by reverse transcriptase-PCR and Northern analysis

Total RNA was extracted by using RNAzol™ B (TEL-TEST INC) from liver, lung, and mammary gland of six weeks old females and from the intestine of newborns. Two micrograms of RNA was reverse transcribed by using Moloney-murine leukemia virus (M-MLV) reverse transcriptase enzyme and the (dT)17-adapter primer as recommended by the manufacturer (Acces RT-PCR System; Promega). PCR was performed to obtain a 367 bp long bFCGRT specific amplicon (914-1280 bp, (Kacskovics et al., 2000) by the primer pairs: B7 5'-GGCGACGAGCACCACTAC-3' (SEQ.ID.NO.: 35) and B8 5'-GATTCCCGGAGGTCWCACA-3' (SEQ.ID.NO.: 36) (where W can be A or T). The amplified segment was separated by electrophoresis on 1% agarose gel and stained with ethidium bromide.

Since ruminant FcRn transcripts have been detected in multiple epithelial cells (Kacskovics et al., 2006b; Mayer et al., 2004; Mayer et al., 2002) and also in vascular endothelial cells (Kacskovics et al., 2006a), bFcRn α-chain expression was analyzed in the lung, liver and mammary gland of adult lactating Tg females and from the intestines of newborns by using RT-PCR. The bovine FcRn α-chain mRNA was expressed in all of the selected tissues of hemizygous animals from lines # 9, # 14 and #19 (**Fig. 4A**).

In order to evaluate the copy number dependence of transgene expression and to compare it with the quantity of the endogenous bovine FcRn α-chain mRNA, liver RNA samples from bovine and individuals of lines #14 and #19 were analyzed using Northern blot. Total RNA was isolated from the livers of young adult female mice and 5 µg of total RNA was size fractionated on 1% agarose/2.2 M formaldehyde gel, transferred to Hybond N+ membrane (Amersham) and hybridized with the ³²P-labeled cDNA probe synthesized by PCR with the B7-B8 primers described above. 18S RNA signal was used as an internal standard to estimate RNA loading on the gels. The signals obtained were evaluated using a Phosphorlmager™ and quantified with STORM™ (Molecular Dynamics). Comparison between the bFcRn mRNA specific signal densities of the two, four, five and ten copies Tg mice was done by using the Student's t-test.

The results show higher levels of transgene mRNA expression in line #19. The level of mRNA expression in the liver of the line #14 hemizygous Tg mice, carrying two copies of the BAC transgene reached 90% of that observed in the bovine liver (**Fig. 4B**).

Quantitative analysis and statistical evaluation of signal intensity from two or three hemi- and homozygote animals from both Tg lines revealed that the amount of bovine FcRn α-chain mRNA in the liver of Tg mice strictly correlates with their transgene copy numbers and the differences were significant at a p<0.05 probability level (**Fig. 4C**). This result along with the fact that the level of FcRn α-chain mRNA expression in the liver of Tg mice carrying two transgene copies was similar to the level of mRNA in the bovine liver indicates that the 128E04 BAC carries all the necessary regulatory elements that ensure copy number dependent, position independent expression for the bFCGRT.

### Detection of bFcRn α-chain protein in the lung of transgenic mice

Expression of the bovine FcRn α-chain at protein level was examined by Western analysis. Protein extracts were resolved on polyacrilamide denaturing Tris-glycine gel; blots were probed with affinity purified rabbit antiserum (raised against the peptide CLEWKEPPSMRLKAR representing the highly conserved 173-186 aminoacid residues of bFcRn α-chain plus an N terminal Cys for conjugation to KLH (Mayer et al., 2002). Bound bFcRn α-chain antibody was detected with horseradish peroxidase-conjugated goat anti-rabbit antibody and enhanced chemiluminescence, using luminol-based solution as substrate. The bovine mammary epithelial cell line (B4) stable transfected with bFcRn α-chain was used as positive control (Kacskovics et al., 2006a).

In hemizygous lung samples from both Tg lines - consistently with the known molecular weight of bFcRn α-chain (Kacskovics et al., 2000) - a 40 kD protein was detected, which has not been found in the wild type mouse used as negative control (**Fig. 5**). The molecular weight of the transgenic FcRn α-chain was compliant with the recombinant protein produced by the B4 bovine mammary epithelial cell line which was stable transfected with bFcRn (Kacskovics et al., 2006a). Moreover, this data confirmed the results of the Northern blot analysis indicating that the sample from #19 mice expressing 5 copies of transgene bFcRn shows much more bFcRn protein that was detected in line #14 mice expressing 2 transgene copies (**Fig. 5**).

These data demonstrated that the 102 kb long BAC clone posses all the necessary genetic regulatory elements required for the reproducible, tissue-specific expression at physiological levels of bFcRn α-chain (**Fig. 4A**), which emphasize that the behavior of bovine transgenes in mice should, whenever possible, be compared to expression patterns for that gene in the bovine tissues.

### Example 4 -In vivo studies analyzing the mouse and human IgG half-lives in bovine BAC transgenic mice (not claimed)

In order to analyze the expression of the bFcRn α-chain in the Tg mice as well as to test if the bovine -FcRn α-chain and the mouse β2m is able to form a functional receptor, the clearance of the mouse and human IgG have been analyzed. The latter was investigated since it was recently shown that the bovine FcRn binds human IgG much better than it binds bovine IgG (Kacskovics et al., 2006a).

Following a prebleed, age, weight and sex (male) matched homozygote #14 and control mice (three to five in each group) were microinjected intravenously with 10 mg/kg bodyweight (BWkg) of anti-OVA mouse IgG1 (mAb, Sigma) or 10 mg/BWkg or 20 mg/BW_{kg} of human IgG (Gammonativ intended for intravenous use was a kind gift from Octapharma, Stockholm, Sweden) in 50mg/ml saline solution and during the next 216 hours, periodic blood samples (50 µl/occasion) were collected from retroorbital plexus. A quantitative ELISA employing OVA (Sigma) as capture reagent and an HRP-conjugated affinity-purified polyclonal goat anti-mouse IgG (γ chain specific) (Southern Biotech Associates Inc., Birmingham, AL, USA) as detecting reagent was used to evaluate plasma concentrations of anti-OVA mouse IgG1 during the course of the experiment. Serum concentrations of human IgG were determined by quantitative ELISA assay as described earlier (Kacskovics et al., 2006a). A quantitative ELISA employing ovalbumin (Sigma) as capture reagent and an HRP-conjugated affinity-purified polyclonal goat anti-mouse IgG (γ chain specific) (Southern Biotech Associates Inc., Birmingham, AL, USA) as detecting reagent was used to evaluate plasma concentrations of anti-ovalbumin mouse IgG1 during the course of the experiment. The peroxidase-conjugated antibody was detected using TMB (3,3',5,5'-tetramethylbenzidine; Sigma) as the substrate. The samples were assayed in duplicates. Concentrations of Ig are reported based on a reference standard.

Analysis of the mean IgG concentrations of the mice in the first ten days was done by fitting the data to the two-compartmental model using WinNonLin professional, version 4.1 (Pharsight, Mountain View, CA).

The clearance curves for mouse IgG was biphasic, with phase 1 (alpha phase) representing equilibration between the intravascular and extravascular compartments, phase 2 (beta-phase) representing a slow elimination. Mathematical modeling of phases 1 and 2 until hours 216 have shown good correlation to the general scheme of FcRn mediated IgG pharmacokinetics (Lobo et al., 2004), hence we calculated the alpha and beta phase half-lives of mIgG in this time frame. The estimated alpha phase half-lives were similarly around 5 hours in the wt and Tg mice, respectively. In contrast, there was a significant difference (p<0.05) in the beta phase half-lives, as it was 125.4 ± 3.2 hours (mean ± SEM) in the wt and 165.1 ± 7.8 hours in the Tg animals, based on the two-compartmental modeling analyses (**Table 5;** **Fig. 6A**).

In the case of hIgG, similarly to the mIgG clearance data, mathematical modeling of phases 1 and 2 until hours 216 of the data derived from this study have shown good correlation to the general scheme of FcRn mediated IgG pharmacokinetics; therefore we calculated the alpha and beta phase half-lives of hIgG in this time frame. Based on the two-compartmental modeling analyses, the alpha phase was around 10 hours both in the wild-type and also in the transgenic mice, with no difference between the 10 and 20 microgram per gram experiments. In the case of beta phases, we observed significant differences (p<0.05) between wt and Tg mice. The calculated beta-phases were 106 ±3.6 and 171.5 ± 16.2 hours when injecting 10 microgram per gram, whereas, 108.2 ± 3.7 hours and 181 ± 7.7 hours when injecting 20 microgram per gram, in the wt and Tg mice, respectively (T**able 5;** **Fig. 6B**). As expected, the area under the curve (AUC) values were significantly different, as in experiment when we injected 20 microgram per gram it showed roughly doubled values compared to the 10 microgram per gram experiment; on the other hand neither the alpha- nor the beta phase half-lives were significantly different injecting 10 or 20 µg hIgG per gram.

When we compared the mIgG to the hIgG clearance data in these experiments (comparing the 10 microgram IgG per gram experiments), we observed that hIgG cleared significantly faster (p<0.05) from the wt mice (106 versus 125.5 hours of human and mouse IgG, respectively), however there was no difference between the mouse and human IgG clearance data in Tg animals (**Table 5**).

**Table 5. Pharmacokinetic parameters of mouse and human IgGs in wild-type and bFcRn transgenic mice. Values represent the mean ± SEM; MRT, mean residence time; AUC, area under the curve.**

| | Dose *µg*/*BW_{g}* | alpha-phase half-life (*hours*) | **beta-phase half-life *(hours)*** | MRT *hour* | AUC *hour*µg*/*ml* | n |
|---|---|---|---|---|---|---|
| WT-mIgG | 10 | 5.2 ± 0.6 | **125.4 ± 3.2** | 167.7 ± 5.6 | 12444.5 ± 1447 | 5 |
| TG-mIgG | 10 | 4.6 ± 0.2 | **165.1 ± 7.8** | 227.2 ± 11.9 | 14713.3 ± 758.8 | 5 |
| WT-hIgG | 10 | 8.2 ± 1.8 | **106 ± 3.57** | 139.8 ± 4.8 | 7351.2 ± 376.5 | 4 |
| TG-hIgG | 10 | 12.7 ± 1.8 | **171.5 ± 16.2** | 227.9 ± 21.3 | 15456 ± 1214.9 | 5 |
| WT-hIgG | 20 | 108 ± 5.7 | **108.2 ± 3.7** | 151.8 ± 5.8 | 17574.3 ± 1711 | 3 |
| TG-hIgG | 20 | 13.2 ± 7.5 | **181 ± 7.7** | 265.1 ± 5.9 | 30788.5 ± 3018.4 | 4 |

In conclusion, these IgG clearance studies in the BAC transgenic mice confirmed that there was no major deficiency of the β2m and also that the bovine FcRn α-chain formed functional complex with the mouse β2m. The mIgG1 clearance was significantly reduced in Tg mice indicating that the amount of the mouse and hybrid FcRn available for protection of the administered antibodies contributes to the degree of protection from clearance *in vivo.* In addition, there was no difference between the mouse and human IgG clearance data in Tg animals, indicating that the hybrid bFcRn α-chain mβ2m receptor can rescue hIgG1, as well.

### Example 5- Immunization with ovalbumin (not claimed)

Homozygote #14, hemizygote #19 (encoding 4 and 5 copies of bFCGRT, respectively) and age and sex (male) matched wt mice (five in each group) were intraperitoneally immunized with 250 µg ovalbumin (OVA, Sigma-Aldrich, Budapest, Hungary) in complete Freund adjuvant (CFA) and challenged 14 days later with 250 µg OVA with incomplete Freund adjuvant (IFA, Sigma-Aldrich, Budapest, Hungary).

All experimental procedures were approved by the Animal Care and Ethics Committee of the Agricultural Biotechnology Center, Gödöllö, Hungary.

### Antigen specific IgM and IgG titers

To reveal the immunological consequences of the bFcRn overexpression beyond IgG protection, wt and Tg (#14 and #19) mice were immunized, challenged 2 weeks later with OVA, and their serum anti-OVA IgM and IgG titers were measured. Blood samples (50 µl/occasion) from retroorbital plexus were taken for 56 days. Sera were assayed for OVA and FITC specific IgM and IgG. A quantitative ELISA employing OVA and FITC-albumin (Sigma-Aldridh, Budapest, Hungary) as capture reagents and an HRP-conjugated affinity-purified polyclonal goat anti-mouse IgM and goat anti-mouse IgG (µ and γ chain specific) (Southern Biotech Associates Inc., Birmingham, AL, USA) as detecting reagent were used to evaluate plasma concentrations of anti-OVA and anti-FITC mouse IgM and IgG during the course of the experiments. The peroxidase-conjugated antibody was detected using TMB (Sigma-Aldrich, Budapest, Hungary) as the substrate. Serial dilutions of each test serum sample were applied and the Ig concentrations were reported based on absorbance values at 450 nm interpolated from a linear portion of the dose-response curve. Samples were assayed in duplicates. Student's two-tailed *t* test was used to.evaluate the statistical significance of mean values of treatment groups. Values were considered to differ significantly if *p* ≤ 0.05.

The results showed that there was no difference between the wt and Tg animals during the primary immune response, however after the booster immunization the OVA-specific IgM and IgG titers were remarkably different. During the secondary antibody response, the IgM titers showed a typical curve in wt animals as there was a slightly lower IgM peak compared to that of the primary immune response. On the other hand, in the case of Tg mice the IgM titers were higher in the secondary immune response compared to that of the primary immune response, and significantly higher compared with the wt mice **(****Fig. 7A****).** As concerning the IgG titers, the OVA-specific IgG titers were nearly tripled in Tg mice compared with the wt animals during the secondary immune response **(****Fig. 7B****).**

### IgG subclass profile

The IgG subclass profile of OVA-specific serum immunoglobulins was determined next. At 32 days after immunization sera from wt and Tg (#14) mice were assayed for OVA specific IgG isotypes. A quantitative ELISA employing OVA as capture reagents and HRP-conjugated affinity-purified polyclonal goat anti-mouse IgG1, IgG2a, IgG2b and IgG3 (Southern Biotech Associates Inc., Birmingham, AL, USA) as detecting reagent were used to evaluate plasma concentrations of anti-OVA IgG isotypes. The peroxidase-conjugated antibody was detected using TMB as the substrate. Serial dilutions of each test serum sample were applied and the concentrations of Ig were reported based on absorbance values at 450 nm interpolated from a linear portion of the dose-response curve. Samples were assayed in duplicates.

It was found that animals immunized with OVA-CFA and then OVA-IFA showed predominantly IgG1 subclass of anti-OVA antibodies. Data showed that Tg mice generated significantly higher OVA-specific titers of IgG1, IgG2a and IgG2b. Although Tg mice generated slightly higher IgG3 compared to that of wt mice, the difference was not significant because of a large standard deviation in the results. The present data also showed that the OVA specific IgG isotypes were similarly proportioned to that of the wt mice **(****Fig. 7C****).** This indicates that besides of the differences in the quantity, the bFcRn expression in the Tg mice did not modify the OVA-specific immune response.

### Total IgG levels

A quantitative ELISA employing an unlabeled affinity purified goat anti-mouse polyclonal antibody (goat anti-mouse IgG (H+L); Southern Biotechnology Associates, Inc., Birmingham, AL, USA) as capture reagent and a horseradish peroxidase conjugated affinity purified polyclonal goat anti-mouse IgG (γ chain specific; Southern Biotechnology Associates, Inc., Birmingham, AL, USA) as detecting reagent were used to evaluate plasma concentrations of mouse IgG during the course of the experiment. The peroxidase conjugated antibody was detected using TMB (Sigma) as the substrate. Samples were assayed in duplicate.

The analysis of the total IgG levels showed that Tg (#14) mice produced significantly higher amounts of IgG compared with the wt mice even before immunization (2.4 ± 0.4 and 4.8 ± 0.5 mg/ml, mean±SEM, in wt and Tg mice, respectively; p<0.01). Following immunization, the total IgG level constantly raised and reached its peak levels on day 28 and 36 in wt and Tg animals, respectively. Noteworthy, we found a remarkable and significant difference at the highest IgG levels, which were 14.8 ± 2.6 mg/ml (mean±SEM) and 39.9 ± 2.7 mg/ml in wt and Tg mice, respectively (p<0.001) **(****Fig. 7D****).**

### Phagocytosis assay

To evaluate the functional intactness of the OVA specific IgG generated in the Tg mice, OVA-anti-OVA antibody complexes were produced with incubating OVA-FITC with increasing amounts of Tg and wt mice sera, collected at day 35 after OVA immunization, and a phagocytosis assay was performed with the mouse macrophage cell line P388D1. OVA labeling with FITC was performed by procedure described according to the manufacturer's instructions (Molecular Probes, Eugene, OR). Mouse macrophages of the P388D1 cell line were grown at 37°C in RPMI 1640 medium (Sigma-Aldrich, Budapest, Hungary) supplemented with 5% fetal calf serum (Sigma-Aldrich, Budapest, Hungary) and 10 µM 2-mercaptoethanol (Sigma-Aldrich, Budapest, Hungary). 2x10⁵ cell/well were treated at 37°C for 60 minutes with 15 ng phorbol myristate acetate (PMA, 30ng/ml, Sigma-Aldrich, Budapest, Hungary) in a 24 well cell culture plate. To produce OVA-anti-ova antibody complex, 50µg OVA-FITC (1 µg/µl) was preincubated at 37°C for 60 minutes with 2.5, 10 and 40 µl serum from OVA immunized Tg and wt mice (35 days after immunization). Phagocytosis was carried out at 37°C for 90 minutes by incubating PMA treated P388D1 cells with OVA-anti-OVA antibody complexes. After extensive washing to remove unbound proteins, cells were analyzed by flow cytometry (FACS). Trypan Blue was used to quench extracellular FITC-labeled OVA.

The efficiency of immunocomplex uptake was analyzed by FACS. There was no enhanced phagocytosis found by using 2.5 µl sera of neither the Tg nor wt mice when compared to the uptake of non-opsonized OVA-FITC. However, a significant enhancement was observed by using 10 µl sera of Tg but not of wt mice. Finally, 40 µl sera each from Tg and wt mice resulted an equally increased phagocytosis compared to the uptake of the non-opsonized antigen **(****Fig. 7E****).** These data indicated that Tg mice sera contained significantly more OVA specific antibody on day 35 of immunization, and that was functionally intact.

### Example 6 - Tg and wt mice respond similarly to FITC-dextran immunization (not claimed)

In order to analyze the immune response that generate primarily IgM, Tg and wt mice were immunized intraperitoneally with FITC-dextran and challenged them similarly two weeks later. As FITC-dextran is a hapten (FITC) conjugated to a typical T cell-independent type II antigen (dextran) (Mond et al., 1995) primarily FITC specific IgM was observed, with very little FITC specific IgG, in the sera. There was no difference between the Tg and wt animals regarding their FITC specific immune response. Analyzing the immune response during the immunization course an increased IgM production was noted after the second immunization both in the Tg and wt mice **(****Fig. 8****).**

### Example 7 - The effect of bFcRn overexpression on OVA specific IgM and IgG producing cells (not claimed)

### OVA specific B-cell analysis by ELISPOT assay

Elevated OVA specific IgM titers in Tg mice suggested that the bFcRn overexpression modifies the immune response at clonal B-cell expansion beyond of its contribution to the greater IgG rescue. In order to investigate this observation, first the number of plasma cells secreting anti-OVA IgM and anti-OVA IgG in the spleen was analyzed at day 25 after immunization by OVA specific B-cell analysis with an ELISPOT assay. Homozygote #14 and wt mice (three in each group) were immunized intraperitoneally with 250 µg OVA in CFA and challenged 14 days later with 250 µg OVA with IFA. 25 days after the first immunization the animals were killed and their spleen cells were analyzed for OVA-specific IgM and IgG cells. For ELISPOT, MultiScreen-HTS plates (Millipore, Bedford, MA) were coated with 5 µg/ml of OVA in PBS at room temperature for 3 hours. The plates were then washed six times with PBS and blocked with RPMI medium containing 5% FCS and mercaptoethanol (50 µM) for 30 min at room temperature. Serial dilutions (starting at 5x10⁵ cells/well) of spleen cells were added to the wells. The plates were incubated at 37°C with 5% CO2 overnight and washed six times with PBS-Tween; horseradish peroxidase conjugated goat anti-mouse IgG (γ heavy-chain specific; 1:4000 fold dilution; Southern Biotechnology) was then added to each well. After one-hour incubation at room temperature, the plates were washed six times with PBS-Tween. The plates were then incubated in the presence of a chromogen substrate 3-amino-9-ethylcarbazole (AEC; Sigma) and H₂O₂ at room temperature and the reaction was terminated by a water wash. The spot-forming units (SFU) per well were counted in an ImmunoScan ELIspot reader (Cellular Technology Ltd., USA) and evaluated by ImmunoSpot software ver 3.2 (Cellular Technology Ltd., USA).

The result showed that the number of OVA-specific IgM producer cells was doubled (p<0.05), while OVA specific IgG cells were more than tripled (p<0.001) in the spleen **(****Fig. 9A****).**

### Immunization results massive influx of neutrophils into the spleen

OVA immunization resulted in an increase in spleen weight **(****Fig. 9B****)** and its cell numbers **(****Fig. 9C****);** and the enlargement of the peripheral lymph nodes was also observed. This phenomenon appeared in the wt and also in Tg mice, however, spleen size was doubled in the Tg animals compared to the wt controls (p<0.001).

We then characterized the cell type distribution of the spleen by flow cytometry. For FACS, splenocytes were isolated and first incubated with anti-CD32/CD16 (clone 2.4G2) for 30 minutes. Then the cells were incubated with fluorochrome-conjugated specific Abs at 4°C for 50 min in staining buffer (PBS with 0.1% BSA and 0.1% sodium azide), washed twice, and then analyzed with using a FACSCalibur equipped with CellQuest software (BD Biosciences, San Jose, CA). Conjugated mAbs, CD45R/B220-PECy5, I-A/I-E-PE, GR-1(Ly-6G)-PE and CD11b-A647 were obtained from BD Pharmingen (San Diego, CA). CD3-A647, CD86-PE, CD11c-A647 and CD11b-PE were purchased from Caltag (Burlingame, CA), eBioscience (San Diego, CA), Serotec (Dusseldorf, Germany) and ImmunoTools GmbH (Friesoythe, Germany), respectively. Isotype controls were obtained from BD Pharmingen or Serotec.

The results showed that that after immunization the ratios of cells bearing CD45R/B220 (B lymphocytes) and I-A/I-E (MHC class II) antigens significantly reduced (p<0.05). There was not a difference or a less radical decrease regarding the cells bearing CD3 marker (T-lymphocyte) in wt and Tg mice, respectively. These phenomena appeared in wt and Tg mice, however, they were more emphasized in the Tg animals **(****Fig. 10****).**

The ratios of the cells bearing CD11b and Gr-1 markers were dramatically raised both in wt and Tg mice after immunization (p<0.001). Regarding the magnitude of this increase, we found approximately a five-fold increase in wt and nine-fold increase in Tg mice. We also found that most of the cells that expressed Gr-1, were Gr-1^{high} **(****Fig. 11A****)** with a typical granulocyte position according to forward angle/side angle light scatter (FCS/SSC) parameters, suggesting that they were neutrophils (data not shown). Again, this increase was more profound in the case of Tg mice (p<0.01) **(****Fig. 11A****).** We also found that the ratio of the cells bearing CD11b and MHC class II antigens (macrophages, dendritic cells) **(****Fig. 11B****)** and CD11b and CD11c antigens (dendritic cells) **(****Fig. 11C** - gated cells) were significantly elevated (p<0.05 and p<0.01, respectively) in Tg mice compared to their wt controls. These results explained the proportional decrease of cells bearing B220 and MHC-11, however this does not imply reduction in the total cell numbers as the spleen with its cell numbers were greater after immunization **(****Fig. 9C****).** Based on these analyses we concluded that the majority of the cells that influxed the spleen 25 days after immunization was neutrophil, however increased number of dendritic cells were also present.

### Example 8 - bFcRn is strongly expressed in peritoneal exudates neutrophils, macrophages and dendritic cells (not claimed)

Expression of the bFcRn α-chain was first analyzed in peritoneal exudates cells deriving from mice two days after they were intraperitoneally treated by Concanavalin A (ConA, 100µg/ mouse in PBS; Sigma-Aldrich, Budapest, Hungary). For a neutrophil enriched cell preparation, mice were injected i.p. with 2 ml of 5% (w/v) casein (Sigma-Aldrich, Budapest, Hungary) in sterile saline and peritoneal cells were isolated 6 hours after injection. Neutrophils were then purified by Ficoll-Paque Plus (GE Healthcare, Uppsala, Sweden) centrifugation (400 × g for 30 min at RT). The purity of the neutrophil was ~80% as determined by flow cytometry using anti-CD11b and anti-Gr-1 reagents **(****Fig. 12****).**

Total RNA was extracted by using Trizol Reagent (Invitrogen, Carlsbad, CA) from these cells and two micrograms of RNA was reverse transcribed by using Moloney-murine leukemia virus (M-MLV) reverse transcriptase enzyme (Promega) and the (dT)17-adapter primer by using standard protocols. PCR was performed to obtain a 422 bp long bFcRn α-chain specific amplicon (289-711 bp of AF139106) by the primer pairs: B3 5'-CGCAGCARTAYCTGASCTACAA-3' (SEQ.ID.NO.: 37, where R can be G or A, Y can be T or C and S can be G or C), and B4 5'-GGCTCCTTCCACTCCAGGTT-3' (SEQ.ID.NO.: 38). The amplified segment was separated by electrophoresis on 1% agarose gel and stained with ethidium bromide.

BFcRn expression was detected by PCR amplification first in peritoneal exudates cell from Tg and wt mice after they were treated by ConA. bFcRn expression was also shown in a neutrophil and macrophage enriched cell population, as the purified cells were ~78% CD11b^{high} / Gr-1^{high} and ~20 % CD11b^{high} / Gr-1⁻ **(****Fig. 12****).**

A more recent study revealed that FcRn fulfils a major role in IgG-mediated phagocytosis in polymorphonuclear leukocytes and monocytes (Vidarsson et al., 2006) which suggest that FcRn is involved in antigen presentation. Based on our data, it can be hypothesized that bFcRn overexpression in these cells mediates enhanced antigen phagocytosis and even antigen presentation, which then results more antigen specific IgM and IgG producing plasma cells in the secondary lymphoid organs. Should this theory be correct this enhancement was obvious once IgG is produced and not earlier. Indeed, increased IgM titers were only found after the OVA specific IgG appeared, mainly in the secondary immune response.

### Example 9 - Presence of B-lymphocytes, neutrophils and dendritic cells bearing OVA in the spleen of immune mice (not claimed)

Our cellular analysis showed that the dominant cell type that influxed the spleen after immunization was neutrophils. Based on another recent report, neutrophils comprised the main population of cells bearing the antigen in secondary lymphoid organs; once there was a specific immune response occurring and IgG-antigen immunocomplex was formed and it contributed to the quality of the established secondary immune response (Maletto et al., 2006). To test whether neutrophil influx was indeed dependent on an antigen specific immune response and carried antigens in our experiments, transgenic mice 56 days after OVA immunization (when OVA specific IgG level is still high - **Fig. 7B****)** and without immunization were intraperitoneally treated with FITC-OVA. In this experiment the mice (homozygote #14), were intraperitoneally treated with OVA-FITC (7.4 mg/ml, 100 µl/mouse). 5 hours after OVA-FITC injection mice were splenectomized and its cells have been analyzed by flow cytometry and confocal microscopy. For *flow cytometry,* the cells were treated as described, with the following reagents: CD45R/B220-PECy5, GR-1(Ly-6G)-PE, CD11c-A647, CDllb- A647 and CD11b-PE. For *confocal microscopy* the cells were stained with a DRAQ5 red fluorescent cell-permeable DNA probe (Biostatus Ltd., United Kingdom) for 10 min at RT. After a washing step, fluorescence and DIC images (512×512 pixels) were recorded with an Olympus FLUOView500 laser scanning confocal microscope (Hamburg, Germany), at high magnification (63× objective). The 488 nm argon laser and 632 He-Ne laser lines were used to excite FITC and DRAQ5 dyes.

Our flow cytometry data showed that among the OVA-FITC positive cells (15.1±1.4%), 61.2±5.4% were B220 positive (B-lymphocytes), 18.5±0.6% were detected as CD11b^{high} and Gr-1^{high} (neutrophils) and 13.5±2.1% were CD11b and CD11c positive (dendritic) cells in the OVA immunized animals. We did not find considerable number of OVA-FITC positive cells in non-immunized animals (2.1±0.3%) **(Fig. 13A and 13B).** Our data from flow cytometry, regarding OVA-FITC positive neutrophils and B-lymphocytes were confirmed by confocal microscopy. We found typical neutrophils that have polylobed nuclei internalizing OVA-FITC, while other cells, presumably B-lymphocytes (having large, round shaped nuclei and thin rims of cytoplasm) were coated by OVA-FITC **(Fig. 13C).** We did not find significant difference in the spleen size and cell numbers between the immunized and non-immunized animals (data not shown). This finding fits very well to the other results we found and give explanation why there is a remarkable influx of CD11b and Gr-1 positive neutrophils in our wt and Tg mice, 25 days after immunization. Considering that Tg mice produced much more OVA specific IgG **(****Fig. 7B****)** and overexpress FcRn in these cells **(****Fig. 12****),** we can also explain why Tg mice show greater influx of these cells into the spleen, larger antigen specific B cell clonal expansion and consequently a robust antibody response.

### Example 10-Generation of bFcRn transgenic mice with lentiviral transgenesis (not claimed)

Recently HIV and EIAV derived lentiviral vectors were used to achieve reproducible high transgenesis rates in mice (Pfeifer et al., 2002), rats (Lois et al., 2002), chickens (McGrew et al., 2004), pigs (Hofmann et al., 2003; Whitelaw et al., 2004) and cattle (Hofmann et al., 2004). The WPRE-P2_bFcRn transfer vector was produced by replacing the original EF1α-EGFP segment of the transfer vector pWPTS-EGFP (Bovia et al., 2003) by an artificially combined 2950 bp long promoter segment of the bovine FcRn (bFcRn) α-chain gene with the coding sequence of the same gene. The bFcRn promoter segment was PCR amplified with Deep Vent polymerase (New England Biolab, Beverly, MA, USA) from the BAC clone #128E04 that harbours the bFcRn α-chain gene and has been used to make the bFcRn transgenic mice (see Example 2). The forward primer contains an XbaI site (italic) (lenti-BORE20: 5-GGG *TCT AGA* ACA CCA AGG GCG GCA TCA-3, SEQ.ID.NO.: 39); the reverse primer contains an EcoRI site (lenti-BORE18: 5-GGG *GAA TTC* CGG CTC CCG TGA CTG GAG AC-3, SEQ.ID.NO.: 40). The PCR generated amplicon was 2950 bp long of the bFCGRT regulator sequence (GenBank: nucleotide 765455 - 762510 base pairs of the NW 001493624.1 clone/Bt18 WGA2132 3/Bos taurus chromosome 18 genomic contig, reference assembly (based on Beau_3.1). The coding segment of the bFcRn heavy chain was PCR amplified with Deep Vent polymerase from a clone that contained the bFcRn cDNA (GenBank AF139106) and had been previously used for making stably transfected cells in our laboratory (Kacskovics et al., 2006a). The forward primer contains an EcoRI site (BORE10. 5-GGG *GAA TTC* TGG GGC CGC AGA GGG AAG G-3, SEQ.ID.NO.: 41); the reverse primer contains an MluI site (lenti-BORE19: 5-GGG *ACG CGT* GAG GCA GAT CAC AGG AGG AGA AAT-3, SEQ.ID.NO.: 42). The PCR generated amplicon was 1285 bp long of the bFcRn α-chain cDNA (GenBank: nucleotide 64 - 1344 of the NM_176657 reference sequence of the bFcRn). After purification, the two amplicons (promoter and coding sequence of the bFcRn) were digested with EcoRI (Promega) and ligated by using T4 ligase (Promega) to produce a P2-bFcRn construct encoding a 2950 bp segment of the bFcRn heavy chain promoter and a 1285 bp segment of the bFcRn heavy chain cDNA (SEQ.ID.NO.: 43). The bFcRn promoter-cDNA fragment was then inserted into the transfer vector plasmid pWPTS. The vectors were amplified by transient transfection into 293T cells as described previously (Bovia et al., 2003). Vectors used to create lentivirus were obtained from Tronolab, Cantonal Medical University, Geneva, Switzerland. The following vectors were used: pMD.G (envelope construct, 6kbp); pCMV R8.91 (packaging construct, 12kbp); pWPTS (transfer construct, 12.7kbp, in which the FcRn promoter (P2)-FcRn cDNA sequence was inserted between the ClaI és SalI sites. Lentiviral titers were estimated by the transduction of Jurkat cells with serial dilutions of supernatants of a virus stock prepared in parallel. This virus harbored the pWPRE-EGFP transfer vector. GFP+ cells were counted by fluorescence activated cell sorter (FACS). Titer was in the range of 10⁷ - 10⁸ transducing unit per milliliter. Transgenic mice were created by injecting the lentivirus HIV-P2-FcRn into the perivitelline space of one cell stage zygotes. Microinjections were performed on FVB/N and Balb/c genetic backgrounds, Injected zygotes were then transferred into recipient females **(Table 6).** Transgenic founders were identified by PCR with the following primers: bFcSuf 5' CTCCTTTGTCTTGGGCACTT 3' (SEQ.ID.NO.: 9) and bFcL 5' GCCGCGGATCCCTTCCCTCTG 3' (SEQ.ID.NO.: 10).

**Table 6. Data on lentiviral injected mouse embryos**

| | FVB/N | Balb/c |
|---|---|---|
| Embryos injected and transferred | 37 | 38 |
| No of recipient females | 2 | 3 |
| Successful transfers | 2 | 1 |
| No. of newborns | 17 or 18 | 4 |
| Newborns alive | 13 | 4 |
| PCR+ (transgenic) | 5 | 1 |

To detect transgene expression, RT-PCR was performed on the RNA samples isolated from the liver, spleen, lung and intestine of a newborn HIV-P2-FcRn mice as shown in **Fig. 14****.** Primers planned for P2 promoter (FcRnprf: CGGCCACCTCTATCACATTT, SEQ.ID.NO.: 3; FcRnprr: TGCATTGACCACACTTGGTT, SEQ.ID.NO.: 4; expected fragment size: 579 bp) were included to exclude the possibility of contaminating genomic DNA in the RNA samples, and the transgene specific mRNA was detected with primers planned for bovine FcRn exon 4 (bFcRnex4f: CCAAGTTTGCCCTGAACG, SEQ.ID.NO.: 19; bFcRnex4r: GAGGCAGATCACAGGAGGAG, SEQ.ID.NO.: 44, expected fragment size: 161 bp). The results clearly indicate the expression of bFcRn mRNA.

Transgenic founders are bred individually by mating with wild type mice to establish transgenic lines.

### Example 11 -The level of albumin is also increased in the serum of Tg mice (not claimed)

Since the transgenic mice overexpress bFcRn, two points were investigated: (1) whether bFcRn interacts with mouse albumin and (2) if albumin metabolism was influenced. Thus, the albumin levels of mice that do not express FcRn (FcRn KO - purchased from The Jackson Laboratory, USA), wt animals, homozygous #14 and #19 (express 4 and 10 copies of bFcRn, respectively) were measured by using albumin specific sandwich ELISA. Based on this study, it was found that bFcRn binds albumin and it seriously influences its serum level **(****Fig. 15****).** The albumin content in the milk of the transgenic mice was also measured and it was found that it contains significantly higher concentration compared to its wild type controls (data not shown). These data suggest, therefore, that besides of the advantageous effect of the FcRn overexpression on the humoral immune response, albumin concentration is also elevated, which might be harmful, as it makes higher than normal level of osmotic pressure in the blood, although at even the copy number of 10, no perceptible harmful effects were there, and the Tg animals lived a long and healthy life a up to 14 months.

### Example 12 - In vivo study analyzing the rabbit IgG half-life in mouse FcRn KO - bovine BAC transgenic mice (not claimed)

In order to analyze if bFcRn binds rabbit IgG and protects it from fast clearance, we analyzed the rabbit IgG half-life in mouse FcRn KO - bovine BAC transgenic mice. Bovine FcRn transgenic, mouse FcRn α-chain null mice (bFcRn/mFcRn^{-/-}) were created by crossbreeding the bFcRn homozygote mice (#14) carrying 4 copies of the 128E04 BAC transgene (Bender et al., 2007) with the mouse strain homozygote for the knockout allele of mFcRn. This mouse strain was purchased form The Jackson Laboratories (USA) under name: B6.129X1-Fcgrt^{tmlDcr/Dcr}**.** Double transgenic mice with the desired genotype (bFcRn/mFcRn^{-/-}) were selected by multiplex PCR with the following primers: NEO-F: GGA ATT CCC AGT GAA GGG C (SEQ.ID.NO.: 45); NEO-R: CGA GCC TGA GAT TGT CAA GTG TAT T (SEQ.ID.NO.: 46); FcRn wt-F: GGG ATG CCA CTG CCC TG (SEQ.ID.NO.: 47); bFcSuF: CTC CTT TGT CTT GGG CAC TT (SEQ.ID.NO.: 9); bFcL: GCC GCG GAT CCC TTC CCT CTG (SEQ.ID.NO.: 10). A suitable F2 mouse was identified as shown on **Fig. 16A****.**

The rabbit IgG half-life was analyzed essentially as described in Example 4. Briefly, following a prebleed, age, weight and sex (male) matched mFcRn^{-/-} and bFcRn^{+/+}mFcRn^{-/-} mice (three in each group) were microinjected intravenously with 150 µg of rabbit IgG (Sigma) in 50 mg/ml saline solution and during the next 216 hours, periodic blood samples (50 µl/occasion) were collected from retroorbital plexus. A quantitative ELISA employing goat anti-rabbit IgG (H+L specific) (Caltag) as capture reagent and an HRP-conjugated goat anti-rabbit IgG (H+L specific) (Vector Laboratories) as detecting reagent was used to evaluate plasma concentrations of rabbit IgG during the course of the experiment. The peroxidase-conjugated antibody was detected using TMB (3,3',5,5'-tetramethylbenzidine; Sigma) as the substrate. The samples were assayed in duplicates. Concentrations of rabbit IgG were reported based on a reference standard. Rabbit IgG was cleared rapidly in FcRn^{-/-} mice (half-life: 15 hours), but it was protected in bFcRn/FcRn^{-/-} (half-life: 67 hours) **(****Fig. 16B****).** The fast IgG clearance in FcRn^{-/-} animals is documented (Roopenian et al., 2003) and also in animals that are lacking functional FcRn due to the lack of the beta-2-microglobulin (Ghetie et al., 1996; Israel et al., 1996; Junghans and Anderson, 1996). In our experiment, the rabbit IgG clearance was significantly reduced (p<0.0001) in bFcRn/mFcRn^{-/-} animals due to the activity of the bFcRn. Nevertheless, the half-life of the rabbit IgG in these animals is still much shorter compared to the half-life of the rabbit IgG in rabbit (half-life: 132-153 hours (Andersen and Bjorneboe, 1964; Dima et al., 1983; Sabiston and Rose, 1976) or in mouse (half-life: 106 hours) (Dima et al., 1983), suggesting that the interaction of rabbit IgG and bFcRn/mouse beta-2-microglobulin complex is relatively weak. Indeed, previous data detected differences in IgG-FcRn interactions across species (Ober et al., 2001), emphasizing the need to the experimental selection of the appropriate FcRn for increasing the IgG half-life and consequently its level upon immunization.

### Example 13 - Creation of double transgenic rabbits which are producing humanized immunoglobulins and overexpressing the bovine FcRn by crossing

Double transgenic rabbit lines producing human IgG and bovine FcRn are good candidates for improving the immunoglobulin level in the serum. The effect of the overexpressed bovine FcRn on the half-life of human IgG has been already shown, therefore these double transgenic rabbit lines are very useful for the production of polyclonal antisera, or as a starting point to generate rabbit monoclonal antibodies.

The double Tg rabbit can be generated by standard grossing Although the previous experiment showed that bFcRn is not the best candidate with respect to binding to rabbit IgG, generating a bFcRn transgenic rabbit would be still a desirable intermediate step to create the double transgenic rabbit producing both human IgG and bovine FcRn. In a later step, two Tg rabbit lines, each harboring one of the transgenes, may be crossed in order to generate the final animal.

The 128E04 bovine BAC clone was introduced with pronuclear microinjection into rabbit zygotes and a transgenic founder was generated. The presence of the bovine FcRn gene was detected by PCR, with primers designed to amplify a 160-bp fragment from the bovine FCGRT 4th exon: bFcRnex4F: 5'-CCAAGTTTGCCCTGAACG 3' (SEQ.ID.NO.: 19) and bFcRnex4R: 5'- GTGTGGGCAGGAGTAGAGGA - 3' (SEQ.ID.NO.: 20). **Fig. 17** shows the presence of the fragment as expected.

F1 litters of the founder rabbit will be suitable to cross with genetically modified rabbits producing humanized immunoglobulins (Thorey et al., 2006) to result the double transgenic animals suitable for enhanced levels of humanized polyclonal antibody production.

### Example 14 - Creation of double transgenic rabbits which are producing humanized immunoglobulins and overexpressing the bovine FcRn

As an alternative to Example 13, double transgenic rabbit lines can be generated by using transgenic rabbits producing humanized immunoglobulins (Thorey et al., 2006) as receiving animals in similar transgenesis experiments as described in Example 3. Using the 128E04 bovine BAC clone, transgenic rabbit lines are generated with pronuclear microinjection. Transgenic rabbits are identified with Southern blot and/or PCR using primers and probes specific for the bovine BAC clone, and the half-lives of injected human IgG are evaluated. A substantially longer half-life in the sera of the rabbits indicates that the introduced bovine FcRn protects the human immunoglobulin produced by the transgenic rabbit from degradation.

### Example 15 - Isolation and characterization of a BAC clone that harbors the rabbit FcRn α-chain gene

As many of the early studies on FcRn activity used rabbit as model animal, it was predicted that the rabbit FcRn is expressed by endothelial cells among other tissues and contribute to IgG homeostasis. Therefore, addition of extra copies of the rabbit FcRn gene results increased IgG half-life. Accordingly, the rabbit FcRn was cloned and sequenced (SEQ.ID.NO.: 48).

In a parallel effort, the rabbit BAC clone 262E02 was isolated from a rabbit BAC library (Rogel-Gaillard et al., 2001). The BAC library was constructed in the pBeloBAC11 vector, the high molecular weight DNA was prepared from white blood cells of a New Zealand rabbit. The rabbit BAC library is handled by the INRA resource centre for domestic animals and is publicly available. The rabbit FCGRT gene specific primers: OCU_FCGRT_F: GGGACTCCCTCCTTCTTTGT (SEQ.ID.NO.: 49) and OCU_FCGRT_R: AGCACTTCGAGAGCTTCCAG (SEQ.ID.NO.: 50) were planned by the Primer 3 program (http://bioinfo.genopole-toulouse.prd.fr/iccare/cgi-bin/primer3_aTg.cgi.pl) on the rabbit expressed sequence tag (EST) EB377775 was identified by aligning it with the human FCGRT gene (GenBank NM_004107). with the Iccare program (http://bioinfo.genopole-toulouse.prd.fr/Iccare/. The EB 377775 EST sequence is identical with the corresponding part of the rabbit FcRn cDNA (SEQ.ID.NO.: 55). The 262E02 rabbit BAC clone was analysed for the presence of candidate genes based on the orthologous *Bos Taurus* chromosome. Primers were planned either for *Oryctolagus cuniculus* ESTs or the bovine gene specific primers were used in 50 kb vicinity of the FCGRT gene in 5' and 3' directions. The following rabbit genes were identified on the 262E02 BAC clone: RPL13A; RPS11; FCGRT; RCN3, PRRG2 (see **Fig. 18****).**

**Table 7. Primers and PCR conditions for evaluating the presence of orthologous bovine genes on the the rabbit BAC clone 262E02**

| Putative gene | Primer | Tm (°C) | Product (bp) |
|---|---|---|---|
| PRRG | PRRG2_L: 5'-GTTGTTCACACCAGGGAACC-3' (SEQ.ID.NO.: 25) | 61 | 295 |
| | PRRG2_R: 5'-CCTTTGCCATTGTAGATGTAGC-3' (SEQ.ID.NO.: 26) | | |
| RCN3 | RCN3_L: 5'-GACGCCGAGACCTACAAGAA-3' (SEQ.ID.NO.: 51) | 61 | 168 |
| | RCN3_R: 5'-CATGTGCGGGAACTCCTC-3'(SEQ.ID.NO.: 52) | | |
| FCGRT | FCGRT_L: 5'-CTGAACGGTGAGGACTTCAT-3' (SEQ.ID.NO.: 53) | 61 | 210 |
| | FCGRT_R: 5'-GCTCCTTCCACTCCAGGTT-3' (SEQ.ID.NO.: 54) | | |
| RPS11 | RPS11_L: 5'-AGATCGGCGACATCGTCA-3' (SEQ.ID.NO.: 55) | 61 | 108 |
| | RPS11_R: 5-TCTGGAACTGCTTCTTGGTG-3'(SEQ.ID.NO.: 56) | | |
| RPL13A | RPLI3A_L: 5'-CATGAGGTGGGCTGGAAGTA-3' (SEQ.ID.NO.: 57) | 61 | 82 |
| | RPL13A_R:5'-TCCGGTAGTGGATCTTAGCC-3'(SEQ.ID.NO.:58) | | |

The presence of the FLT3LG gene was also detected, based on a slot-blot analysis, when a bovine FLT3LG 547 bp long PCR product produced by the primers: FLT3LG_L: 5'-TCGGAGATGGAGAAACTGCT-3' (SEQ.ID.NO.: 15) and FLT3LG_R: 5'-CTGGACGAAGCGAAGACAG-3' (SEQ.ID.NO.: 16) was hybridized to 262E02 rabbit BAC and resulted strong positive signal under high stringency conditions.

The structure of the rabbit BAC clone 262E02 seems very similar to that of the bovine 128E04 bovine BAC clone, therefore, it can be expected to provide similar good results in transgenesis experiments.

### Example 16 - Creation of transgenic rabbits overexpressing the rabbit FcRn and in vivo studies to analyze the rabbit IgG half-lives

A genetic construct that harbors either the rabbit BAC clone 262E02 characterized in the previous example, or a construct comprising the rabbit FcRn cDNA (SEQ.ID.NO.: 48) driven by the well characterized bFcRn promoter and supplemented downstream of the rabbit FcRn cDNA with a heterologous intron and a commercially available SV40 polyA region is introduced with pronuclear microinjection into rabbit zygotes. Alternatively, transgenic rabbits could be created with the P2-rabbitFcRn cDNA construct inserted into a lentiviral transfer vector as described in Example 10 and injected into the perivitelline space of rabbit embryos. Transgenic rabbits are identified with Southern blot and/or PCR using specific primers/probes. IgG half-lives in the rabbit FcRn overexpressing transgenic rabbits will be evaluated by standard methods, for example by those exemplified above.

The transgenic rabbits overexpressing the rabbit FcRn α-chain thus produced can be advantageously used for the enhanced production of polyclonal antisera.

### References

Abbas A. K. and Lichtman A. H. (2003) Cellular and molecular immunology. WB Saunders Co, Philadelphia.
Adamski F. M., King A. T. and Demmer J. (2000) Expression of the Fc receptor in the mammary gland during lactation in the marsupial Trichosurus vulpecula (brushtail possum). Mol Immunol 37, 435-44.
Ahouse J. J., Hagerman C. L., Mittal P., Gilbert D. J., Copeland N. G., Jenkins N. A. and Simister N. E. (1993) Mouse MHC class I-like Fc receptor encoded outside the MHC. J Immunol 151, 6076-88.
Akilesh S., Christianson G. J., Roopenian D. C. and Shaw A. S. (2007) Neonatal FcR Expression in Bone Marrow-Derived Cells Functions to Protect Serum IgG from Catabolism. J Immunol 179,4580-8.
Andersen J. T., Dee Qian J. and Sandlie I. (2006) The conserved histidine 166 residue of the human neonatal Fc receptor heavy chain is critical for the pH-dependent binding to albumin. Eur J Immunol 36, 3044-51.
Andersen S. B. and Bjomeboe M. (1964) Gamma Globulin Turnover in Rabbits before and During Hyperimmunization. J Exp Med 119, 537-46.
Anderson C. L., Chaudhury C., Kim J., Bronson C. L., Wani M. A. and Mohanty S. (2006) Perspective - FcRn transports albumin: relevance to immunology and medicine. Trends Immunol 27, 343-8.
Antohe F., Radulescu L., Gafencu A., Ghetie V. and Simionescu M. (2001) Expression of functionally active FcRn and the differentiated bidirectional transport of IgG in human placental endothelial cells. Hum Immunol 62, 93-105.
Ausubel F. M., Brent R., Kingston R. E., Moore D. D., Seidman J. G., Smith J. A. and Struhl K. (1998) Current protocols in Molecular Biology. John Wiley & Sons., Hoboken, NJ, USA.
Beers M. H. and Berkow R. (1999) The Merck Manual of Diagnosis and Therapy. Merck Research Laboratories, New York, N.J.
Bender B., Bodrogi L., Mayer B., Schneider Z., Zhao Y., Hammarstrom L., Eggen A., Kacskovics I. and Bosze Z. (2007) Position independent and copy-number-related expression of the bovine neonatal Fc receptor alpha-chain in transgenic mice carrying a 102 kb BAC genomic fragment. Transgenic Res 16, 613-27.
Bender B., Bodrogi L., Yaofeng Z., Kacskovics I., Hammarstrom L. and Bösze Z. (2004) Generation of bovine FcRn alpha chain BAC transgenic mice. Tissue Antigens 64, 374-375.
Borvak J., Richardson J., Medesan C., Antohe F., Radu C., Simionescu M., Ghetie V. and Ward E. S. (1998) Functional expression of the MHC class I-related receptor, FcRn, in endothelial cells of mice. Int Immunol 10, 1289-98.
Bovia F., Salmon P., Matthes T., Kvell K., Nguyen T. H., Wemer-Favre C., Barnet M., Nagy M., Leuba F., Arrighi J. F., Piguet V., Trono D. and Zubler R. H. (2003) Efficient transduction of primary human B lymphocytes and nondividing myeloma B cells with HIV-1-derived lentiviral vectors. Blood 101, 1727-33.
Brambell F. W. R. (1958) The passive immunity of the young mammal. Biological Reviews 33, 488-531.
Brambell F. W. R., Hemmings W. A. and Morris I. G. (1964) A theoretical model of gammaglobulin catabolism. Nature 203, 1352-1355.
Butler J. E. (2006) Why I agreed to do this. Dev Comp Immunol 30, 1-17.
Chaudhury C., Brooks C. L., Carter D. C., Robinson J. M. and Anderson C. L. (2006) Albumin binding to FcRn: distinct from the FcRn-IgG interaction. Biochemistry 45, 4983-90.
Chaudhury C., Mehnaz S., Robinson J. M., Hayton W. L., Pearl D. K., Roopenian D. C. and Anderson C. L. (2003) The Major Histocompatibility Complex-related Fc Receptor for IgG (FcRn) Binds Albumin and Prolongs Its Lifespan. The Journal of Experimental Medicine 197, 315-322.
Cibelli J. B., Stice S. L., Golueke P. J., Kane J. J., Jerry J., Blackwell C., Ponce de Leon F. A. and Robl J. M. (1998) Transgenic bovine chimeric offspring produced from somatic cell-derived stem-like cells. Nat Biotechnol 16, 642-6.
Claypool S. M., Dickinson B. L., Wagner J. S., Johansen F. E., Venu N., Borawski J. A., Lencer W. I. and Blumberg R. S. (2004) Bidirectional transepithelial IgG transport by a strongly polarized basolateral membrane Fcgamma-receptor. Mol Biol Cell 15, 1746-59.
Cowan P. J., Tsang D., Pedic C. M., Abbott L. R., Shinkel T. A., d'Apice A. J. and Pearse M. J. (1998) The human ICAM-2 promoter is endothelial cell-specific in vitro and in vivo and contains critical SpI and GATA binding sites. J Biol Chem 273, 11737-44.
Dickinson B. L., Badizadegan K., Wu Z., Ahouse J. C., Zhu X., Simister N. E., Blumberg R. S. and Lencer W. I. (1999) Bidirectional FcRn-dependent IgG transport in a polarized human intestinal epithelial cell line. J Clin Invest 104, 903-11.
Dima S., Medesan C., Mota G., Moraru I., Sjoquist J. and Ghetie V. (1983) Effect of protein A and its fragment B on the catabolic and Fc receptor sites of IgG. Eur J Immunol 13, 605-14.
Dobie K. W., Lee M., Fantes J. A., Graham E., Clark A. J., Springbett A., Lathe R. and McClenaghan M. (1996) Variegated transgene expression in mouse mammary gland is determined by the transgene integration locus. Proc Natl Acad Sci U S A 93, 6659-64.
Eggen A., Gautier M., Billaut A., Petit E., Hayes H., Laurent P., Urban C., Pfister-Genskow M., Eilertsen K. and Bishop M. D. (2001) Construction and characterization of a bovine BAC library with four genome-equivalent coverage. Genet Sel Evol 33, 543-8.
Emanuel S. L. and Pestka S. (1993) Amplification of specific gene products from human serum. Genet Anal Tech Appl 10, 144-6.
Ghetie V., Hubbard J. G., Kim J. K., Tsen M. F., Lee Y. and Ward E. S. (1996) Abnormally short serum half-lives of IgG in beta 2-microglobulin-deficient mice. Eur J Immunol 26, 690-6.
Ghetie V. and Ward E. S. (2002) Transcytosis and catabolism of antibody. Immunol Res 25, 97-113.
Giraldo P. and Montoliu L. (2001) Size matters: use of YACs, BACs and PACs in transgenic animals. Transgenic Res 10, 83-103.
Guyton A. C. (1991) Textbook of Medical Physiology. W.B. Saunders Company.
Hao Y. H., Yong H. Y., Murphy C. N., Wax D., Samuel M., Rieke A., Lai L., Liu Z., Durtschi D. C., Welbern V. R., Price E. M., McAllister R. M., Turk J. R., Laughlin M. H., Prather R. S. and Rucker E. B. (2006) Production of endothelial nitric oxide synthase (eNOS) over-expressing piglets. Transgenic Res.
Harmsen M. M., Van Solt C. B., Fijten H. P. and Van Setten M. C. (2005) Prolonged in vivo residence times of Ilama single-domain antibody fragments in pigs by binding to porcine immunoglobulins. Vaccine 23, 4926-34.
Hayes H., Petit E., Lemieux N. and Dutrillaux B. (1992) Chromosomal localization of the ovine beta-casein gene by non-isotopic in situ hybridization and R-banding. Cytogenet Cell Genet 61, 286-8.
Hofmann A., Kessler B., Ewerling S., Weppert M., Vogg B., Ludwig H., Stojkovic M., Boelhauve M., Brem G., Wolf E. and Pfeifer A. (2003) Efficient transgenesis in farm animals by lentiviral vectors. EMBO Rep 4, 1054-60.
Hofmann A., Zakhartchenko V., Weppert M., Sebald H., Wenigerkind H., Brem G., Wolf E. and Pfeifer A. (2004) Generation of transgenic cattle by lentiviral gene transfer into oocytes. Biol Reprod 71, 405-9.
Israel E. J., Patel V. K., Taylor S. F., Marshak-Rothstein A. and Simister N. E. (1995) Requirement for a beta 2-microglobulin-associated Fc receptor for acquisition of maternal IgG by fetal and neonatal mice. J Immunol 154, 6246-51.
Israel E. J., Wilsker D. F., Hayes K. C., Schoenfeld D. and Simister N. E. (1996) Increased clearance of IgG in mice that lack beta 2-microglobulin: possible protective role of FcRn. Immunology 89, 573-8.
Janeway Jr. C. A., Travers P., Walport M. and Shlomchik M. J. (2001) Immunobiology: the immune system in health and disease. Garland Publishing, New York, NY.
Junghans R. P. (1997) Finally! The Brambell receptor (FcRB). Mediator of transmission of immunity and protection from catabolism for IgG. Immunol Res 16,29-57.
Junghans R. P. and Anderson C. L. (1996) The protection receptor for IgG catabolism is the beta2-microglobulin-containing neonatal intestinal transport receptor. Proc Natl Acad Sci U S A 93, 5512-6.
Jurka J., Kapitonov V. V., Pavlicek A., Klonowski P., Kohany O. and Walichiewicz J. (2005) Repbase Update, a database of eukaryotic repetitive elements. Cytogenet Genome Res 110, 462-7.
Kacskovics I., Kis Z., Mayer B., West A. P., Jr., Tiangco N. E., Tilahun M., Cervenak L., Bjorkman P. J., Goldsby R. A., Szenci O. and Hammarstrom L. (2006a) FcRn mediates elongated serum half-life of human IgG in cattle. Int Immunol 18, 525-36.
Kacskovics I., Mayer B., Kis Z., Frenyo L. V., Zhao Y., Muyldermans S. and Hammarstrom L. (2006b) Cloning and characterization of the dromedary (Camelus dromedarius) neonatal Fc receptor (drFcRn). Dev Comp Immunol 30, 1203-15.
Kacskovics I., Wu Z., Simister N. E., Frenyo L. V. and Hammarstrom L. (2000) Cloning and characterization of the bovine MHC class I-like Fc receptor. J Immunol 164, 1889-97.
Karlsson R., Michaelsson A. and Mattsson L. (1991) Kinetic analysis of monoclonal antibody-antigen interactions with a new biosensor based analytical system. J Immunol Methods 145, 229-40.
Kobayashi N., Suzuki Y., Tsuge T., Okumura K., Ra C. and Tomino Y. (2002) FcRn-mediated transcytosis of immunoglobulin G in human renal proximal tubular epithelial cells. Am J Physiol Renal Physiol 282, F358-65.
Kohler G. and Milstein C. (1975) Continuous cultures of fused cells secreting antibody of predefined specificity. Nature 256, 495-7.
Leenaars M. and Hendriksen C. F. (2005) Critical steps in the production of polyclonal and monoclonal antibodies: evaluation and recommendations. Ilar J 46, 269-79.
Lin Z. and Floros J. (2000) Protocol for genomic DNA preparation from fresh or frozen serum for PCR amplification. Biotechniques 29, 460-2, 464, 466.
Lipman N. S., Jackson L. R., Trudel L. J. and Weis-Garcia F. (2005) Monoclonal versus polyclonal antibodies: distinguishing characteristics, applications, and information resources. Ilar J 46, 258-68.
Lobo E. D., Hansen R. J. and Balthasar J. P. (2004) Antibody pharmacokinetics and pharmacodynamics. J Pharm Sci 93, 2645-68.
Lois C., Hong E. J., Pease S., Brown E. J. and Baltimore D. (2002) Germline transmission and tissue-specific expression of transgenes delivered by lentiviral vectors. Science 295, 868-72.
Lonberg N. (2005) Human antibodies from transgenic animals. Nat Biotechnol 23, 1117-25.
Lu W., Zhao Z., Zhao Y., Hammarström L. and Li N. (2006) Over-expression of the bovine neonatal Fc receptor gene in the mammary gland of transgenic mice. In 8th World Congress on Genetics Applied to Livestock Production, Belo Horizonte, MG, Brasil.
Lu W., Zhao Z., Zhao Y., Yu S., Zhao Y., Fan B., Kacskovics I., Hammarstrom L. and Li N. (2007) Over-expression of the bovine FcRn in the mammary gland results in increased IgG levels in both milk and serum of transgenic mice. Immunology 122,401-8.
Maletto B. A., Ropolo A. S., Alignani D. O., Liscovsky M. V., Ranocchia R. P., Moron V. G. and Pistoresi-Palencia M. C. (2006) Presence of neutrophil-bearing antigen in lymphoid organs of immune mice. Blood 108, 3094-102.
Manz R. A., Hauser A. E., Hiepe F. and Radbruch A. (2005) Maintenance of serum antibody levels. Annu Rev Immunol 23, 367-86.
Mayer B., Kis Z., Kajan G., Frenyo L. V., Hammarstrom L. and Kacskovics I. (2004) The neonatal Fc receptor (FcRn) is expressed in the bovine lung. Vet Immunol Immunopathol 98, 85-9.
Mayer B., Zolnai A., Frenyo L. V., Jancsik V., Szentirmay Z., Hammarstrom L. and Kacskovics I. (2002) Redistribution of the sheep neonatal Fc receptor in the mammary gland around the time of parturition in ewes and its localization in the small intestine of neonatal lambs. Immunology 107, 288-96.
McCarthy K. M., Yoong Y. and Simister N. E. (2000) Bidirectional transcytosis of IgG by the rat neonatal Fc receptor expressed in a rat kidney cell line: a system to study protein transport across epithelia. J Cell Sci 113, 1277-85.
McCullough K. C. and Summerfield A. (2005) Basic concepts of immune response and defense development. Ilar J 46, 230-40.
McGrew M. J., Sherman A., Ellard F. M., Lillico S. G., Gilhooley H. J., Kingsman A. J., Mitrophanous K. A. and Sang H. (2004) Efficient production of germline transgenic chickens using lentiviral vectors. EMBO Rep 5, 728-33.
Mond J. J., Lees A. and Snapper C. M. (1995) T cell-independent antigens type 2. Annu Rev Immunol 13, 655-92.
Ober R. J., Martinez C., Lai X., Zhou J. and Ward E. S. (2004) Exocytosis of IgG as mediated by the receptor, FcRn: an analysis at the single-molecule level. Proc Natl Acad Sci U S A 101, 11076-81.
Ober R. J., Radu C. G., Ghetie V. and Ward E. S. (2001) Differences in promiscuity for antibody-FcRn interactions across species: implications for therapeutic antibodies. Int Immunol 13, 1551-9.
Opsahl M. L., Springbett A., Lathe R., Colman A., McClenaghan M. and Whitelaw C. B. (2003) Mono-allelic expression of variegating transgene locus in the mouse. Transgenic Res 12,661-9.
Palmiter R. D., Wilkie T. M., Chen H. Y. and Brinster R. L. (1984) Transmission distortion and mosaicism in an unusual transgenic mouse pedigree. Cell 36, 869-77.
Peterson N. C. (2005) Advances in monoclonal antibody technology: genetic engineering of mice, cells, and immunoglobulins. Ilar J 46, 314-9.
Petkova S. B., Akilesh S., Sproule T. J., Christianson G. J., Al Khabbaz H., Brown A. C., Presta L. G., Meng Y. G. and Roopenian D. C. (2006) Enhanced half-life of genetically engineered human IgG1 antibodies in a humanized FcRn mouse model: potential application in humorally mediated autoimmune disease. Int Immunol 18, 1759-69.
Pfeifer A. (2006) Lentiviral transgenesis--a versatile tool for basic research and gene therapy. Curr Gene Ther 6, 535-42.
Pfeifer A., Ikawa M., Dayn Y. and Verma I. M. (2002) Transgenesis by lentiviral vectors: lack of gene silencing in mammalian embryonic stem cells and preimplantation embryos. Proc Natl Acad Sci U S A 99, 2140-5.
Popov S., Hubbard J. G., Kim J., Ober B., Ghetie V. and Ward E. S. (1996) The stoichiometry and affinity of the interaction of murine Fc fragments with the MHC class I-related receptor, FcRn. Mol Immunol 33, 521-30.
Raguz S., Hobbs C., Yague E., Ioannou P. A., Walsh F. S. and Antoniou M. (1998) Muscle-specific locus control region activity associated with the human desmin gene. Dev Biol 201, 26-42.
Rodewald R. (1976) pH-dependent binding of immunoglobulins to intestinal cells of the neonatal rat. J Cell Biol 71, 666-9.
Rogel-Gaillard C., Piumi F., Billault A., Bourgeaux N., Save J. C., Urien C., Salmon J. and Chardon P. (2001) Construction of a rabbit bacterial artificial chromosome (BAC) library: application to the mapping of the major histocompatibility complex to position 12q.1.1. Mamm Genome 12, 253-5.
Roopenian D. C. and Akilesh S. (2007) FcRn: the neonatal Fc receptor comes of age. Nat Rev Immunol 7, 715-25.
Roopenian D. C., Christianson G. J., Sproule T. J., Brown A. C., Akilesh S., lung N., Petkova S., Avanessian L., Choi E. Y., Shaffer D. J., Eden P. A. and Anderson C. L. (2003) The MHC class I-like IgG receptor controls perinatal IgG transport, IgG homeostasis, and fate of IgG-Fc-coupled drugs. J Immunol 170, 3528-33.
Sabiston B. H. and Rose J. E. (1976) Effect of cold exposure on the metabolism of immunoglobulins in rabbits. J Immunol 116, 106-11.
Sachs U. J., Socher I., Braeunlich C. G., Kroll H., Bein G. and Santoso S. (2006) A variable number of tandem repeats polymorphism influences the transcriptional activity of the neonatal Fc receptor alpha-chain promoter. Immunology 119, 83-9.
Sandford A. J. and Pare P. D. (1997) Direct PCR of small genomic DNA fragments from serum. Biotechniques 23, 890-2.
Schedl A., Grimes B. and Montoliu L. (1996) YAC transfer by microinjection. Methods Mol Biol 54, 293-306.
Schnulle P. M. and Hurley W. L. (2003) Sequence and expression of the FcRn in the porcine mammary gland. Vet Immunol Immunopathol 91, 227-31.
Schunk M. K. and Macallum G. E. (2005) Applications and optimization of immunization procedures. Ilar J 46, 241-57.
Simister N. E. and Mostov K. E. (1989) An Fc receptor structurally related to MHC class I antigens. Nature 337, 184-7.
Simister N. E. and Rees A. R. (1985) Isolation and characterization of an Fc receptor from neonatal rat small intestine. Eur J Immunol 15, 733-8.
Smit A., Hubley R. and Green P. (1996-2004) RepeatMasker Open-3.0.
Spiekermann G. M., Finn P. W., Ward E. S., Dumont J., Dickinson B. L., Blumberg R. S. and Lencer W. I. (2002) Receptor-mediated immunoglobulin G transport across mucosal barriers in adult life: functional expression of FcRn in the mammalian lung. J Exp Med 196, 303-10.
Stills H. F., Jr. (2005) Adjuvants and antibody production: dispelling the myths associated with Freund's complete and other adjuvants. Ilar J 46, 280-93.
Stirling C. M., Charleston B., Takamatsu H., Claypool S., Lencer W., Blumberg R. S. and Wileman T. E. (2005) Characterization of the porcine neonatal Fc receptor--potential use for trans-epithelial protein delivery. Immunology 114, 542-53.
Story C. M., Mikulska J. E. and Simister N. E. (1994) A major histocompatibility complex class I-like Fc receptor cloned from human placenta: possible role in transfer of immunoglobulin G from mother to fetus. J Exp Med 180,2377-81.
Sutherland H. G., Kearns M., Morgan H. D., Headley A. P., Morris C., Martin D. I. and Whitelaw E. (2000) Reactivation of heritably silenced gene expression in mice. Mamm Genome 11, 347-55.
Tesson L., Heslan J. M., Menoret S. and Anegon I. (2002) Rapid and accurate determination of zygosity in transgenic animals by real-time quantitative PCR. Transgenic Res 11, 43-8.
Thorey I., Offner S., Ros F., Schueler N., Siewe B., Niersbach H., Buelow R., van Schooten W. and Platzer J. (2006) Expression of a humanized antibody repertoire in transgenic rabbits. In International Congress of Immunogenomics and Immunomics, Budapest, Hungary.
Vidarsson G., Stemerding A. M., Stapleton N. M., Spliethoff S. E., Janssen H., Rebers F. E., de Haas M. and van de Winkel J. G. (2006) FcRn: an IgG receptor on phagocytes with a novel role in phagocytosis. Blood 108, 3573-9.
Wakayama T., Perry A. C., Zuccotti M., Johnson K. R. and Yanagimachi R. (1998) Full-term development of mice from enucleated oocytes injected with cumulus cell nuclei. Nature 394, 369-74.
Whitelaw C. B., Radcliffe P. A., Ritchie W. A., Carlisle A., Ellard F. M., Pena R. N., Rowe J., Clark A. J., King T. J. and Mitrophanous K. A. (2004) Efficient generation of transgenic pigs using equine infectious anaemia virus (EIAV) derived vector. FEBS Lett 571, 233-6.
Yoshida M., Kobayashi K., Kuo T. T., Bry L., Glickman J. N., Claypool S. M., Kaser A., Nagaishi T., Higgins D. E., Mizoguchi E., Wakatsuki Y., Roopenian D. C., Mizoguchi A., Lencer W. I. and Blumberg R. S. (2006) Neonatal Fc receptor for IgG regulates mucosal immune responses to luminal bacteria. J Clin Invest 116, 2142-2151.
Zhao Y., Kacskovics I., Zhao Z. and Hammarstrom L. (2003) Presence of the di-leucine motif in the cytoplasmic tail of the pig FcRn alpha chain. Vet Immunol Immunopathol 96, 229-33.
Zhu X., Meng G., Dickinson B. L., Li X., Mizoguchi E., Miao L., Wang Y., Robert C., Wu B., Smith P. D., Lencer W. I. and Blumberg R. S. (2001) MHC class I-related neonatal Fc receptor for IgG is functionally expressed in monocytes, intestinal macrophages, and dendritic cells. J Immunol 166, 3266-76.

## Claims

1. Transgenic (Tg) rabbit capable of developing an enhanced humoral immune response, in particular capable of developing a more robust immune response, against an antigen as compared to a non-transgenic control animal of the same species, comprising an introduced genetic construct providing for the expression of a nucleic acid sequence encoding the α-chain of the rabbit FcRn protein, wherein said genetic construct provides for enhanced MHC class I-related neonatal Fc receptor (FcRn) activity.

2. The transgenic (Tg) rabbit according to claim 1, wherein the genetic construct comprises the insert of the rabbit BAC clone #262E02 of the rabbit BAC library handled by the INRA resource centre for domestic animals.

3. The transgenic (Tg) rabbit according to claim 1 or 2, wherein said nucleic acid sequence encoding the α-chain of the rabbit FcRn protein is mutated, in particular, wherein said mutation renders the albumin binding site of said FcRn protein non-functional.

4. The transgenic (Tg) rabbit according to any one of claims 1 to 3, wherein said enhanced FcRn activity is provided by integrating more than one functional copy of a nucleic acid sequence encoding said rabbit FcRn protein into the genome of said rabbit.

5. The transgenic (Tg) rabbit according to any one of claims 1 to 4, wherein said transgenic rabbit is made from a rabbit, which is transgenic for producing human or humanized immunoglobulins.

6. Method for producing a transgenic (Tg) rabbit according to any of claims 1 to 5 comprising introducing into said rabbit the genetic construct providing for the expression of a nucleic acid sequence encoding the α-chain of the rabbit FcRn protein, wherein said genetic construct provides for enhanced FcRn activity.

7. The method according to claim 6, wherein the genetic construct comprises the rabbit BAC clone #262E02 of the rabbit BAC library handled by the INRA resource centre for domestic animals.

8. The method according to claim 6 or 7, wherein said nucleic acid sequence encoding the α-chain of the rabbit FcRn protein is mutated, such that said mutation renders the albumin binding site of said FcRn protein non-functional.

9. The method according to any one of claims 6 to 8, wherein said enhanced FcRn activity is provided by integrating more than one functional copy of a nucleic acid sequence encoding said rabbit FcRn protein into the genome of said rabbit.

10. The method according to any one of claims 6 to 9, wherein said transgenic rabbit is made from a rabbit, which is transgenic for producing human or humanized immunoglobulins.

11. Use of a genetic construct providing for enhanced MHC class I-related neonatal (FcRn) activity for the generation of a transgenic (Tg) rabbit according to any of claims 1 to 5, wherein said genetic construct provides for the expression of a nucleic acid sequence encoding the α-chain of the rabbit FcRn protein.

12. Animal propagation material obtained from a transgenic rabbit according to any one of claims 1 to 5, wherein the animal propagation material comprises a genetic construct providing for enhanced FcRn activity, wherein the genetic construct provides for the expression of a nucleic acid sequence encoding the α-chain of the rabbit FcRn protein.

13. Use of a Tg rabbit as claimed by any of claims 1 to 5 in a non-therapeutical method comprising a step of developing an enhanced humoral immune response in said Tg rabbit against an antigen of interest.

14. Non-therapeutic method for producing immunoglobulins, comprising a step of using a Tg rabbit according to any of claims 1 to 5 in accordance with any established protocol enabling the production of immunoglobulins.

15. Non-therapeutic method for producing immunoglobulins, comprising using a Tg rabbit according to any of claims 1 to 5 said method comprising at least one step in which the applied protocol is adjusted to the fact that said Tg rabbit develops the same level of humoral immune response upon fewer numbers of antigen challenges or faster as compared to a non-transgenic control rabbit.

16. Hybridoma cell line, generated from cells obtained from a transgenic rabbit according to any of claims 1 to 5, or from a transgenic rabbit used according to any of claims 13 to 15, wherein said cells comprise a genetic construct providing for enhanced FcRn activity, wherein the genetic construct provides for the expression of a nucleic acid sequence encoding the α-chain of the rabbit FcRn protein.

## Patentansprüche

1. Transgenes (Tg) Kaninchen, das eine verstärkte humorale Immunantwort gegen ein Antigen, entwickeln kann, insbesondere das eine robustere Immunantwort gegen ein Antigen, entwickeln kann, verglichen mit einem nicht-transgenen Kontrolltier derselben Spezies, umfassend ein eingeführtes genetisches Konstrukt, das zur Expression einer Nukleinsäuresequenz, die für die α-Kette des Kaninchen FcRn-Proteins kodiert, wobei dieses genetische Konstrukt eine verstärkte MHC Klasse I-bezogene neonatale Fc-Rezeptor (FcRn)-Aktivität bereitstellt.

2. Das transgene (Tg) Kaninchen nach Anspruch 1, wobei das genetische Konstrukt das Insert des Kaninchen BAC-Klons #262E02 der Kaninchen BAC Bibliothek, wie durch das INRA-Resourcencenter für Haustiere gehandhabt.

3. Das transgene (Tg) Kaninchen nach Anspruch 1 oder 2, wobei diese Nukleinsäuresequenz, die die α-Kette des Kaninchen FcRn kodiert, mutiert ist, insbesondere, wobei diese Mutation die Albuminbindungsstelle dieses FcRn-Proteins nicht funktional macht.

4. Das transgene (Tg) Kaninchen nach einem der Ansprüche 1 - 3, wobei diese verstärkte FcRn-Aktivität bereitgestellt wird durch die Integration von mehr als einer funktionalen Kopie einer Nukleinsäuresequenz, die dieses Kaninchen FcRn-Protein kodiert, in das Genom dieses Kaninchens.

5. Das transgene (Tg) Kaninchen nach einem der Ansprüche 1 - 4, wobei das transgene Kaninchen aus einem Kaninchen gemacht ist, das transgen für die Produktion von humanen oder humanisierten Immunoglobulinen ist.

6. Das Verfahren zur Herstellung eines transgenen (Tg) Kaninchens nach einem der Ansprüche 1 - 5, umfassend das Einführen des genetischen Konstrukts in dieses Kaninchen, wobei das genetische Konstrukt die Expression einer Nukleinsäuresequenz bereitstellt, die die α-Kette des Kaninchen FcRn-Proteins kodiert, wobei dieses genetische Konstrukt die verstärkte FcRn-Aktivität bereitstellt.

7. Das Verfahren nach Anspruch 6, wobei das genetische Konstrukt den Kaninchen BAC-Klon #262E02 der Kaninchen BAC-Bibliothek, wie durch das INRA Resourcencenter für Haustiere gehandhabt.

8. Das Verfahren nach einem der Ansprüche 6 oder 7, wobei diese Nukleinsäuresequenz, die die α-Kette des Kaninchen FcRn-Proteins kodiert, mutiert ist, sodass diese Mutation die Albuminbindungsstelle dieses FcRn-Proteins nicht funktional macht.

9. Das Verfahren nach einem der Ansprüche 6 - 8, wobei die verstärkte FcRn-Aktivität durch die Integration von mehr als einer funktionalen Kopie einer Nukleinsäuresequenz, die dieses Kaninchen FcRn-Protein kodiert, in das Genom dieses Kaninchens bereitgestellt wird.

10. Das Verfahren nach einem der Ansprüche 6 - 9, wobei dieses transgene Kaninchen aus einem Kaninchen gemacht ist, welches transgen für die Produktion von humanen oder humanisierten Immunoglobulinen ist.

11. Verwendung eines genetischen Konstrukts zur Bereitstellung einer verstärkten MHC-Klasse I-bezogenen neonatalen (FcRn) Aktivität, für die Herstellung eines transgenen (Tg) Kaninchens nach einem der Ansprüche 1 bis 5, wobei dieses genetische Konstrukt die Expression einer Nukleinsäuresequenz, die die α-Kette des Kaninchen FcRn-Proteins kodiert, bereitstellt.

12. Tierfortpflanzungsmaterial erhalten aus einem transgenen Kaninchen nach einem der Ansprüche 1 bis 5, wobei das Tierfortpflanzungsmaterial ein genetisches Konstrukt umfasst, das eine verstärkte FcRn-Aktivität bereitstellt, wobei das genetische Konstrukt die Expression einer Nukleinsäure bereitstellt, die die α-Kette des Kaninchen FcRn-Proteins kodiert.

13. Verwendung eines transgenen Kaninchens, wie nach einem der Ansprüche 1 bis 5 beansprucht, in einem nicht-therapeutischen Verfahren, umfassend einen Schritt der Entwicklung einer verstärkten humoralen Immunantwort in diesem transgenen Kaninchen gegen ein Antigen von Interesse.

14. Nicht-therapeutisches Verfahren zur Herstellung von Immunoglobulinen, umfassend einen Schritt der Verwendung eines transgenen Kaninchens nach einem der Ansprüche 1 bis 5 in Übereinstimmung mit einem etablierten Protokoll, das die Herstellung von Immunoglobulinen ermöglicht.

15. Nicht-therapeutisches Verfahren zur Herstellung von Immunoglobulinen, umfassend die Verwendung eines transgenen Kaninchens nach einem der Ansprüche 1 bis 5, wobei dieses Verfahren mindestens einen Schritt umfasst, in welchem das angewandte Protokoll so angepasst wird, dass das transgene Kaninchen das gleiche Niveau einer humoralen Immunantwort nach einer geringeren Anzahl von Antigen-Expositionen oder schneller, verglichen mit einem nicht-transgenen Kontrollkaninchens, entwickelt.

16. Hybridomzelllinie, hergestellt aus Zellen, die aus einem transgenen Kaninchen nach einem der Ansprüche 1 bis 5 erhalten werden oder aus einem transgenen Kaninchen, wie nach einem der Ansprüche 13 bis 15 verwendet, wobei diese Zellen ein genetisches Konstrukt umfassen, das eine verstärkte FcRn-Aktivität bereitstellt, wobei das genetische Konstrukt die Expression einer Nukleinsäuresequenz, die die α-Kette des Kaninchen FcRn-Proteins kodiert, bereitstellt.

## Revendications

1. Lapin transgénique (Tg) capable de développer une réponse immunitaire humorale renforcée, en particulier capable de développer une réponse immunitaire plus robuste, contre un antigène comparé à un animal témoin non transgénique de la même espèce, comprenant une construction génétique introduite permettant l'expression d'une séquence d'acide nucléique codant pour la chaîne α de la protéine FcRn de lapin, dans lequel ladite construction génétique permet une activité renforcée du récepteur Fc néonatal (FcRn) apparenté au CMH de classe I.

2. Lapin transgénique (Tg) selon la revendication 1, dans lequel la construction génétique comprend l'insert du clone de BAC de lapin #262E02 de la banque de BAC de lapin gérée par le centre de ressources des animaux domestiques de l'INRA.

3. Lapin transgénique (Tg) selon la revendication 1 ou 2, dans lequel ladite séquence d'acide nucléique codant pour la chaîne α de la protéine FcRn de lapin est mutée, en particulier dans lequel la mutation rend le site de liaison de l'albumine de ladite protéine FcRn non fonctionnel.

4. Lapin transgénique (Tg) selon l'une quelconque des revendications 1 à 3, dans lequel ladite activité renforcée de FcRn est permise par intégration de plus d'une copie fonctionnelle d'une séquence d'acide nucléique codant pour ladite protéine FcRn de lapin dans le génome dudit lapin.

5. Lapin transgénique (Tg) selon l'une quelconque des revendications 1 à 4, dans lequel ledit lapin transgénique est préparé à partir d'un lapin, qui est transgénique pour la production d'immunoglobulines humaines ou humanisées.

6. Procédé de production d'un lapin transgénique (Tg) selon l'une quelconque des revendications 1 à 5, comprenant l'introduction dans ledit lapin de la construction génétique permettant l'expression d'une séquence d'acide nucléique codant pour la chaîne α de la protéine FcRn de lapin, dans lequel ladite construction génétique permet l'activité renforcée de FcRn.

7. Procédé selon la revendication 6, dans lequel la construction génétique comprend le clone de BAC de lapin #262E02 de la banque de BAC de lapin gérée par le centre de ressources des animaux domestiques de l'INRA.

8. Procédé selon la revendication 6 ou 7, dans lequel ladite séquence d'acide nucléique codant pour la chaîne α de la protéine FcRn de lapin est mutée, de telle sorte que ladite mutation rend le site de liaison de l'albumine de ladite protéine FcRn non fonctionnel.

9. Procédé selon l'une quelconque des revendications 6 à 8, dans lequel ladite activité renforcée de FcRn est permise par intégration de plus d'une copie fonctionnelle d'une séquence d'acide nucléique codant pour ladite protéine FcRn de lapin dans le génome dudit lapin.

10. Procédé selon l'une quelconque des revendications 6 à 9, dans lequel ledit lapin transgénique est préparé à partir d'un lapin, qui est transgénique pour la production d'immunoglobulines humaines ou humanisées.

11. Utilisation d'une construction génétique permettant une activité renforcée du récepteur Fc néonatal (FcRn) apparenté au CMH de classe I pour la génération d'un lapin transgénique (Tg) selon l'une quelconque des revendications 1 à 5, dans laquelle ladite construction génétique permet l'expression d'une séquence d'acide nucléique codant pour la chaîne α de la protéine FcRn de lapin.

12. Matériau de propagation animal obtenu d'un lapin transgénique selon l'une quelconque des revendications 1 à 5, dans lequel le matériau de propagation animal comprend une construction génétique permettant une activité renforcée de FcRn, dans lequel la construction génétique permet l'expression d'une séquence d'acide nucléique codant pour la chaîne α de la protéine FcRn de lapin.

13. Utilisation d'un lapin Tg selon l'une quelconque des revendications 1 à 5 dans un procédé non thérapeutique comprenant une étape de développement d'une réponse immunitaire humorale renforcée dans ledit lapin Tg contre un antigène d'intérêt.

14. Procédé non thérapeutique de production d'immunoglobulines, comprenant une étape d'utilisation d'un lapin Tg selon l'une quelconque des revendications 1 à 5 conformément à tout protocole établi activant la production d'immunoglobulines.

15. Procédé non thérapeutique de production d'immunoglobulines, comprenant l'utilisation d'un lapin Tg selon l'une quelconque des revendications 1 à 5, ledit procédé comprenant au moins une étape dans laquelle le protocole appliqué est ajusté au fait que ledit lapin Tg développe le même niveau de réponse immunitaire humorale après un nombre moins important d'expositions à un antigène ou plus rapidement par rapport à un lapin témoin non transgénique.

16. Lignée cellulaire d'hybridome, générée à partir de cellules obtenues d'un lapin transgénique selon l'une quelconque des revendications 1 à 5, ou d'un lapin transgénique utilisé selon l'une quelconque des revendications 13 à 15, dans laquelle lesdites cellules comprennent une construction génétique permettant l'activité renforcée de FcRn, dans laquelle la construction génétique permet l'expression d'une séquence d'acide nucléique codant pour la chaîne α de la protéine FcRn de lapin.
